# EUROPEAN PATENT SPECIFICATION

(11) **EP 4 216 830 B1**
(45) Date of publication and mention of the grant of the patent: **28.01.2026**
(21) Application number: 21794956.9
(22) Date of filing: 23.09.2021
(51) Int. Cl.: A61B 17/04, A61F 2/24, A61B 17/068, A61B 17/00, A61B 90/00, A61B 17/064

(54) **ANCHOR MAGAZINES**
ANKERMAGAZINE
CHARGEURS D'ÉLÉMENTS D'ANCRAGE

(30) Priority: 25.09.2020 US 202063083571 P
(43) Date of publication of application: 02.08.2023
(60) Divisional application: 25222708.7
(73) Proprietor: Edwards Lifesciences Innovation (Israel) Ltd., 3079892 Caesarea (IL)
(72) Inventor: HALABI, Ido, 53631 Givatayim (IL); KASHER, Yuval, 9978800 Kfar Shmu'el (IL); HERMAN, Yaron, 3780800 Givat Ada (IL); SHARON, Assaf, 6291606 Tel Aviv (IL); ZUARETZ, Yosef, 5827016 Holon (IL)
(74) Representative: Eisenführ Speiser
(86) International application number: PCT/IB2021/058665
(87) International publication number: WO 2022/064401

(56) References cited:
- EP-A2- 3 501 405
- US-A1- 2005 251 177
- US-A1- 2017 360 420
- US-A1- 2018 153 543
- US-B1- 6 457 625

## Description

### CROSS-REFERENCES TO RELATED APPLICATIONS

The present application claims priority to US Provisional Patent Application 63/083,571 to Halabi et al., filed September 25, 2020, and entitled "ANCHOR MAGAZINES".

### BACKGROUND

Annuloplasty involves remodeling tissue of an annulus. This can be done by pulling tissue about the annulus to a new shape. Tissue anchors can be used to facilitate medical procedures including annuloplasty, other remodeling of tissues, and securing implants. In some instances, tissue anchors can be used as an alternative to sutures. For example, a tissue anchor may be used for a procedure in which there is no line-of-sight to the target.

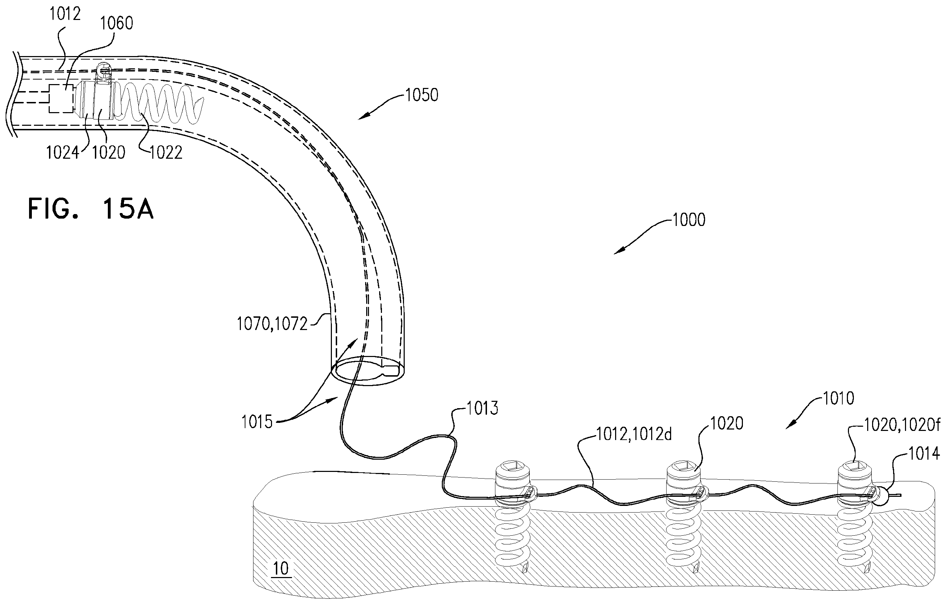

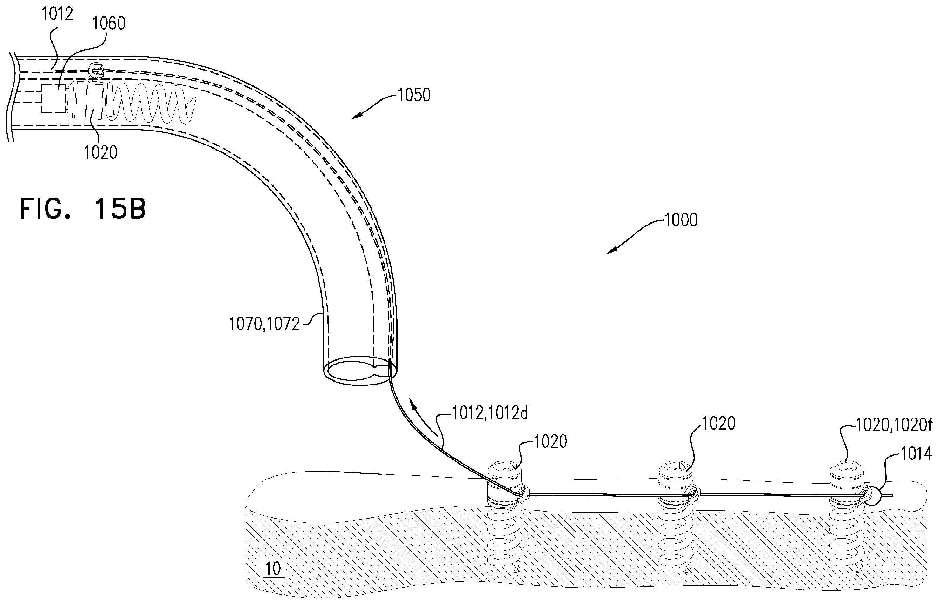

US 2005/251177 A1 (US'177) describes an apparatus for rapid deployment of tissue anchors. A tissue manipulation assembly is pivotably coupled to the distal end of a tubular member and has a lower jaw member and an upper jaw member pivotably coupled to the lower jaw member. A reconfigurable launch tube is pivotably coupled to the upper jaw member and is used to urge the jaw members from a low-profile configuration to an open configuration. A needle assembly can be advanced through the launch tube across tissue received between the jaw members of the tissue manipulation assembly. Tissue anchors can be advanced through the needle assembly for securing received tissue. The tissue anchors can be positioned within a reloadable chamber of a control handle disposed outside the patient, then advanced through the needle assembly (cf. abstract of US' 177). As shown in Figs. 15A and 15B of US' 177, a plurality of cartridges having anchor assemblies can be disposed within a spring-loaded magazine clip for rapid reloading of a needle deployment assembly. The cartridges may be provided to the medical practitioner pre-loaded within the clip or may be loaded within the clip by the medical practitioner (cf. [0090], [0091] of US' 177). US' 177 does not disclose that a cartridge holding a tissue anchor is moveable by hand by the human operator along a track of an extracorporeal controller from an initial position to a deployment position.

### SUMMARY

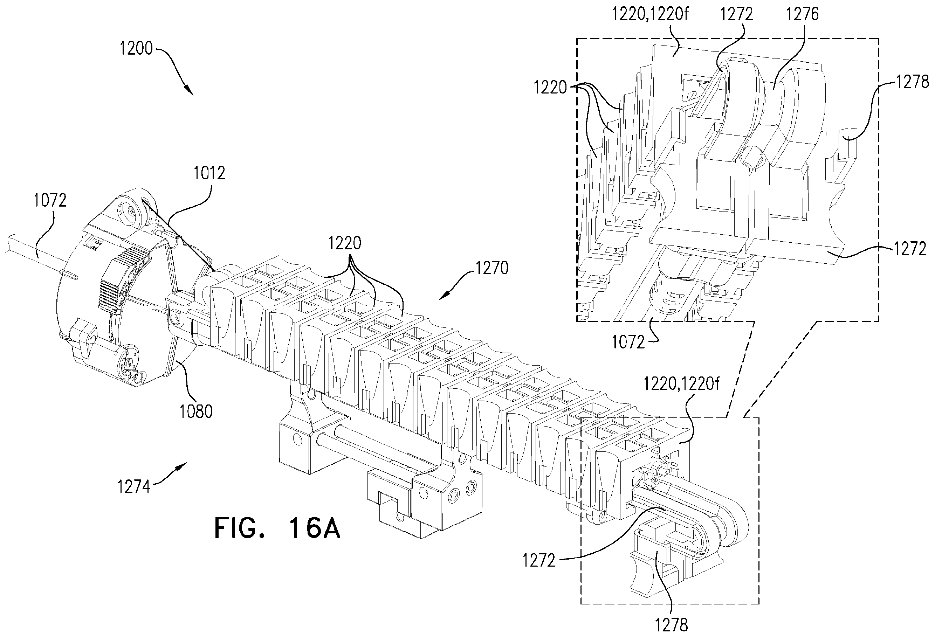

The claimed invention is defined in independent claim 1 and relates to a system for use with a subject. Preferred configurations of the claimed invention are defined in dependent claims 2 to 15. Certain aspects and/or particularly preferred configurations of the claimed invention are discussed hereafter for example in conjunction with Figs. 16A-18B. Also described herein are related examples, embodiments and arrangements useful for understanding the claimed invention.

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure may be included in the examples summarized here.

For some applications, systems, apparatuses, and methods are provided for percutaneously (e.g., transluminally) delivering one or more anchors to tissue of a subject and anchoring the anchors to the tissue. For some such applications, the tissue anchors are components of an implant that further comprises a tether on which the tissue anchors are pre-threaded. For some such applications, a distal portion of the tether becomes chronically implanted with the anchors, and a proximal portion of the tether is removed from the subject after the anchors (and therefore the implant) is anchored to the tissue.

The methods herein can be performed on a living animal or on a simulation, such as on a cadaver, cadaver heart, simulator (e.g., with the body parts, tissue, etc. being simulated), etc.

Some applications relate to systems, apparatuses, and methods for determining successful (e.g., complete) anchoring of one or more tissue anchors to a tissue that is not in line-of-sight, such as during percutaneous (e.g., transluminal) techniques.

In some applications, the tissue anchor includes a tissue-engaging element and a head. The tissue-engaging element can be or comprise one or more of hook(s), clip(s), dart(s), barb(s), staple(s), tine(s), needle(s), helical portion(s), screw(s), tissue-penetrating portion(s), etc.

In some applications, an anchor driver can engage the anchor at the head (e.g., reversibly attaching to the head), and drives the tissue-engaging element into the tissue. Often, successful anchoring includes the tissue-engaging element becoming fully embedded in the tissue, e.g., such that the head abuts the surface of the tissue.

For some applications, respective catheter systems are provided which include transluminally-advanceable catheters including respective mechanisms for controlling the advancement of tissue anchors stored within an anchor-storage zone of the transluminally-advanceable catheters and or of an extracorporeal unit (e.g., a controller and/or handle) coupled to the catheter. For some applications, the mechanism can include a helical thread that is rotatable to control the advancement of an anchor beyond the anchor-storage zone and to prevent the advancement of too many anchors at a given time out of the anchor-storage zone.

For some applications, the mechanism can include a plurality of detents to control the advancement of an anchor beyond the anchor-storage zone and to prevent the advancement of too many anchors at a given time out of the anchor-storage zone. For some applications, the mechanism can include a dispenser to control the advancement of an anchor beyond the anchor-storage zone and to prevent the advancement of too many anchors at a given time out of the anchor-storage zone. For some applications, the mechanism can include a revolving stopper at the extracorporeal unit which controls the advancement of an anchor beyond the anchor-storage zone and prevents the advancement of too many anchors at a given time out of the anchor-storage zone.

For some applications, respective catheter systems are provided which include respective anchor drivers for reversibly coupling to the tissue anchor and facilitating driving of the anchor into the tissue.

For some applications, respective catheter systems are provided which include a tensioner that applies tension (e.g., a pre-determined and/or constant amount of tension) to the tether during implantation, e.g., to reduce a likelihood of inadvertent tangling or ensnaring. For some such applications, the anchors can be threaded, along the tether, between the tensioner and the distal end of the tether. For other applications, the tensioner can be disposed, along the tether, between the anchors and the distal end of the tether. For some applications, the tensioner is reversibly engageable with the tether, e.g., such that an operator can choose when to apply tension to the tether and when not.

There is therefore provided, in accordance with some applications, system and/or apparatus for use with a subject, including a catheter device, a series of tissue anchors, a series of cartridges, and an anchor driver.

In some applications, the catheter device includes a tube that has a distal opening that is configured to be advanced into the subject and a proximal opening.

In some applications, the catheter device includes an extracorporeal unit (e.g., a handle, a controller, etc.) that defines a deployment position, and includes a track that leads to the deployment position.

In some applications, each cartridge of the series of cartridges holds a respective tissue anchor of the series of tissue anchors.

In some applications, each cartridge of the series is coupled to the extracorporeal unit at a respective initial position, e.g., of a series of initial positions.

In some applications, each cartridge of the series, while remaining coupled to the extracorporeal unit, is moveable along the track from the respective initial position to the deployment position such that the cartridge holds the respective tissue anchor opposite the proximal opening.

In some applications, each cartridge of the series is subsequently removable from the deployment position such that the deployment position is vacant for a successive cartridge in the series.

In some applications, the anchor driver, for each of the anchors, is configured to be coupled to the anchor while the anchor is held by the respective cartridge opposite the proximal opening, and to advance the anchor distally out of the respective cartridge, through the proximal opening, and through the catheter toward the distal opening.

In some applications, the catheter device further includes a port at the proximal opening of the tube, the port including a membrane, and the membrane is shaped to define a first aperture through the membrane, a second aperture through the membrane, and a closed slit connecting the first aperture with the second aperture.

In some applications, the first aperture is wider in diameter than the second aperture.

In some applications, the first aperture is 3-10 times larger than the second aperture.

In some applications, the anchor driver is configured to, for each of the anchors, advance the anchor distally out of the respective cartridge, through the proximal opening, and through the tube toward the distal opening while the respective cartridge remains in the deployment position.

In some applications, wherein, for each cartridge of the series of cartridges, the cartridge is configured such that, while (i) the cartridge is at the deployment position, and (ii) the anchor driver is extended distally beyond the cartridge and through the tube toward the distal opening, the cartridge is removable from the deployment position.

In some applications, for each cartridge of the series of cartridges, the cartridge is configured such that, while (i) the cartridge is at the deployment position, and (ii) the anchor driver is extended distally beyond the cartridge and through the tube toward the distal opening, the anchor driver inhibits removal of the cartridge from the deployment position.

In some applications each of the tissue anchors includes a tissue-engaging element, and a head that defines an eyelet. The tissue-engaging element can be or comprise one or more of hook(s), clip(s), dart(s), barb(s), staple(s), tine(s), needle(s), helical portion(s), screw(s), tissue-penetrating portion(s), etc.

In some applications, the system and/or apparatus further includes a tether. The tether can be threaded through the eyelet of each of the tissue anchors, have a proximal portion that includes a proximal end of the tether, and having a distal portion that includes a distal end of the tether. In some applications, the distal end of the tether being advanceable distally through the catheter into the subject while the proximal end of the tether remains outside of the subject.

In some applications, the anchor driver, for each of the anchors, is configured to advance the anchor distally out of the respective cartridge, through the proximal opening, and through the catheter toward the distal opening, while the eyelet of the anchor remains threaded on the tether.

In some applications, the catheter device further includes a port at the proximal opening of the tube, the port including a membrane. In some applications, the membrane is shaped to define a first aperture through the membrane, a second aperture through the membrane, and a closed slit connecting the first aperture with the second aperture. In some applications, the port is arranged such that, for each of the anchors, the anchor driver is configured to advance the anchor distally out of the respective cartridge and through the membrane with the tissue-engaging element passing through the first aperture and the tether extending through the second aperture.

In some applications, the port is arranged such that, for each of the anchors, the anchor driver is configured to advance the anchor distally out of the respective cartridge and through the membrane with the tissue-engaging element passing through the first aperture and the tether extending through the second aperture.

In some applications, the port is arranged such that, for each of the anchors, as the anchor driver advances the anchor distally out of the respective cartridge and through the membrane, the first aperture transiently widens as the tissue-engaging element of the anchor passes through the first aperture.

In some applications, the port is arranged such that, for each of the anchors, after the anchor driver advances the anchor distally out of the respective cartridge and through the membrane, the first aperture seals around the anchor driver.

In some applications, the port is arranged such that, for each of the anchors, as the anchor driver advances the anchor distally out of the respective cartridge and through the membrane, the second aperture transiently widens as the eyelet of the anchor passes through the second aperture.

In some applications, the port is arranged such that, for each of the anchors, as the anchor driver advances the anchor distally out of the respective cartridge and through the membrane, the slit transiently opens as the anchor passes through the membrane.

In some applications, the catheter device further includes a port at the proximal opening of the tube, the port including a membrane. In some applications, the membrane is shaped to define a first aperture through the membrane, a second aperture through the membrane, and a closed slit connecting the first aperture with the second aperture. In some applications, the port is arranged such that the membrane is oriented substantially transversely to the proximal end of the tube, and for each cartridge of the series of cartridges, while the cartridge is in the deployment position, the tissue-engaging element of the respective tissue anchor is aligned with the first aperture, thereby defining an anchor-advancement axis from the respective tissue anchor, through the first aperture, and through the tube.

In some applications, the port is arranged such that, upon the distal end of the tether being advanced distally through the tube into the subject while the proximal end of the tether remains outside of the subject, the tether extends through the second aperture.

In some applications, the catheter device further includes a spring and a spool, coupled to the spring such that rotation of the spool in a first direction applies stress to the spring.

In some applications, the proximal portion of the tether is wound around the spool such that advancing of the distal portion of the tether distally through the catheter rotates the spool in the first direction.

In some applications, each cartridge of the series of cartridges is configured to lock to the extracorporeal unit upon arriving at the deployment position.

In some applications, each cartridge of the series of cartridges is shaped to be grasped by hand by a human operator and is configured to be moved along the track by hand by the operator.

In some applications, each cartridge of the series of cartridges is removable from the deployment position by being removed from the extracorporeal unit.

In some applications, each cartridge of the series of cartridges includes a displaceable barrier that obstructs the respective anchor from being advanced distally out of the cartridge.

In some applications, each cartridge of the series of cartridges includes a displacement mechanism that, upon actuation of the displacement mechanism, displaces the barrier such that the barrier ceases to obstruct the respective anchor from being advanced distally out of the cartridge.

In some applications, the displacement mechanism is configured such that actuation of the displacement mechanism displaces the barrier by linear translation of the barrier.

In some applications, the displacement mechanism is configured such that actuation of the displacement mechanism displaces the barrier by deflection of the barrier.

In some applications, the displacement mechanism is spring-loaded.

In some applications, the displacement mechanism is configured to be actuated by a force applied to the respective anchor.

In some applications, the displacement mechanism is configured to be actuated by the anchor driver pulling the respective anchor proximally.

In some applications, for each cartridge of the series of cartridges, the cartridge includes:
a first piece that includes the barrier, and
a second piece that holds the respective anchor;
the displacement mechanism is constrained by a detent of the cartridge, and
the displacement mechanism is configured to be actuated by the anchor driver pulling the respective anchor proximally with sufficient force that the respective anchor pulls the second piece proximally with respect to the first piece such that the detent ceases to constrain the displacement mechanism.

In some applications, the displacement mechanism is defined by the first piece.

In some applications, the first piece defines the detent.

In some applications, the detent constrains the displacement mechanism by contacting the respective anchor.

In some applications, the respective anchor includes a helical tissue-engaging element, and the detent constrains the displacement mechanism by being disposed within the helical tissue-engaging element of the respective anchor.

In some applications, the second piece defines the detent.

In some applications, wherein the detent constrains the displacement mechanism by contacting the engagement mechanism.

In some applications, wherein the cartridge is coupled to the extracorporeal unit via coupling between the first piece and the extracorporeal unit.

In some applications, wherein the cartridge is coupled to the extracorporeal unit via coupling between the second piece and the extracorporeal unit.

In some applications, each of the cartridges includes a first piece that couples the cartridge to the extracorporeal unit and holds the respective anchor and a second piece that includes the barrier.

In some applications, the first piece defines a detent that constrains the displacement mechanism, and the displacement mechanism is configured to be actuated by anchor driver pulling the respective anchor proximally with sufficient force that the respective anchor pulls the first piece proximally with respect to the second piece such that the detent ceases to constrain the displacement mechanism.

In some applications, the first piece slidably couples the cartridge to the track.

In some applications, each of the cartridges is configured such that the anchor driver pulling the respective anchor proximally with sufficient force that the respective anchor pulls the first piece proximally with respect to the second piece reconfigures the cartridge into a removable state that facilitates removal of the cartridge from the deployment position.

In some applications, the first piece is mounted inside the second piece, and the second piece is shaped to be grasped by hand by a human operator.

There is additionally provided, system and/or apparatus including a catheter device, a first cartridge, a second cartridge, and an anchor driver. Optionally, the system and/or apparatus can include additional cartridges as well (e.g., a third cartridge, a fourth cartridge, a fifth cartridge, a sixth cartridge, and more), which additional cartridges can be the same as or similar to the first cartridge and/or the second cartridge and include any of the same features.

In some applications, the catheter device includes a tube that has a proximal opening, and a controller or handle that includes a track that leads to a deployment position.

In some applications, the first cartridge holds a first tissue anchor and is coupled to the controller or handle and, while remaining coupled to the controller or handle, is moveable along the track from a first initial position to the deployment position such that the first cartridge holds the first tissue anchor opposite the proximal opening.

In some applications, a second cartridge holds a second tissue anchor and is coupled to the controller or handle and, while remaining coupled to the controller or handle, is moveable along the track from a second initial position to the deployment position such that the second cartridge holds the second tissue anchor opposite the proximal opening.

In some applications, the anchor driver is couplable to the first anchor while the first anchor is held by the first cartridge opposite the proximal opening and is configured to advance the first anchor distally out of the first cartridge through the proximal opening and through the tube, and the anchor driver is couplable to the second anchor while the second anchor is held by the second cartridge opposite the proximal opening and is configured to advance the second anchor distally out of the second cartridge through the proximal opening and through the tube.

In some applications, the first tissue anchor includes a first tissue-engaging element and a first head including a first eyelet. In some applications, the second tissue anchor includes a second tissue-engaging element and a second head including a second eyelet. The tissue-engaging elements can be or comprise one or more of hook(s), clip(s), dart(s), barb(s), staple(s), tine(s), needle(s), helical portion(s), screw(s), tissue-penetrating portion(s), etc.

In some applications, the system and/or apparatus further includes a tether (e.g., a wire, line, suture, elongate member, etc.) threaded through the first eyelet and the second eyelet, the tether having a proximal portion that includes a proximal end of the tether and having a distal portion that includes a distal end of the tether, the distal end of the tether being advanceable distally through the tube into the subject while the proximal end of the tether remains outside of the subject. In some applications, with more cartridges and more anchors, the tether is threaded through multiple additional eyelets of additional anchors.

In some applications, the anchor driver is configured to advance the first anchor distally out of the first cartridge, through the proximal opening, and through the tube, while the first eyelet of the first anchor remains threaded on the tether. In some applications, the anchor driver is configured to advance the second anchor distally out of the second cartridge, through the proximal opening, and through the tube, while the second eyelet of the second anchor remains threaded on the tether. In some applications with additional anchors, the anchor driver can similarly advance the additional anchors out of additional cartridges through the proximal opening and the tube while eyelets of the additional anchors remain threaded on the tether.

In some applications, the catheter device further includes a tensioning device configured to apply tension to the tether. The tensioning device can be configured in a variety of ways.

In some applications, the tensioning device includes a spring and a spool, the spool coupled to the spring such that rotation of the spool in a first direction applies stress to the spring, and wherein the proximal portion of the tether is wound around the spool such that advancing of the distal portion of the tether distally through the tube rotates the spool in the first direction.

In some applications, each of the first cartridge and the second cartridge is configured to lock to the controller or handle upon arriving at the deployment position.

In some applications, each of the first cartridge and the second cartridge is shaped to be grasped by hand by a human operator and is configured to be moved along the track by hand by the human operator.

In some applications, each of the first cartridge and the second cartridge is removable from the deployment position by being removed from the controller or handle.

In some applications, the first cartridge includes a first displaceable barrier that inhibits the first anchor from being advanced distally out of the first cartridge. In some applications, the second cartridge includes a second displaceable barrier that inhibits the second anchor from being advanced distally out of the second cartridge.

In some applications, the first cartridge includes a first displacement mechanism that, upon actuation of the first displacement mechanism, displaces the first displaceable barrier such that the first displaceable barrier ceases to obstruct the first anchor from being advanced distally out of the first cartridge. In some applications, the second cartridge includes a second displacement mechanism that, upon actuation of the second displacement mechanism, displaces the second displaceable barrier such that the second displaceable barrier ceases to obstruct the second anchor from being advanced distally out of the second cartridge.

In some applications, the first displacement mechanism and the second displacement mechanism are each spring-loaded.

In some applications, the first displacement mechanism is configured to be actuated by a force applied to the first anchor, and wherein the second displacement mechanism is configured to be actuated by a force applied to the second anchor.

In some applications, the first displacement mechanism is configured to be actuated by the anchor driver pulling the first anchor proximally, and wherein the second displacement mechanism is configured to be actuated by the anchor driver pulling the second anchor proximally.

In some applications, the first cartridge includes a first piece that couples the first cartridge to the extracorporeal unit and holds the first anchor, and a second piece that includes the barrier.

In some applications, the first piece defines a detent that constrains the first displacement mechanism, and wherein the first displacement mechanism is configured to be actuated by anchor driver pulling the first anchor proximally with sufficient force that the first anchor pulls the first piece proximally with respect to the second piece such that the detent ceases to constrain the first displacement mechanism.

In some applications, the first piece slidably couples the first cartridge to the track.

In some applications, the first cartridge is configured such that the anchor driver pulling the first anchor proximally with sufficient force that the first anchor pulls the first piece proximally with respect to the second piece reconfigures the first cartridge into a removable state that facilitates removal of the first cartridge from the deployment position.

In some applications, the first piece is mounted inside the second piece, and the second piece is shaped to be grasped by hand by a human operator.

In some applications, the system and/or apparatus further includes a third cartridge holding a third tissue anchor and being coupled to the controller or handle and, while remaining coupled to the controller or handle, being moveable along the track from a third initial position to the deployment position such that the third cartridge holds the third tissue anchor opposite the proximal opening.

There is further provided, in accordance with some applications, a catheter system or other system and/or apparatus that includes a catheter system. The catheter system includes an extracorporeal unit (e.g., a controller and/or handle) at a proximal portion of the catheter system, and a transluminally-advanceable catheter (e.g., tubing, etc.) extending distally from the controller. The catheter can extend along a longitudinal catheter axis, from the controller to a distal opening of the catheter, e.g., when in a straight configuration. The catheter includes a channel and a generally tubular wall that defines a lumen along the length of the catheter or along the catheter axis when in a straight configuration.

In some applications, the tubular wall has a lateral slit extending along at least part of the channel at a distal portion thereof.

In some applications the tubular wall has a helical thread, overlapping the channel in part, and operably coupled to the controller such that, via operation of the controller, the thread is rotatable about the catheter axis with respect to the channel.

In some applications, the catheter has an anchor-storage zone in which the thread is longitudinally at least partly coincident with the slit, and at least one tissue anchor is disposed or disposable within the anchor-storage zone.

In some applications, the tissue anchor includes a tissue-engaging portion and an anchor head. The tissue-engaging portion can be disposed within the lumen of the channel and define a tissue anchor axis. The anchor head can be coupled to the tissue-engaging portion and define a driver interface.

In some applications, the anchor and/or anchor head includes a protrusion. For example, the protrusion can be coupled to the tissue-engaging portion via the anchor head. The protrusion can be configured to protrude laterally with respect to the tissue-engaging portion so as to extend through the slit and engage the thread.

The anchor and the slide can be configured to engage each other such that while the thread is stationary with respect to the channel, the tissue anchor is secured within the anchor-storage zone, and rotation of the thread about the tissue anchor axis and/or the catheter axis with respect to the channel advances the tissue anchor distally until the protrusion exits a distal exit of the thread.

In some applications, the slit defines a linear slit disposed in parallel with the catheter axis.

In some applications, the protrusion is rotatably coupled to the tissue-engaging portion via the anchor head.

In some applications, the system and/or apparatus further includes an inner tube surrounding a proximal portion of the channel, and the thread extends from a distal end of the inner tube and over the distal end portion of the channel and defines a tubular structure.

In some applications, the at least one tissue anchor includes at least first and second tissue anchors disposed sequentially within the anchor-storage zone, each of the first and second tissue anchors including a respective protrusion.

In some applications, while the thread is stationary, the first tissue anchor is disposed in a first location distally to the second tissue anchor that is disposed in a second location.

In some applications, rotation of the thread about the catheter axis with respect to the channel (a) advances the first tissue anchor distally until the protrusion of the first tissue anchor exits a distal exit of the thread and the first tissue anchor is moved distally away from the first location, and (b) advances the second tissue anchor distally away from the second location until the second tissue anchor is moved distally into the first location.

In some applications, the system and/or apparatus further includes a spring disposed within the catheter proximally to the second tissue anchor, the spring being configured to facilitate distal movement of the first and second tissue anchors.

In some applications, the system and/or apparatus further includes an anchor driver that includes a driver head coupled to a flexible shaft.

In some applications, the driver head is dimensioned to be axially slid within the lumen of the channel and is actuatable to reversibly engage the driver interface within the anchor-storage zone.

In some applications, the anchor driver is configured to, while the driver head is engaged with the driver interface, advance the anchor distally away from the anchor-storage zone, toward the distal opening of the catheter and drive the tissue-engaging portion along the tissue anchor axis into the tissue.

In some applications, the catheter system is arranged such that the anchor driver is configured to advance the anchor distally during the rotation of the thread about the catheter axis with respect to the channel.

In some applications, the system and/or apparatus includes an outer tube disposed around the channel and thread in a manner in which the thread is disposed between the outer tube and the channel.

In some applications, the thread is moveable and rotatable with respect to the outer tube and with respect to the channel.

In some applications, an inner surface of the outer tube defines a helical groove which defines the thread in a manner in which rotation of the outer tube facilitates rotation of the thread.

In some applications the tissue anchor defines a tissue anchor axis, the tissue-engaging element is disposed along the axis and is rotatable about the tissue anchor axis, and the tissue-engaging element is configured to be driven into tissue of a subject along the tissue anchor axis, and the protrusion extends radially away from the tissue anchor axis.

In some applications, the anchor head is fixedly coupled to the tissue-engaging portion, and the protrusion is rotatably coupled to anchor head.

In some applications, the anchor head is disposed within the lumen.

In some applications, the thread defines a plurality of adjacent ribbons arranged helically, the protrusion rests against a surface defining a depth of a distal one of the plurality of ribbons and in a pitch between the distal one of the plurality of ribbons and an adjacent ribbon proximal to the distal one of the plurality of ribbons, and during the rotation of the thread about the catheter axis, the protrusion slides along the surface defining the depth of the distal one of the plurality of ribbons.

In some applications, the tissue anchor defines a tissue-anchor-lumen along the tissue anchor axis, and the system and/or apparatus further includes an anchor driver that includes a driver head coupled to a flexible shaft.

In some applications, the driver head is dimensioned to be axially slid within the tissue-anchor-lumen and is actuatable to reversibly engage the driver interface within the anchor-storage zone.

In some applications, the anchor driver is configured to, while the driver head is engaged with the driver interface: advance the anchor distally away from the anchor-storage zone, toward the distal opening of the catheter and drive the tissue-engaging portion along the anchor axis into the tissue.

In some applications, the catheter system is arranged such that the anchor driver is configured to advance the anchor distally during the rotation of the thread about the catheter axis with respect to the channel.

In some applications, the protrusion is shaped so as to define an eyelet defining an aperture.

In some applications, the system includes a wire slidably coupled to the at least one tissue anchor and the wire is threaded through the aperture of the eyelet of the protrusion.

There is additionally provided, in accordance with some applications, a system and/or an apparatus, including at least one tissue anchor and a catheter system.

In some applications, the catheter system includes an extracorporeal unit (e.g., a controller and/or handle) at a proximal portion of the catheter system, and a transluminally-advanceable catheter (e.g., tubing, etc.) extending distally from the controller to a distal opening of the catheter. In some applications, the catheter extends along a longitudinal catheter axis, e.g., when in a straight configuration.

In some applications, the catheter includes a channel, which can include a generally tubular wall that defines a lumen along the length of the catheter or along the catheter axis. The anchor can be disposed in the lumen.

In some applications, a dispenser is distal to the anchor and includes a proximal detent and a distal detent, and operably coupled to the controller so as to be transitionable, via operation of the controller, between a receiving state, a closed state, and a dispensing state.

In some applications, in the receiving state: (i) the proximal detent is collapsed away from the catheter axis, permitting the anchor to move distally past the proximal detent and into the dispenser, and (ii) the distal detent extends into the lumen, obstructing the anchor from moving distally past the distal detent and out of the dispenser.

In some applications, in the closed state: (a) the proximal detent extends into the lumen, obstructing the anchor from returning proximally past the proximal detent and out of the dispenser, and (b) the distal detent extends into the lumen, obstructing the anchor from moving distally past the distal detent and out of the dispenser.

In some applications, in the dispensing state: (1) the proximal detent extends into the lumen, obstructing the anchor from returning proximally past the proximal detent and out of the dispenser, and (2) the distal detent is collapsed away from the catheter axis, permitting the anchor to move distally past the distal detent and out of the dispenser.

In some applications, the system and/or apparatus further includes a tubular structure surrounding at least a portion of the channel, the tubular structure being coupled to the dispenser in a manner in which movement of the tubular structure longitudinally distally and proximally along the channel facilitates collapsing of the proximal and distal detents and extending of the proximal and distal detents into the lumen of the channel.

In some applications, the tubular wall of the channel is shaped so as to define a lateral window out of the lumen, the window having a proximal edge and a distal edge, and the tubular structure surrounding the at least the portion of the channel at the lateral window.

In some applications, movement of the tubular structure proximally with respect to the lateral window facilitates collapsing of the proximal detent against the proximal edge of the window such that the proximal detent moves away from the catheter axis.

In some applications, movement of the tubular structure distally with respect to the lateral window facilitates collapsing of the distal detent against the distal edge of the window such that the distal detent moves away from the catheter axis.

In some applications, the system and/or apparatus further includes an outer tube disposed around the tubular structure in a manner in which the tubular structure is disposed between the outer tube and the channel.

In some applications, the at least one tissue anchor defines a first tissue anchor, and the system and/or apparatus further includes at least one additional tissue anchor, disposed within the lumen proximal to the dispenser.

In some applications, subsequently to the dispensing state in which the first tissue anchor is moved distally past the distal detent and out of the dispenser: the dispenser returns to the receiving state in which the dispenser permits the at least one additional anchor to move distally past the proximal detent, into the dispenser, the dispenser transitions to the closed state in which the dispenser obstructs the at least one additional anchor from moving distally past the distal detent and out of the dispenser, and subsequently, the dispenser transitions to the dispensing state, in which the dispenser permits the at least one additional tissue anchor to move distally past the distal detent and out of the dispenser.

In some applications, the system and/or apparatus includes a spring disposed within the catheter proximally to the at least one additional tissue anchor, the spring being configured to facilitate distal movement of the first tissue anchor and at least one additional tissue anchor.

In some applications, the at least one tissue anchor includes a tissue-engaging portion, and anchor head, and an eyelet. The tissue-engaging portion can be disposed within the lumen of the channel and define a tissue anchor axis. The anchor head can be coupled to the tissue-engaging portion and define a driver interface; and the eyelet can define an aperture. In some applications, the eyelet protrudes laterally with respect to the tissue-engaging portion.

In some applications, the eyelet is rotatably coupled to the tissue-engaging portion via the anchor head.

In some applications, the system and/or apparatus further includes a wire slidably coupled to the at least one tissue anchor and the wire is threaded through the aperture of the eyelet.

In some applications, the system and/or apparatus further includes an anchor driver that includes a driver head coupled to a flexible shaft. The driver head can be dimensioned to be axially slid within the lumen, and actuatable to reversibly engage the driver interface of the at least one tissue anchor. The anchor driver can be configured to, while the driver head is engaged with the driver interface: advance the anchor distally toward the distal opening of the catheter and drive the tissue-engaging portion along the tissue anchor axis into the tissue.

In some applications, the tissue anchor defines a tissue-anchor-lumen along the tissue anchor axis. The driver head of the anchor driver can be dimensioned to be axially slid within the tissue-anchor-lumen, and actuatable to reversibly engage the driver interface, and the flexible shaft of the anchor driver can be dimensioned to be axially slid within the tissue-anchor-lumen.

In some applications, the tubular wall of the channel is shaped so as to define a lateral slit extending along at least part of the channel at a distal portion thereof.

In some applications, the eyelet protrudes laterally with respect to the tissue-engaging portion so as to extend through the lateral slit.

There is yet additionally provided, in accordance with some applications, system and/or apparatus, including at least one tissue anchor and a catheter system. The catheter system can include an extracorporeal unit (e.g., a controller and/or handle) at a proximal portion of the catheter system, and a transluminally-advanceable catheter extending distally from the controller to a distal opening of the catheter.

The catheter can extend along a longitudinal catheter axis, from the controller to a distal opening of the catheter, e.g., in a straight configuration.

In some applications, the catheter includes a channel, including a generally tubular wall that defines a lumen along the catheter axis. The anchor can be disposed in the lumen. The tubular wall can be shaped so as to define at least one detent having a lateral detent-slit defining a moveable tab. A projection can be coupled to an inner surface of the moveable tab facing the lumen of the channel.

In some applications, an overtube is slidably disposed around the channel and operably coupled to the controller so as to be transitionable, via operation of the controller, such that the detent is transitionable between a closed state and an opened state.

In some applications, in the closed state: (i) the overtube surrounds the moveable tab, (ii) the moveable tab is in line with the tubular wall of the channel, and (iii) the projection extends into the lumen of the channel, obstructing the anchor from moving distally past the projection.

In some applications, in the opened state: (a) the overtube is retracted from the moveable tab, and (b) the moveable tab is displaceable away from the catheter axis to move the projection away from the catheter axis, enabling the anchor to move distally past the projection.

In some applications, the overtube is shaped so as to define a lateral window which is configured to align with the projection during the opened state in a manner which facilitates movement of the projection away from the catheter axis.

In some applications, the at least one anchor defines a first tissue anchor, and the system and/or apparatus further includes at least one additional tissue anchor, disposed within the lumen proximal to the first tissue anchor.

In some applications, the at least one detent defines a first detent, and the tubular wall is shaped so as to define at least one additional detent having a lateral additional-detent-slit defining an additional-detent-moveable-tab.

In some applications, the system and/or apparatus further includes an additional-detent-projection coupled to an inner surface of the additional-detent-moveable-tab facing the lumen of the channel.

In some applications, subsequently to the opened state of the first detent in which the first tissue anchor is moved distally past the projection of the first detent, the overtube is retractable distally such that the at least one additional detent is transitionable between a closed state and an opened state.

In some applications, in the closed state (i) the overtube surrounds the additional-detent-moveable-tab, (ii) the additional-detent-moveable-tab is in line with the tubular wall of the channel, and (iii) the additional-detent-projection extends into the lumen, obstructing the anchor from moving distally past the additional-detent-projection.

In some applications, in the opened state (i) the overtube is retracted from the additional-detent-moveable-tab, and (ii) the additional-detent-moveable-tab is displaceable away from the catheter axis to move the additional-detent-projection away from the catheter axis, enabling the anchor to move distally past the additional-detent-projection.

In some applications, the first tissue anchor is disposed in a first location with respect to the channel, and the additional tissue anchor is disposed in a second location with respect to the channel proximal to the first location.

In some applications, the system and/or apparatus further includes an anchor driver configured to (1) engage the first tissue anchor in the first location during the closed state of the first detent, and (2) engage the additional tissue anchor in the second location during the closed state of the additional detent.

In some applications, the at least one tissue anchor includes a tissue-engaging portion and an anchor head. In some applications, the tissue-engaging portion is disposed or positionable within the lumen of the channel. In some applications, the tissue-engaging portion defines a tissue anchor axis.

In some applications, the anchor head is coupled to the tissue-engaging portion and defines a driver interface. In some applications, the anchor head includes an eyelet defining an aperture. In some applications, the eyelet protrudes laterally with respect to the tissue-engaging portion.

In some applications, the eyelet is rotatably coupled to the tissue-engaging portion via the anchor head.

In some applications, the tissue-engaging portion is shaped so as to define a helical tissue-engaging portion, and the projection of the detent is configured to be disposed between adjacent subcomponents of the helical tissue-engaging portion in the closed state of the detent.

In some applications, the system and/or apparatus further includes a wire slidably coupled to the at least one tissue anchor and the wire is threaded through the aperture of the eyelet.

In some applications, the system and/or apparatus further includes an anchor driver that includes a driver head coupled to a flexible shaft.

In some applications, the driver head is dimensioned to be axially slid within the lumen and is actuatable to reversibly engage the driver interface of the at least one tissue anchor.

In some applications, the anchor driver is configured to, while the driver head is engaged with the driver interface, advance the anchor distally toward the distal opening of the catheter, and drive the tissue-engaging portion along the tissue anchor axis into the tissue.

In some applications, the tissue anchor defines a tissue-anchor-lumen along the tissue anchor axis, and: the driver head of the anchor driver is dimensioned to be axially slid within the tissue-anchor-lumen, and is actuatable to reversibly engage the driver interface, and the flexible shaft of the anchor driver is dimensioned to be axially slid within the tissue-anchor-lumen.

In some applications, the tubular wall of the channel is shaped so as to define a lateral slit extending along at least part of the channel at a distal portion thereof.

In some applications, the eyelet protrudes laterally with respect to the tissue-engaging portion so as to extend through the lateral slit.

There is further provided, in accordance with some applications, system and/or apparatus, including at least first and second tissue anchors and a catheter system. In some applications, the catheter system includes a transluminally-advanceable catheter extending distally from the controller. The catheter can extend along a longitudinal catheter axis from the controller to a distal opening of the catheter, e.g., when in a straight configuration.

In some applications, the catheter includes a channel and an extracorporeal unit (e.g., a controller and/or handle). In some applications, the channel includes a generally tubular wall that defines a lumen along the catheter axis for passage therethrough of the first and second tissue anchors.

In some applications, the extracorporeal unit is at a proximal portion of the catheter system and the extracorporeal unit includes a handle.

In some applications, the handle is shaped so as to define an anchor-driver sublumen having an anchor-driver-sublumen axis in alignment with the lumen of the channel of the transluminally-advanceable catheter and along the catheter axis.

In some applications, the handle is shaped so as to define an anchor-storage sublumen offset with respect to the lumen of the channel of the transluminally-advanceable catheter and with respect to the anchor-driver sublumen, the anchor-storage sublumen defining an anchor-storage zone housing the first and second tissue anchors.

In some applications, the handle includes a revolving stopper at a distal end of the handle, the revolving stopper shaped so as to define a receiving lumen to receive one of the first and second tissue anchors.

In some applications, the revolving stopper is revolvable about the catheter axis in a manner in which, in a receiving state of the revolving stopper, the receiving lumen of the revolving stopper is brought into alignment with the anchor-storage sublumen in order to receive the one of the first and second tissue anchors from the anchor-storage zone, and in a loaded state of the revolving stopper the receiving lumen of the revolving stopper is brought into alignment with the anchor-driver sublumen such that the one of the first and second tissue anchors is passable through the lumen of the channel of the transluminally-advanceable catheter.

In some applications, the anchor-storage sublumen and the anchor-driver sublumen are parallel.

In some applications, the system and/or apparatus further includes a spring disposed within the anchor-storage sublumen proximally to at least the second tissue anchor, the spring being configured to facilitate distal movement of the first and second tissue anchors.

In some applications, the anchor-storage sublumen and the anchor-driver sublumen are in fluid communication.

In some applications, each of the first and second tissue anchor includes a respective tissue-engaging portion defining a tissue anchor axis; a respective anchor head, coupled to the tissue-engaging portion, and defining a driver interface; and a respective eyelet defining an aperture, the respective eyelet protruding laterally with respect to the tissue-engaging portion.

In some applications, each eyelet is rotatably coupled to the respective tissue-engaging portion via the respective anchor head.

In some applications, the system and/or apparatus further includes a wire slidably coupled to the first and second tissue anchors and the wire is threaded through the apertures of the respective eyelets of the first and second tissue anchors.

In some applications, the wire is disposed between the anchor-driver sublumen and the anchor-storage sublumen.

In some applications, the wire extends within the lumen of the channel of the transluminally-advanceable catheter.

In some applications, each one of the first and second tissue anchors is revolvable by the revolving stopper about the wire.

In some applications, the system and/or apparatus further includes an anchor driver slidable in part within the anchor-driver sublumen and in part within the lumen of the channel of the transluminally-advanceable catheter, the anchor driver including a driver head coupled to a flexible shaft.

In some applications, the driver head is dimensioned to be axially slid within the anchor-driver sublumen and in part within the lumen of the channel of the transluminally-advanceable catheter and is actuatable to reversibly engage the driver interface of each one of the first and second tissue anchors.

In some applications, the anchor driver is configured to, while the driver head is engaged with the driver interface, advance the tissue anchor distally toward the distal opening of the catheter and drive the tissue-engaging portion along the tissue anchor axis into the tissue.

In some applications, the tissue anchor defines a tissue-anchor-lumen along the tissue anchor axis, and the driver head of the anchor driver is dimensioned to be axially slid within the tissue-anchor-lumen, and is actuatable to reversibly engage the driver interface, and the flexible shaft of the anchor driver is dimensioned to be axially slid within the tissue-anchor-lumen.

In some applications, the tubular wall of the channel is shaped so as to define a lateral slit extending along at least part of the channel at a distal portion thereof.

In some applications, each eyelet protrudes laterally with respect to the tissue-engaging portion so as to extend through the lateral slit.

There is further provided, in accordance with some applications, an anchor driver for use with a tissue anchor shaped so as to define a driver interface having a proximal rim and a recessed portion surrounded by a wall of the tissue anchor extending distally from the proximal rim.

In some applications, the anchor driver includes a flexible shaft and a driver head coupled to the flexible shaft. In some applications, the driver head is shaped so as to define first and second legs having a resting state in which the first and second legs define a first space between each other having a first distance and in which the first and second legs do not contact the wall of the tissue anchor surrounding the recessed portion.

In some applications, a connecting bar is disposed at least in part between the first and second legs.

In some applications, first and second expander elements are slidable with respect to the flexible shaft and with respect to the first and second legs, the first and second expander elements being disposed upstream of the connecting bar in the resting state of the first and second legs of the anchor driver. In some applications, the first and second expander elements can be configured to be or include one or more of wire(s), arm(s), rod(s), latch(es), lever(s), extension(s), expandable ring(s), expandable helix(es), etc. For example, in some applications, the first and second expander elements include first and second wires.

In some applications, the first and second expander elements are advanceable around the connecting bar and into the first space between the first and second legs to expand the first and second legs such that the first and second legs assume an engaged state in which the first and second legs define a second space between each other having a second distance greater than the first distance and in which the first and second legs contact and engage the wall of the tissue anchor surrounding the recessed portion.

In some applications, respective distal surfaces at each end of the first and second expander elements, in the engaged state of the first and second legs, push against the proximal rim of the tissue anchor to facilitate pushing of the tissue anchor in the engaged state of the first and second legs.

In some applications, the system and/or apparatus further includes an advancing element slidably coupled to the flexible shaft, the advancing element being coupled to the first and second expander elements and slidable with respect to the flexible shaft to push the first and second expander elements distally. In some applications, the advancing element can be configured to be or include one or more of a wire, rod, shaft, hypotube, extension, etc.

In some applications, the first and second expander elements expand along a plane that is perpendicular with respect to a plane along which the first and second legs expand.

In some applications, the anchor driver is dimensioned to be axially slid through a lumen defined by the tissue anchor in the resting state of the first and second legs and to selectively and reversibly engage the wall of the tissue anchor surrounding the recessed portion.

In some applications, the first and second expander elements are dimensioned to (a) assume a collapsed state during the resting state of the first and second legs, (b) expand around the connecting bar to transition the of the first and second legs into the engaged state of the first and second legs, and (c) subsequently be retractable to return the first and second expander elements to the collapsed state to disengage the anchor driver from the tissue anchor.

There is also provided, in accordance with some applications, an anchor driver for use with a tissue anchor shaped so as to define a driver interface having a proximal rim and a recessed portion surrounded by a wall of the tissue anchor extending distally from the proximal rim.

In some applications, the anchor driver includes a flexible advancing element and a driver head coupled to the flexible advancing element. In some applications, the advancing element can be configured to be or include one or more of a wire, rod, shaft, hypotube, extension, etc.

In some applications, the driver head is shaped so as to define first and second legs having a resting state in which the first and second legs define a first space between each other having a first distance and in which the first and second legs do not contact the wall of the tissue anchor surrounding the recessed portion.

In some applications, an expander element is slidable with respect to the flexible advancing element and between the first and second legs.

In some applications, the first and second expander elements are disposed at a distal surface of the first and second legs in the resting state of the first and second legs of the anchor driver.

In some applications, the first and second expander elements are advanceable proximally and into the first space between the first and second legs to expand the first and second legs such that the first and second legs assume an engaged state in which the first and second legs define a second space between each other having a second distance greater than the first distance and in which the first and second legs contact and engage the wall of the tissue anchor surrounding the recessed portion.

In some applications, in the resting state, the first and second legs collectively assume a circular profile, and in the engaged state, the first and second legs collectively assume an elliptical profile.

In some applications, the expander element includes a circular disc having a diameter that is larger than the first space between the first and second legs in the resting state such that the first and second legs expand to assume the engaged state when the expander element is positioned between the first and second legs.

In some applications, the expander element includes an elliptical disc having a major axis diameter that is larger than the first space between the first and second legs in the resting state such that the first and second legs expand to assume the engaged state when the expander element is positioned between the first and second legs.

In some applications, the system and/or apparatus further includes a displacing element slidably coupled to the advancing element, the displacing element being coupled to the expander element and slidable with respect to the advancing element to pull the expander element proximally to into the first space between the first and second legs.

In some applications, the anchor driver is dimensioned to be axially slid through a lumen defined by the tissue anchor and to selectively and reversibly engage the wall of the tissue anchor surrounding the recessed portion.

In some applications, the expander element is pushable distally to return the first and second legs to the to the resting state to disengage the anchor driver from the tissue anchor.

There is also provided, in accordance with some applications, an anchor driver for use with a tissue anchor shaped so as to define a driver interface having a proximal rim and a recessed portion surrounded by a wall of the tissue anchor extending distally from the proximal rim.

In some applications, the anchor driver includes a flexible tube and a driver head coupled to the flexible tube.

In some applications, the driver head is shaped so as to a deflectable protrusion having a resting state in which the protrusion assumes a collapsed state toward a central axis defined by the flexible tube and does not contact the tissue anchor.

In some applications, a shaft is disposed within a lumen of the flexible tube and engageable with the deflectable protrusion such that when the shaft slides alongside the deflectable protrusion, the shaft deflects the deflectable protrusion from the collapsed state, away from the central axis, and into an engaged state of the deflectable protrusion in which the deflectable protrusion engages with the tissue anchor.

In some applications, first and second expander elements coupled to an outer surface of the flexible tube and expandable proximally to the tissue anchor, the first and second expanded elements having respective distal surfaces at each end of the first and second expander elements which, in the engaged state of the deflectable protrusion, push against the proximal rim of the tissue anchor to facilitate pushing of the tissue anchor.

In some applications, the anchor driver is dimensioned to be axially slid through a lumen defined by the tissue anchor and to selectively and reversibly engage the tissue anchor responsively to movement of the shaft.

In some applications, the first and second expander elements are collapsible while being pulled through a lumen of the tissue anchor, and expandable once exposed from the lumen.

In some applications, the shaft is retractable away from the deflectable protrusion to facilitate deflecting of the deflectable protrusion from the engaged state, toward the central axis and into the collapsed state to disengage the anchor driver from the tissue anchor.

There is additionally provided, system and/or apparatus for use with a subject, including a catheter device, a tether, multiple tissue anchors, and an anchor driver.

In some applications, the catheter device includes a distal member or distal portion configured to be advanced into the subject and an extracorporeal proximal unit (e.g., a controller, handle, etc.) that includes a housing and a tensioner.

In some applications, the tether has a proximal portion that includes a proximal end of the tether. In some applications, the tether extends from the proximal portion, distally through the catheter device to a distal portion of the tether, the distal portion being disposed distally from the tensioner and including a distal end of the tether. In some applications, the tensioner is configured to pull proximally on the tether.

In some applications, the multiple tissue anchors each include a tissue-engaging element and a head that defines an eyelet that is threaded onto the tether such that the multiple tissue anchors are distributed in a series along the tether proximally from the tensioner.

In some applications, the anchor driver, for each of the anchors consecutively, is configured be reversibly coupled to the anchor and to advance the anchor along the tether, distally past the tensioner.

In some applications, the tensioner includes a spring that is coupled to the housing, and is reversibly lockable to the tether such that, while the spring is under stress and is locked to the tether, the spring causes the tensioner to pull proximally on the tether.

In some applications, the spring is a compression spring, is configured to be stressed by compression, and is configured to pull proximally on the tether by extending.

In some applications, the tensioner further includes a clamp, coupled to the spring, and reversibly transitionable between an unclamped state and a clamped state, the tether extending distally through the catheter device such that the tether extends through the clamp in a manner in which, when the clamp is in the clamped state, the spring is locked to the tether.

In some applications, the tensioner includes an actuator, actuation of the actuator transitioning the clamp between the unclamped state and the clamped state.

In some applications, the actuator includes a ring that circumscribes the clamp, rotation of the ring transitioning the clamp between the unclamped state and the clamped state.

In some applications, the clamp is biased toward assuming the unclamped state.

In some applications, the clamp and the ring collectively function as a chuck.

In some applications, the ring has an inner surface, and the clamp has an outer surface, and at least one surface selected from the group consisting of the inner surface of the ring and the outer surface of the clamp is tapered such that axial movement of the ring with respect to the clamp squeezes the clamp toward the clamped state.

In some applications, the ring has an inner screw thread, and the clamp has an outer screw thread, such that rotation of the ring screws the ring over the clamp.

In some applications, the anchor driver includes a shaft, and a driver head at a distal end of the shaft, the driver head being reversibly couplable to each of the tissue anchors. In some applications, the tether extends through the tensioner, thereby defining a tether axis, and the catheter device is shaped to provide a non-clamping zone laterally from the tether axis, the non-clamping zone being shaped and dimensioned to accommodate the shaft being shifted laterally after the driver has advanced a given one of the anchors along the tether, distally past the tensioner, such that the shaft extends distally toward the anchor, bypassing the clamp.

In some applications, the clamp defines a plurality of clamp surfaces, configured to engage the tether when the clamp is in the clamped state, and the catheter device is shaped to provide the non-clamping zone by the clamp surfaces being eccentric with respect to the tether axis.

In some applications, the clamp defines first and second clamp surfaces, configured to engage the tether when the clamp is in the clamped state, and in the unclamped state, the clamp defines a gap between the first and second clamp surfaces, each of the tissue anchors being dimensioned to be advanced, by the anchor driver, along the tether, distally through the gap.

In some applications, the anchor driver includes a shaft, and a driver head at a distal end of the shaft, the driver head being reversibly couplable to each of the tissue anchors, and for each of the anchors consecutively, the anchor driver is dimensioned to advance the anchor along the tether such that the anchor, the driver head, and at least a distal section of the shaft pass distally through the gap.

In some applications, the extracorporeal unit is shaped to define a non-clamping zone laterally from the clamp, the non-clamping zone being shaped and dimensioned to receive a part of the shaft that is disposed in the gap, upon the part of the shaft being moved laterally out of the gap and into the non-clamping zone.

In some applications, the extracorporeal unit includes a lock that is configured, when locked, to lock the spring in a stressed state in which the spring is under stress.

In some applications, the clamp is transitionable into the clamped state while the lock remains locked.

In some applications, the lock is configured, when locked, to lock the spring in the stressed state by locking the clamp to the housing.

In some applications, the lock is configured such that, while the clamp remains in the clamped state, unlocking of the clamp from the housing triggers the spring to cause the tensioner to pull proximally on the tether.

In some applications, the lock includes a detent and a recess.

In some applications, the clamp defines the recess, and the housing includes the detent.

In some applications, the clamp is transitionable into the clamped state by squeezing the clamp.

In some applications, the lock is configured such that, once the clamp is in the clamped state, further squeezing of the clamp unlocks the clamp from the housing, thereby triggering the spring to cause the tensioner to pull proximally on the tether.

There is further provided, in accordance with some applications, a system and/or an apparatus for use with a subject, including: a catheter device, a tether, multiple tissue anchors, and an anchor driver.

In some applications, the catheter device includes a distal member or distal portion configured to be advanced into the subject, and an extracorporeal unit at a proximal portion of the catheter device. In some applications, the extracorporeal unit includes a winch.

The winch can be configured to include a spring and a spool, coupled to the spring such that rotation of the spool in a first rotational direction applies stress to the spring.

In some applications, the tether has a distal portion that includes a distal end of the tether, and a proximal portion that includes a proximal end of the tether. In some applications, the proximal portion is wound around the spool such that pulling of the distal portion of the tether away from the winch unwinds the tether from the spool by rotating the spool in the first rotational direction.

In some applications, each of the multiple tissue anchors includes a tissue-engaging element and a head. In some applications, the head defines an eyelet.

In some applications, the eyelets of the multiple tissue anchors are threaded onto the tether such that the multiple tissue anchors are distributed in a series along the tether, e.g., between the spool and the distal end of the tether.

In some applications, the anchor driver, for each of the anchors consecutively, is configured be reversibly coupled to the anchor and to be used to advance the anchor, distally along the tether toward the distal end of the tether.

In some applications, the winch is configured to reduce slack in the tether by the spring, in response to the stress applied to the spring, applying tension to the tether by pushing the spool in a second rotational direction that is opposite to the first rotational direction.

In some applications, the winch further includes a reversibly-activatable ratchet that, while activated, allows rotation of the spool in the first rotational direction, and inhibits rotation of the spool in the second rotational direction and, while deactivated, allows rotation of the spool in both the first rotational direction and the second rotational direction.

In some applications, the ratchet includes a gear, coupled to the spool such that rotation of the spool rotates the gear; a pawl, reversibly engageable with the gear, such that when the pawl is engaged with the gear the ratchet is activated, and when the pawl is disengaged from the gear the ratchet is deactivated; and a switch, configured to switch the ratchet between being activated and deactivated by moving the pawl between being engaged with the gear and being disengaged from the gear.

In some applications, the system and/or apparatus further includes a stopper fixedly attached to the distal portion of the tether, the stopper obstructing advancement of any anchor of the series distally along the tether beyond the stopper, such that advancement, by the anchor driver, of a first tissue anchor of the series distally away from the winch pushes the stopper distally, which pulls the distal portion of the tether away from the winch.

In some applications, the distal member or distal portion includes a catheter that has a proximal opening, has a distal opening that is configured to be advanced into the subject, and defines a lumen between the proximal opening and the distal opening.

In some applications, the multiple tissue anchors are mounted in a series on the extracorporeal unit of the catheter device.

In some applications, the system and/or apparatus further includes a series of cartridges.

In some applications, each of the cartridges holds a respective tissue anchor of the multiple anchors or of the series of tissue anchors.

In some applications, each cartridge is coupled to the extracorporeal unit at a respective initial position of a series of initial positions. In some applications, each cartridge, while remaining coupled to the extracorporeal unit, is moveable from the respective initial position to a deployment position in which the cartridge holds the respective tissue anchor opposite the proximal opening. In some applications, each cartridge is subsequently removable from the deployment position such that the deployment position is vacant for a successive cartridge in the series.

In some applications, the anchor driver is, for each of the anchors, configured to be coupled to the anchor while the anchor is held by the respective cartridge opposite the proximal opening, and to advance the anchor distally out of the respective cartridge, through the proximal opening, and through the catheter toward the distal opening.

In some applications, the spool is mounted to rotate about a rotation axis, the lumen defines a lumen axis, and the rotation axis is parallel with the lumen axis.

In some applications, the spool is mounted to rotate about a rotation axis, the lumen defines a lumen axis, and the rotation axis is coaxial with the lumen axis.

In some applications, the spool circumscribes the catheter.

In some applications, the spool is concentric with the catheter.

The methods herein or methods of using the systems, apparatuses, devices, etc. described herein can be performed on a living animal or on a simulation, such as on a cadaver, cadaver heart, simulator (e.g., with the body parts, tissue, etc. being simulated), etc.

The present invention will be more fully understood from the following detailed description, taken together with the drawings, in which:

### BRIEF DESCRIPTION OF THE DRAWINGS

Figs. 1A-B and 2A-B are schematic illustrations of examples of a catheter system comprising a transluminally-advanceable catheter comprising a helical thread for controlling the advancement of tissue anchors, in accordance with some applications;
Figs. 3A-B and 4A-B are schematic illustrations of examples of a catheter system comprising a transluminally-advanceable catheter comprising a plurality of detents for controlling the advancement of tissue anchors, in accordance with some applications;
Figs. 5A-C are schematic illustrations of examples of a catheter system comprising a transluminally-advanceable catheter comprising a dispenser for controlling the advancement of tissue anchors, in accordance with some applications;
Figs. 6A-E are schematic illustrations of examples of a catheter system comprising a transluminally-advanceable catheter comprising a handle comprising a revolving stopper for controlling the advancement of tissue anchors, in accordance with some applications;
Figs. 7A-F, 8A-C, and 9A-B are schematic illustrations of respective examples of a catheter system comprising a transluminally-advanceable catheter, at least one tissue anchor, an implant, and a respective anchor driver for use in implanting the tissue anchor, in accordance with respective applications;
Figs. 10, 11A-B, 12A-B, 13A-E, 14A-E, and 15A-B are schematic illustrations of an example system for use with a subject, in accordance with some applications;
Figs. 16A-F are schematic illustrations of another system for use with a subject, in accordance with some applications;
Figs. 17A-F and 18A-B are schematic illustrations of an example system for use with a subject, in accordance with some applications;
Figs. 19A-B are schematic illustrations of a port, in accordance with some applications; and
Figs. 20A-H are schematic illustrations of an example system for use with a subject, in accordance with some applications.

### DETAILED DESCRIPTION

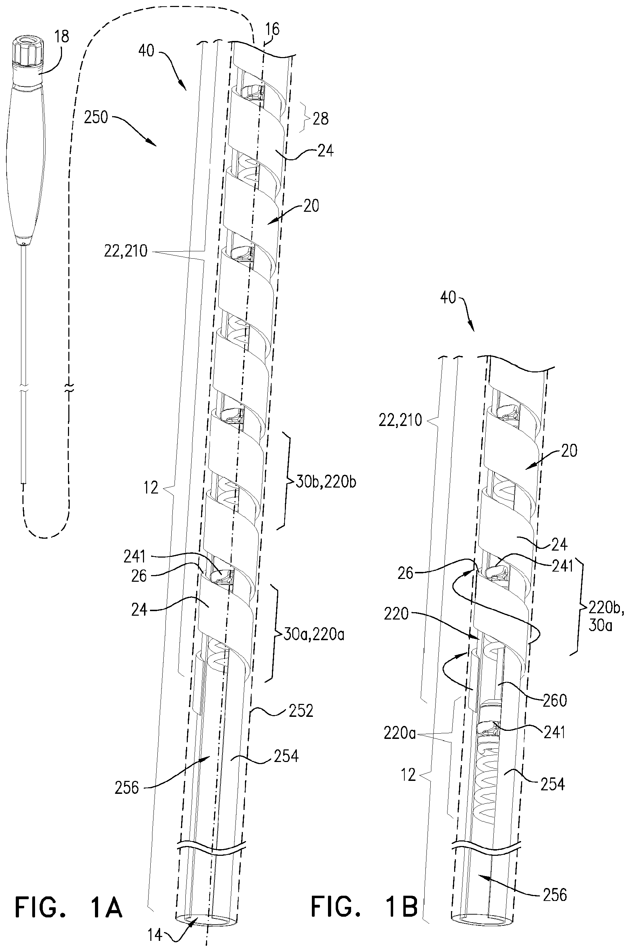

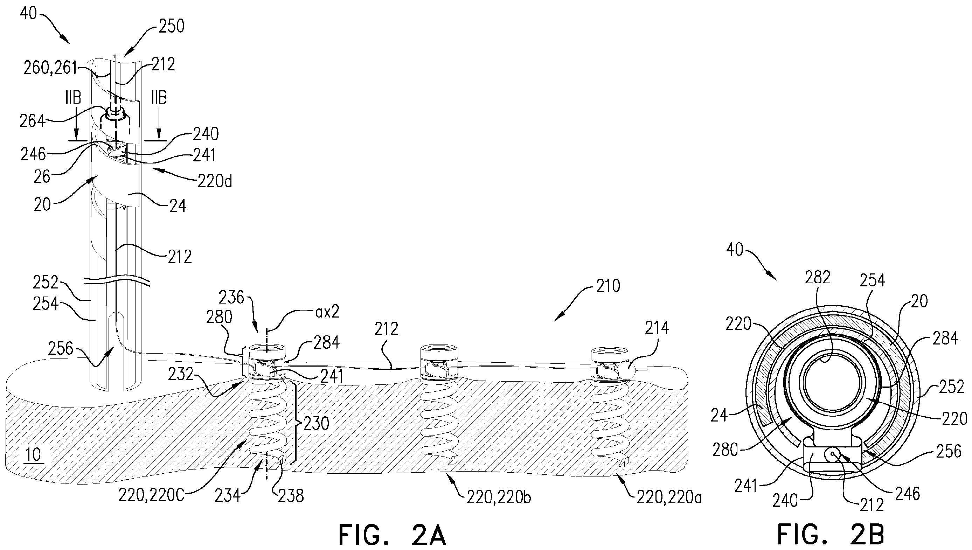

Reference is made to Figs. 1A-B and 2A-B, which are schematic illustrations of examples of a catheter system 40 comprising a transluminally-advanceable catheter 12, at least one tissue anchor 220, and an implant 210 comprising the tissue anchor, and techniques for use therewith, in accordance with some applications. System 40 is a tissue-adjustment system can be used for adjusting a dimension of a tissue structure. For example, system 40 can be an annuloplasty system, and implant 210 can be an annuloplasty structure.

System 40 comprises an extracorporeal unit (e.g., a controller and/or handle) 18 at a proximal portion of system 40. Transluminally-advanceable catheter 12 is coupled to extracorporeal unit 18 at a proximal end of catheter 12 and extends distally therefrom along a longitudinal catheter axis 16 of catheter 12, from unit 18 to a distal opening 14 of catheter 12. Transluminally-advanceable catheter 12 comprises a channel 254, e.g., a flexible tube, having a generally tubular wall that defines a lumen along catheter axis 16. The tubular wall of channel 254 is shaped so as to define a lateral slit 256 extending along at least part of channel 254 at a distal portion thereof. Slit 256 defines a linear slit disposed in parallel with axis 16. The tubular wall of channel 254 defines a longitudinal lumen along channel 254. For the portion the tubular wall of channel 254 that defines slit 256, the lumen of channel 254 at slit 256 is not closed due to slit 256.

Channel 254 is shaped so as to house implant 210 during delivery of implant 210 to tissue 10 of a patient. For applications in which tissue 10 represents tissue of the annulus of a native heart valve, such as the mitral valve, implant 210 is an annuloplasty structure comprising a wire 212 and multiple anchors 220. Each anchor 220 is shaped so as to define a central longitudinal anchor axis ax2. Each anchor 220 comprises a tissue-engaging element 230 and an anchor head 280. Tissue-engaging element 230 has a proximal end 232, a distal end 234, and defines central longitudinal axis ax2 of anchor 220. At distal end 234, tissue-engaging element 230 has a sharpened distal tip 238, and the tissue-engaging element is configured to be driven (e.g., screwed) into tissue of the subject. For some applications, and as shown, tissue-engaging element 230 is helical and defines a central lumen 436 along axis ax2. Optionally, tissue-engaging element 230 can be another type of tissue-engaging element, such as a dart or a staple.

Head 280 is coupled to proximal end 232 of tissue-engaging element 230 and comprises a driver interface 282 and an eyelet 240 that defines an aperture 246 therethrough. Driver interface 282 is configured to be reversibly engaged by a flexible anchor driver 260. In some applications, eyelet 240 comprises a structural element that protrudes laterally with respect to tissue-engaging element 230. Driver 260 can comprise an elongate and flexible shaft 261, and a driver head 264 coupled to a distal end of the shaft. Driver head 264 is the component of anchor driver 260 that reversibly engages driver interface 282. Driver interface 282 can be rigidly coupled to tissue-engaging element 230.

For some applications, driver interface 282 comprises a wall is shaped so as to define a recessed portion surrounded by a wall. For some applications, the wall surrounding the recessed portion is shaped as a circular wall, as shown in Fig. 2B. For some applications, the wall surrounding the recessed portion is square.

In some applications, and as shown, driver interface 282 is disposed on central longitudinal axis ax2, and eyelet 240 is disposed laterally from axis ax2.

As described in more detail hereinbelow, anchor 220 (e.g., eyelet 240 thereof) is configured to facilitate sliding of the anchor along wire 212 (or sliding of the wire through the anchor) while the anchor is aligned with the wire - e.g., while axis ax2 is parallel with the wire. This is hypothesized to facilitate transcatheter advancement of anchor 220 along the wire. As also described in more detail hereinbelow, anchor 220 (e.g., eyelet 240 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 240. This is hypothesized to be useful, inter alia, for applications in which the wire is tensioned after implantation in order to adjust anatomical dimensions, such as annuloplasty.

Transluminally-advanceable catheter 12 comprises a helical thread 20 overlapping with, e.g., coaxial with, channel 254 and operably connected to extracorporeal unit 18 such that, via operation of unit 18, thread 20 is rotatable about catheter axis 16 with respect to channel 254. Transluminally-advanceable catheter 12 has an anchor-storage zone 22 in which thread 20 is longitudinally at least partly coincident with slit 256 of channel 254. The coincidence of thread 20 and channel 254 defines anchor-storage zone 22. In some applications, thread 20 and channel 254 are concentrically disposed. The plurality of anchors 220 of implant 210 are disposed within anchor-storage zone 22. For some applications, transluminally-advanceable catheter 12 comprises an outer tube 252. For applications in which catheter 12 comprises outer tube 252, outer tube 252 is shaped so as to define a distal linear slit which extends from opening 14 of catheter 12 proximally and aligns with slit 256 of channel 254. For such applications, catheter 12 defines an inner tube disposed between outer tube 252 and channel 254, and thread 20 is coupled to a distal end of the inner tube in a manner in which rotation of the inner tube rotates thread 20. Often, as shown, thread 20 defines a tubular structure. For some applications, thread 20 is created from the inner tube by cutting away a helical slit in the inner tube. For either application in which thread 20 is coupled to a distal end of the inner tube or is formed from the inner tube, thread 20 is rotatable with respect to both channel 254 and outer tube 252. For some applications, catheter 12 does not comprise an inner tube, rather the inner surface of outer tube 252 defines a helical groove which defines thread 20 in a manner in which rotation of outer tube 252 facilitates rotation of thread 20 with respect to channel 254. For some applications, outer tube 252 comprises a polymer.

Anchor-storage zone 22 has a distal end that is disposed proximally to distal opening 14 of catheter 12. In such a manner, the distal portion of catheter 12 is devoid of anchors 220 to enable flexibility and manipulation of the end portion of catheter 12. The distal end of anchor-storage zone 22 can be at a distance of no more than 90 cm from distal opening 14, such as no more than 25 cm, to maintain the comfort and stability level of the user. For some applications, the distal end of zone 22 is at a distance of between 3 cm and 25 cm from distal opening 14, such as between 5 cm and 25 cm.

For some applications, and as shown, eyelet 240 is mounted to be revolvable around axis ax2. For example, head 280 can comprise a ring 284 on which eyelet 240 is mounted. Ring 284 circumscribes and is rotatable about axis ax2, e.g., by being rotatably coupled to tissue-engaging element 230, such as by being rotatably coupled to another component of head 280 (e.g., driver interface 282) that is fixedly coupled to the tissue-engaging element.

For some applications, and as shown, eyelet 240 is mounted to be stationary with respect to axis ax2. For such applications, head 280 does not comprise ring 284, but rather, eyelet 240 is mounted directly on to head 280.

Each tissue anchor 220 comprises a protrusion 241 coupled to tissue-engaging element 230 via anchor head 280. For some applications, protrusion 241 is coupled to or is shaped and extends from eyelet 240, e.g., from the structural element of eyelet 240. For some applications, protrusion 241 is shaped so as to define eyelet 240 defining aperture 246. For some applications, protrusion 241 is rotatably coupled to tissue-engaging element 230, e.g., by being coupled to ring 284. For other applications, head 280 does not comprise ring 284, but rather, protrusion 241 is mounted directly on to head 280. Protrusion 241 protrudes laterally with respect to tissue-engaging element 230 so as to extend through slit 256 of channel 254 and engage thread 20, such that (1) while thread 20 is stationary with respect to channel 254, tissue anchor 220 is secured within anchor-storage zone 22, and (2) rotation of thread 20 about catheter axis 16 with respect to channel 254 advances tissue anchor 220 distally until protrusion 241 exits a distal exit of thread 20.

Thread 20 is shaped so as to define a plurality of adjacent ribbons 24, or coils, arranged helically. The plurality of ribbons have a pitch 28 between adjacent ribbons. Each ribbon 24 has a surface 26 defining a depth of the ribbon 24. Protrusion 241 of each anchor 220 rests against a respective surface 26 of a given ribbon 24 and in pitch 28 between adjacent ribbons. During rotation of thread 20 about catheter axis 16, protrusion 241 of each anchor 220 slides along surface 26 of ribbon 24. As thread 20 rotates and protrusion 241 slides along surface 26, anchor 220 is advanced linearly distally along axis 16 of catheter 12. As shown in Fig. 1A, a first, distal-most tissue anchor 220a, prior to rotation of thread 20 and while thread 20 is stationary, is positioned at a first location 30a with respect to channel 254, distally to a second tissue anchor 220b that is disposed in a second location 30b proximal to first location 30a.

Rotation of thread 20 about catheter axis 16 with respect to channel 254, e.g., clockwise, as shown in Fig. 1B, (a) advances first tissue anchor 220a distally until protrusion 241 of first tissue anchor 220a exits a distal exit of thread 20 and first tissue anchor 220a is moved distally away from first location 30a and out of anchor-storage zone 22, and (b) advances second tissue anchor 220b distally away from second location 30b until second tissue anchor 220b is moved distally into first location 30a.

As each anchor moves distally, protrusion 241 and eyelet 240 advance distally linearly along slit 256 of channel 254. Each anchor 220 in anchor-storage zone 22 is disposed in a manner in which protrusions 241 and eyelet 240 are disposed within slit 256, while tissue-engaging element 230 and anchor head 280 are disposed within the lumen of channel 254. For some applications, catheter 12 comprises a spring (not shown), disposed within a lumen of catheter 12 proximally to at least second tissue anchor 220b, e.g., proximally to the plurality of anchors 220. For some applications, the spring is disposed proximally to anchor-storage zone 22. The spring is configured to facilitate distal movement of tissue anchors 220 and is arranged to apply a distally-directed force to the proximal-most anchor 220 within anchor-storage zone 22, thereby holding anchors 220 within storage zone 22, and helping to facilitate distal advancing of the remaining anchors 220 as thread 20 is rotated and each of anchors 220 is separately deployed.

It is to be noted that rotation of thread 20 by less than a complete turn, e.g., 180 degrees, provides the operating physician with an indication of the connection between driver 260 and anchor 220. Additionally, the interaction between thread 20 and anchors 220 functions as a safety mechanism to prevent premature release of an anchor 220.

Figs. 2A-B show system 40, which comprises implant 210, and a delivery tool 250 for percutaneous (e.g., transluminal, such as transfemoral) implantation of the implant. Tool 250 comprises flexible anchor driver 260 that is configured to reversibly engage driver interface 282 of anchor 220. Via this engagement, driver 260 is configured to drive tissue-engaging element 230 into tissue, e.g., by rotating (and distally pushing) anchor 220. In some applications, tool 250 further comprises flexible tube 252 (e.g., a transluminal catheter) via which each anchor 220, engaged with driver 260, is advanceable to the tissue to which the anchor is to be anchored.

In Fig. 2A, multiple anchors 220 have been anchored to tissue 10. Each anchor 220 was delivered to the tissue in a delivery state in which wire 212 extends through and is slidable with respect to aperture 246 of eyelet 240 while generally parallel to axis ax2. This is illustrated for an anchor 220d that is shown in Fig. 2A as being currently delivered. A straight path is possible for wire 212 to take through aperture 246 of eyelet 240.

As subsequent anchors 220 are anchored to the same tissue, wire 212 becomes oriented laterally with respect to the anchors. Due to the configuration of eyelet 240, despite this reorientation of wire 212, the wire can still take a clear straight path through aperture 246 of eyelet 240.

After a desired number of anchors 220 have been anchored, an adjustment tool is introduced (e.g., over and along a proximal portion of wire 212) and is used to facilitate tensioning of the wire. Therefore, the tensioning of wire 212 draws anchors 220 closer together, thereby contracting the tissue to which the anchors are anchored. This is facilitated by eyelets 240 providing smooth sliding of wire 212 through apertures 246 while the wire is orthogonal to the anchors, as described hereinabove. Respective stoppers 214 are coupled to wire 212 to prevent wire 212 from sliding out of anchors 220. Fig. 2A shows a stopper distal to first anchor 220a. Excess wire 212 can then be cut and removed from the patient.

For simplicity, Fig. 2A shows implant 210 in a linear configuration. However, for annuloplasty, implant 210 is often implanted in a curve (or even a complete ring) around the valve annulus, such that the contraction reduces the size of the valve annulus, improving coaptation of the valve leaflets.

As described hereinabove, for some applications eyelet 240 is mounted to be revolvable around axis ax2. This therefore provides independence between the rotational position of the eyelet and that of tissue-engaging element 230. It is hypothesized that, for applications in which tissue-engaging element 230 is helical, this independence advantageously allows the tissue-engaging element to be screwed into tissue to the extend needed for optimal anchoring, without a requirement for the anchor to finish in a particular rotational orientation. It is further hypothesized that, irrespective of the type of tissue-engaging element 230 used, this independence allows eyelet 240 (and wire 212) to be in an optimal position, with respect to axis ax2 of each anchor 220, for a given application. For example, for an application in which implant 210 is used for annuloplasty, anchors 220 are often anchored in a curve around the valve annulus, and eyelets 240 and wire 212 can be disposed on the inside of the curve relative to axis ax2.

To anchor anchor 220, the anchor is advanced out of a distal end of channel 254 and of tube 252 while driver 260 rotates driver interface 282 (and thereby tissue-engaging element 230) with respect to the tube, and while slit 256 can inhibit rotation of ring 284 with respect to the tube. For some applications, it is advantageous for the distal end of tube 252 to be disposed (or even pressed) against tissue 10 during anchoring of the anchor, e.g., as shown in Fig. 2A. For applications in which tube 252 is used to implant an implant comprising multiple anchors on a wire, such as implant 210, interference may occur between the wire and the contact between the distal end of the tube and the tissue. For some applications, tube 252 defines a lateral slit extending proximally from the distal end of tube 252 and channel 254 defines slit 256 extending proximally from the distal end of channel 254, such that the slits are continuous with distal opening 14 of catheter 12. For some applications, the slits are adjacent to (e.g., laterally outward from) the lumen of catheter 12, and allows wire 212, but not anchor 220, to exit tube 252 and channel 254 laterally, proximally from distal opening 14. It is believed that this facilitates implantation of implants such as implant 210, comprising multiple anchors coupled to (e.g., threaded on) a wire.

Each anchor 220 is shaped so as to define a respective tissue-anchor-lumen extending through a longitudinal length of anchor 220 from a proximal end of anchor head 280 to a distal end of tissue-engaging element 230. Anchor driver 260, e.g., particularly driver head 264, is dimensioned to be axially slid within the lumen of channel 254 and is actuatable to reversibly engage the driver interface 282 of each tissue anchor within anchor-storage zone 22. Anchor driver 260 is configured to, while driver head 264 is engaged with driver interface 282, advance anchor 220 distally away from anchor-storage zone 22, toward distal opening 14 of catheter 12, and drive tissue-engaging element 230 along tissue anchor axis ax2 into tissue 10. Driver 260 advances tissue anchor 220 distally together with rotation of thread 20. In the absence of rotation of thread 20, anchor driver 260 is prevented from advancing anchor 220 distally.

Each anchor 220 defines the respective tissue-anchor-lumen, and anchor driver 260, e.g., particularly driver head 264, is dimensioned to be axially slid within all of the tissue-anchor-lumens of anchors 220. For example, when the distal-most anchor 220 is engaged by driver 260, driver 260 passes through the tissue-anchor-lumens of all of anchors 220 until it reaches anchors head 280 of the distal-most anchor. Once driver 260 implants the distal-most anchor 220, it is disengaged from anchor 220 and retracted through the lumen of channel 254 and through the tissue-anchor-lumen of the next anchor 220 in sequence that was disposed proximally adjacent to the distal-most anchor. In order to engage anchor head 280 of the next anchor 220 in sequence, driver 260 is retracted through the tissue-anchor-lumen of the next anchor 220 in sequence until it is in line with anchor head 280 to engage head 280 and thereby engage the next anchor 220 in sequence.

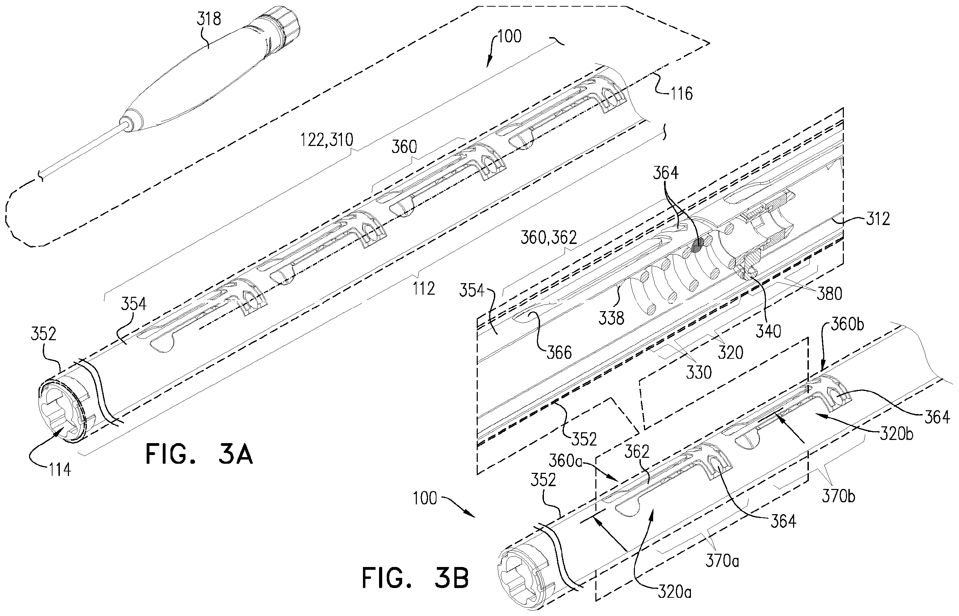

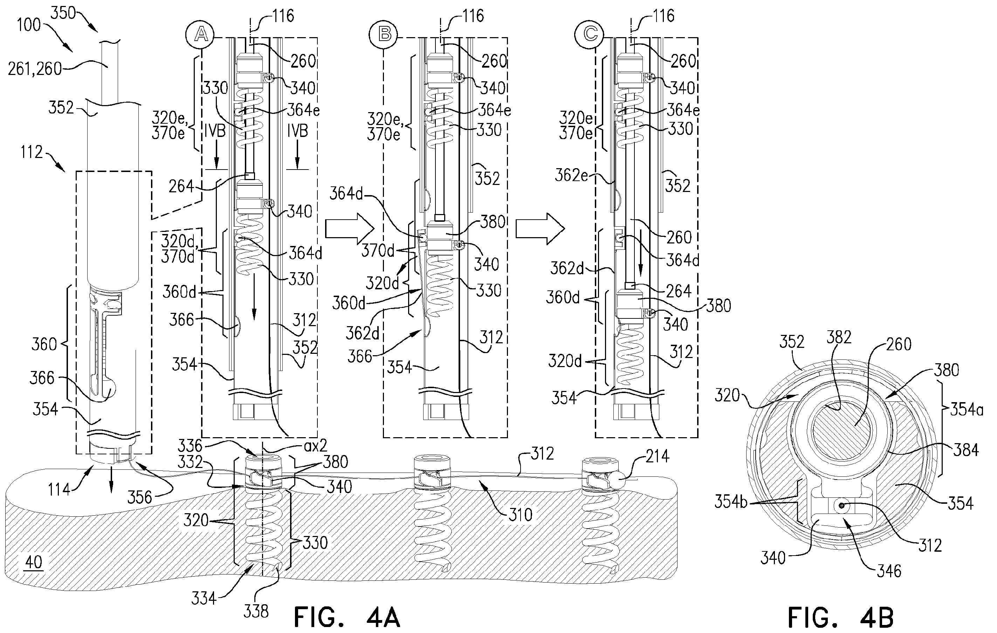

Reference is made to Figs. 3A-B and 4A-B, which are schematic illustrations of examples of a catheter system 100 comprising a transluminally-advanceable catheter 112, at least one tissue anchor 320, and an implant 310 comprising the tissue anchor, and techniques for use therewith, in accordance with some applications. System 100 is a tissue-adjustment system and can be used for adjusting a dimension of a tissue structure. For example, system 100 can be an annuloplasty system, and implant 310 can be an annuloplasty structure.

System 100 comprises an extracorporeal unit 318 (e.g., a controller and/or handle) at a proximal portion of system 100. Transluminally-advanceable catheter 112 is coupled to extracorporeal unit 318 at a proximal end of catheter 112 and extends distally therefrom along a longitudinal catheter axis 116 of catheter 112, from unit 318 to a distal opening 114 of catheter 112. Transluminally-advanceable catheter 112 comprises a channel 354, e.g., a flexible tube, having a generally tubular wall that defines a lumen along catheter axis 116. The tubular wall of channel 354 is shaped so as to define a lateral slit 356 extending along at least part of channel 354 at a distal portion thereof. Slit 356 defines a linear slit disposed in parallel with axis 116. The tubular wall of channel 354 defines a longitudinal lumen along channel 354. For the portion the tubular wall of channel 354 that defines slit 356, the lumen of channel 354 at slit 356 is not closed due to slit 356.

Channel 354 is shaped so as to house implant 310 during delivery of implant 310 to tissue 10 of a patient. For applications in which tissue 10 represents tissue of the annulus of a native heart valve, such as the mitral valve, implant 310 is an annuloplasty structure comprising a wire 312 and multiple anchors 320. Each anchor 320 is shaped so as to define a central longitudinal anchor axis ax2. Each anchor 320 comprises a tissue-engaging element 330 and an anchor head 380. Tissue-engaging element 330 has a proximal end 332, a distal end 334, and defines central longitudinal axis ax2 of anchor 320. At distal end 334, tissue-engaging element 330 has a sharpened distal tip 338, and the tissue-engaging element is configured to be driven (e.g., screwed) into tissue of the subject. For some applications, and as shown, tissue-engaging element 330 is helical and defines a central lumen 336 along axis ax2. Optionally, tissue-engaging element 330 can be another type of tissue-engaging element, such as a dart or a staple. Head 380 is coupled to proximal end 332 of tissue-engaging element 330 and comprises a driver interface 382 and an eyelet 340 that defines an aperture 246 therethrough. Driver interface 382 is configured to be reversibly engaged by a flexible anchor driver 260. In some applications, eyelet 340 comprises a structural element that protrudes laterally with respect to tissue-engaging element 330. Driver 260 can comprise an elongate and flexible shaft 261, and a driver head 264 coupled to a distal end of the shaft. Driver head 264 is the component of anchor driver 260 that reversibly engages driver interface 382. Driver interface 382 can be rigidly coupled to tissue-engaging element 330.

For some applications, driver interface 382 comprises a wall is shaped so as to define a recessed portion surrounded by a wall. For some applications, the wall surrounding the recessed portion is shaped as a circular wall, as shown in Fig. 4B. For some applications, the wall surrounding the recessed portion is square.

In some applications, and as shown, driver interface 382 is disposed on central longitudinal axis ax2, and eyelet 340 is disposed laterally from axis ax2.

As described in more detail hereinbelow, anchor 320 (e.g., eyelet 340 thereof) is configured to facilitate sliding of the anchor along wire 312 (or sliding of the wire through the anchor) while the anchor is aligned with the wire - e.g., while axis ax2 is parallel with the wire. This is hypothesized to facilitate transcatheter advancement of anchor 320 along the wire. As also described in more detail hereinbelow, anchor 320 (e.g., eyelet 340 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 340. This is hypothesized to be useful, *inter alia,* for applications in which the wire is tensioned after implantation in order to adjust anatomical dimensions, such as annuloplasty.

The tubular wall of channel 354 of transluminally-advanceable catheter 112 is shaped so as to define at least one detent 360 having a lateral detent-slit 366 defining a moveable tab 362. In some applications, as shown, channel 354 of catheter is shaped so as to define a plurality of detents 360 arranged longitudinally in parallel with axis 116. Transluminally-advanceable catheter 112 has an anchor-storage zone 122 in which the plurality of anchors 320 are disposed and held in place by the plurality of detents 360. For some applications, anchor-storage zone 122 is longitudinally at least partly coincident with slit 356 of channel 354. Transluminally-advanceable catheter 112 comprises an overtube 352. In some applications, overtube 352 is shaped so as to define a distal linear slit which extends from opening 114 of catheter 112 proximally and aligns with slit 356 of channel 354.

Anchor-storage zone 122 has a distal end that is disposed proximally to distal opening 114 of catheter 112. In such a manner, the distal portion of catheter 112 is devoid of anchors 320 to enable flexibility and manipulation of the end portion of catheter 112. The distal end of anchor-storage zone 122 is often at a distance of no more than 90 cm from distal opening 114, such as no more than 25 cm, to maintain the comfort and stability level of the user. For some applications, the distal end of zone 122 is at a distance of between 3 cm and 25 cm from distal opening 114, such as between 5 cm and 25 cm.

For some applications, and as shown, eyelet 340 is mounted to be revolvable around axis ax2. For example, head 380 can comprise a ring 384 on which eyelet 340 is mounted. Ring 384 circumscribes and is rotatable about axis ax2, e.g., by being rotatably coupled to tissue-engaging element 330, such as by being rotatably coupled to another component of head 380 (e.g., driver interface 382) that is fixedly coupled to the tissue-engaging element.

For some applications, and as shown, eyelet 340 is mounted to be stationary with respect to axis ax2. For such applications, head 380 does not comprise ring 384, but rather, eyelet 340 is mounted directly on to head 380.

As described hereinabove, the tubular wall of channel 354 of transluminally-advanceable catheter 112 is shaped so as to define at least one detent 360 having a lateral detent-slit 366 defining a moveable tab 362. In some applications, as shown, channel 354 of catheter is shaped so as to define a plurality of detents 360 arranged longitudinally in parallel with axis 116. Tab 362 is coupled at an inner surface thereof to a projection 364 which faces the lumen of channel 354. For some applications, tab 362 is shaped at an inner surface thereof so as to define projection 364. For some application, projection 364 functions as a baffle and a stop which inhibits movement of tissue anchor 320 linearly within the lumen of channel 354. For some applications, as shown, projection 364 is shaped so as to define a cylinder which is disposed perpendicularly with respect to axis 116 of catheter 112. As shown, projection 364 is coupled to tab 362 at a proximal portion of tab 362. Projection 364 is shaped so as to fit between adjacent coils of tissue-engaging element 330 of anchor 320, as shown in the enlarged image of Fig. 3B. When no force is applied to projection 364, projection 364 remains disposed between the adjacent coils of tissue-engaging element 330. Pulling or pushing on anchor 320 facilitates displacement of projection 364 and at least the proximal portion of tab 362 radially away from axis 116 of catheter 112, moving projection 364 out from between the adjacent coils of tissue-engaging element 330, thereby unlocking anchor 320 from detent 360, as is described hereinbelow. Additionally, when tube over352 surrounds detent 360, tab 362 is restricted from being moved radially away from axis 116.

Overtube 352 is slidably disposed around channel 354 and operably coupled to unit 318 so as to be transitionable, via operation of unit 318, such that each detent 360 is transitionable between (1) a closed state, in which (i) overtube 352 surrounds moveable tab 362, (ii) moveable tab 362 is in line with the tubular wall of channel 354, and (iii) projection 364 extends into the lumen of channel 354, obstructing anchor 320 from moving distally past projection 364, and (2) an opened state, in which (i) overtube 352 (i.e., the distal end of outer tube 252) is retracted from moveable tab 362, and (ii) moveable tab 362 is displaceable away from catheter axis 116 to move projection 364 away from catheter axis 116, enabling anchor 320 to move distally past projection 364.

For some applications (not shown) overtube 352 is shaped so as to define a lateral window which is configured to align with projection 364 during the opened state of detent 360 in a manner which facilitates movement of projection 364 away from the catheter axis. For such applications, the distal end of overtube 352 need not be retracted proximally to detent 360, rather, the lateral window of overtube aligns with detent 360 (in particular, with the portion of overtube 352 coupled to projection 364) in order to facilitate movement of projection 364 radially away from axis 116. For some application, overtube 352 is not retracted, but rather rotated around axis 116 in order to align the lateral window with detent 360.

As shown in Fig. 3B, a first, distal-most tissue anchor 320a, is positioned at a first location 370a with respect to channel 354, distally to a second tissue anchor 320b that is disposed in a second location 370b proximal to first location 370a. In system 100, each anchor 320 is held in its respective location and is secured in place as long as overtube 352 surrounds detent 360.

Once each anchor 320 is freed from its respective detent 360, anchor 320 is moved distally away from first its respective location 370 and out of anchor-storage zone 122. A long as overtube 352 surrounds detent 360, anchor 320 is locked in place within its respective location 370.

As each anchor 320 moves distally, eyelet 340 advance distally linearly along slit 356 of channel 354. Each anchor 320 in anchor-storage zone 122 is disposed in a manner in which eyelets 340 are disposed opposite detents 360. Projections 364 maintain each anchor 320 fixed in a manner in which eyelet 340 is disposed opposite detent 360 so as to prevent rotation of anchor 320 and twisting of wire 312. As such, for some applications, ring 384 is fixed with respect to anchor head 380 and does not revolve around axis ax2. For some applications, slit 356 of channel 354 extends proximally into anchor-storage zone 122 so as to maintain eyelet 340 within slit 356 and thereby prevent rotation of anchor 320 and entangling of wire 312.

Figs. 4A-B show system 100, which comprises implant 310, and a delivery tool 350 for percutaneous (e.g., transluminal, such as transfemoral) implantation of the implant. Tool 350 comprises flexible anchor driver 260 that is configured to reversibly engage driver interface 382 of anchor 320. Via this engagement, driver 260 is configured to drive tissue-engaging element 330 into tissue, e.g., by rotating (and distally pushing) anchor 320. In some applications, tool 350 further comprises flexible overtube 352 (e.g., a transluminal catheter) via which each anchor 320, engaged with driver 260, is advanceable to the tissue to which the anchor is to be anchored.

In Fig. 4A, multiple anchors 320 have been anchored to tissue 10. Each anchor 320 was delivered to the tissue in a delivery state in which wire 312 extends through and is slidable with respect to aperture 346 of eyelet 340 while generally parallel to axis ax2. This is illustrated for an anchor 320d that is shown in Fig. 4A as being currently delivered. A straight path is possible for wire 312 to take through aperture 346 of eyelet 340.

As subsequent anchors 320 are anchored to the same tissue, wire 312 becomes oriented laterally with respect to the anchors. Due to the configuration of eyelet 340, despite this reorientation of wire 312, the wire can still take a clear straight path through aperture 346 of eyelet 340.

After a desired number of anchors 320 have been anchored, an adjustment tool is introduced (e.g., over and along a proximal portion of wire 312) and is used to facilitate tensioning of the wire. Therefore, the tensioning of wire 312 draws anchors 320 closer together, thereby contracting the tissue to which the anchors are anchored. This is facilitated by eyelets 240 providing smooth sliding of wire 312 through apertures 346 while the wire is orthogonal to the anchors, as described hereinabove. Respective stoppers 214 are coupled to wire 312 to prevent wire 312 from sliding out of anchors 320. Fig. 4A shows a stopper distal to first anchor 320a. Excess wire 312 can then be cut and removed from the patient.

For simplicity, Fig. 4A shows implant 310 in a linear configuration. However, for annuloplasty, implant 310 is often implanted in a curve (or even a complete ring) around the valve annulus, such that the contraction reduces the size of the valve annulus, improving coaptation of the valve leaflets.

As described hereinabove, for some applications eyelet 340 is mounted to be revolvable around axis ax2. This therefore provides independence between the rotational position of the eyelet and that of tissue-engaging element 330. It is hypothesized that, for applications in which tissue-engaging element 330 is helical, this independence advantageously allows the tissue-engaging element to be screwed into tissue to the extend needed for optimal anchoring, without a requirement for the anchor to finish in a particular rotational orientation. It is further hypothesized that, irrespective of the type of tissue-engaging element 330 used, this independence allows eyelet 340 (and wire 312) to be in an optimal position, with respect to axis ax2 of each anchor 320, for a given application. For example, for an application in which implant 310 is used for annuloplasty, anchors 320 are often anchored in a curve around the valve annulus, and eyelets 340 and wire 312 can be disposed on the inside of the curve relative to axis ax2.

To anchor anchor 320, the anchor is advanced out of a distal end of channel 354 and of overtube 352 while driver 260 rotates driver interface 382 (and thereby tissue-engaging element 330) with respect to the tube, and while slit 356 can inhibit rotation of ring 384 with respect to the tube. For some applications, it is advantageous for the distal end of overtube 352 to be disposed (or even pressed) against tissue 10 during anchoring of the anchor, e.g., as shown in Fig. 4A. As described hereinabove, slit 356 of channel 354 is adjacent to (e.g., laterally outward from) the lumen of catheter 112, and allows wire 312, but not anchor 320, to exit tube channel 354 laterally, proximally from distal opening 114. It is believed that this facilitates implantation of implants such as implant 310, comprising multiple anchors coupled to (e.g., threaded on) a wire.

Each anchor 320 is shaped so as to define a respective tissue-anchor-lumen extending through a longitudinal length of anchor 320 from a proximal end of anchor head 380 to a distal end of tissue-engaging element 330. Anchor driver 260, e.g., particularly driver head 264, is dimensioned to be axially slid within the lumen of channel 354 and is actuatable to reversibly engage the driver interface 382 of each tissue anchor within anchor-storage zone 122. Anchor driver 260 is configured to, while driver head 264 is engaged with driver interface 382, advance anchor 320 distally away from anchor-storage zone 122, toward distal opening 114 of catheter 112, and drive tissue-engaging element 330 along tissue anchor axis ax2 into tissue 10. Driver 260 advances tissue anchor 320 distally only once the distal end of overtube 352 is retracted proximally to detent 360 currently holding tissue anchor 320. For applications in which overtube 352 defines the lateral window, driver 260 advances tissue anchor 320 distally only once the lateral window of overtube 352 aligns with detent 360 currently holding tissue anchor 320. If overtube 352 covers detent 360, anchor driver 260 is prevented from advancing anchor 320 distally since projection 364 of detent 360 remains engaged with tissue-engaging element 330 of anchor 320.

Each anchor 320 defines the respective tissue-anchor-lumen, and anchor driver 260, e.g., particularly driver head 264, is dimensioned to be axially slid within all of the tissue-anchor-lumens of anchors 320. For example, when the distal-most anchor 320 is engaged by driver 260, driver 260 passes through the tissue-anchor-lumens of all of anchors 320 until it reaches anchors head 380 of the distal-most anchor. Once driver 260 implants the distal-most anchor 320, it is disengaged from anchor 320 and retracted through the lumen of channel 354 and through the tissue-anchor-lumen of the next anchor 320 in sequence that was disposed proximally adjacent to the distal-most anchor. In order to engage anchor head 380 of the next anchor 320 in sequence, driver 260 is retracted through the tissue-anchor-lumen of the next anchor 320 in sequence until it is in line with anchor head 380 to engage head 380 and thereby engage the next anchor 320 in sequence.

As shown in Fig. 4A, a series of anchors 320 have been deployed at tissue 10. In Step A, anchor driver 260 engages the next proximally-disposed tissue anchor 320d from its respective location 370d in anchor-storage zone 122. Anchor 320d is locked in place in location 370d since overtube 352 surrounds detent 360d in a manner in which projection 364d of tab 362d of detent 360d is disposed between coils of tissue-engaging element 330. In Step B, overtube 352 is retracted such that its distal end is disposed proximal to tab 362d thereby freeing detent 360d and allowing for lateral and radial displacement of tab 362d away from axis 116 during distal pushing of anchor 320d by anchor driver 260. Such distal pushing of anchor 320d pushes tissue-engaging element 330d and anchor head 380 against projection 364d to push projection 364d out of the lumen of channel 354. In such a manner, detent 360d assumes the opened state in which tab 362d is not in line with the tubular wall of channel 354 and projection 364d is displaced away from axis 116. The next proximally-disposed anchor 320e is held in place in location 370e despite movement of anchor 320d out of location 370d. Once anchor 320d has been moved distally from location 370d, there is no object and/or force acting on tab 362d, and therefore, tab 362d returns to a position in which tab 362 is in line with the wall of channel 354 and projection 364d again projects within the lumen of channel 354. As shown, anchor 330e remains locked in location 370e since overtube 352 surrounds detent 360e.

In Fig. 4B, for some applications, channel 354 is shaped to control, during delivery and anchoring, a rotational position of eyelet 340 with respect to axis ax2 and/or tissue-engaging element 330. For some such applications, channel 354 defines a major channel region 354a and a minor channel region 354b. Major channel-region 354a has a larger cross-sectional area than does minor channel region 354b. Anchor 320 is slidable through channel 354 with tissue-engaging element 330 sliding (often snugly) through primary channel region 354a, and eyelet 340 sliding (often snugly) through minor channel region 354b and along wire 312. Rotational control of channel 354 thereby controls the position of eyelet 340, and therefore of wire 312, around axis ax2 of each anchor. While driver interface 382 and tissue-engaging element 330 are rotatable within overtube 352 and within channel 354, ring 384 and eyelet 340 are not. For some applications, and as shown, channel 354 has a keyhole-shaped orthogonal cross-section.

For some applications, eyelet 340 comprises or is shaped so as to define protrusion 241 described hereinabove with reference to Figs. 1A-B and 2A-B.

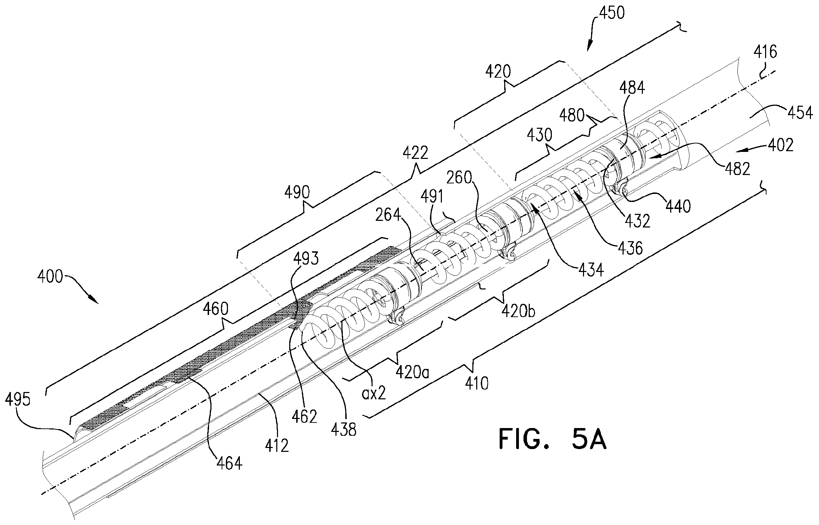

Reference is made to Figs. 5A-C, which are schematic illustrations of examples of a catheter system 400 comprising a transluminally-advanceable catheter 402, at least one tissue anchor 420, and an implant 410 comprising the tissue anchor, and techniques for use therewith, in accordance with some applications. System 400 is a tissue-adjustment system and can be used for adjusting a dimension of a tissue structure. For example, system 400 can be an annuloplasty system, and implant 410 can be an annuloplasty structure.

At a proximal portion of system 400, the system comprises an extracorporeal unit (e.g., a controller and/or handle), which can be similar or identical to extracorporeal unit 18 and/or 318 described hereinabove. Transluminally-advanceable catheter 402 is coupled to the extracorporeal unit at a proximal end of catheter 402 and extends distally therefrom along a longitudinal catheter axis 16 of catheter 402, from the extracorporeal unit to a distal opening of catheter 402. Transluminally-advanceable catheter 402 comprises a channel 454, e.g., a flexible tube, having a generally tubular wall that defines a lumen along catheter axis 416. The tubular wall of channel 454 is shaped so as to define a lateral slit (e.g., as described hereinabove with reference to slit 256 of channel 254 of Figs. 1A-B and 2A-B) extending along at least part of channel 454 at a distal portion thereof. The slit defines a linear slit disposed in parallel with axis 416. The tubular wall of channel 454 defines a longitudinal lumen along channel 454. For the portion the tubular wall of channel 454 that defines the slit, the lumen of channel 454 at the slit is not closed due to the slit.

Channel 454 is shaped so as to house implant 410 during delivery of implant 410 to tissue 10 of a patient. For applications in which tissue 10 represents tissue of the annulus of a native heart valve, such as the mitral valve, implant 410 is an annuloplasty structure comprising a wire 412 and multiple anchors 420. Each anchor 420 is shaped so as to define a central longitudinal anchor axis ax2. Each anchor 420 comprises a tissue-engaging element 430 and an anchor head 480. Tissue-engaging element 430 has a proximal end 432, a distal end 434, and defines central longitudinal axis ax2 of anchor 420. At distal end 434, tissue-engaging element 430 has a sharpened distal tip 438, and the tissue-engaging element is configured to be driven (e.g., screwed) into tissue of the subject. For some applications, and as shown, tissue-engaging element 430 is helical and defines a central lumen 436 along axis ax2. Optionally, tissue-engaging element 430 can be another type of tissue-engaging element, such as a dart or a staple. Head 480 is coupled to proximal end 432 of tissue-engaging element 430 and comprises a driver interface 482 and an eyelet 440 that defines an aperture therethrough. Driver interface 482 is configured to be reversibly engaged by a flexible anchor driver 260. In some applications, eyelet 440 comprises a structural element that protrudes laterally with respect to tissue-engaging element 430. Driver 260 can comprise an elongate and a flexible shaft, and a driver head 264 coupled to a distal end of the shaft. Driver head 264 is the component of anchor driver 260 that reversibly engages driver interface 482. Driver interface 482 can be rigidly coupled to tissue-engaging element 430.

For some applications, driver interface 482 comprises a wall is shaped so as to define a recessed portion surrounded by a wall. For some applications, the wall surrounding the recessed portion is shaped as a circular wall. For some applications, the wall surrounding the recessed portion is square.

In some applications, and as shown, driver interface 482 is disposed on central longitudinal axis ax2, and eyelet 440 is disposed laterally from axis ax2.

As described in more detail hereinbelow, anchor 420 (e.g., eyelet 440 thereof) is configured to facilitate sliding of the anchor along wire 412 (or sliding of the wire through the anchor) while the anchor is aligned with the wire - e.g., while axis ax2 is parallel with the wire. This is hypothesized to facilitate transcatheter advancement of anchor 420 along the wire. As also described in more detail hereinbelow, anchor 420 (e.g., eyelet 440 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 440. This is hypothesized to be useful, inter alia, for applications in which the wire is tensioned after implantation in order to adjust anatomical dimensions, such as annuloplasty.

Transluminally-advanceable catheter 402 comprises a dispenser 460 distal to a distal-most anchor 420a. Dispenser 460 is overlapping with, e.g., coaxial with, channel 454 and operably connected to the extracorporeal unit such that, via operation of the extracorporeal unit, dispenser 460 is moveable with respect to channel 454 to facilitate dispensing of one anchor 420 at a time. Transluminally-advanceable catheter 402 has an anchor-storage zone 422 comprising dispenser 460 and a plurality of tissue anchors 420. For some applications, anchor-storage zone 422 is longitudinally at least partly coincident with the slit of channel 454. In some applications, dispenser 460 and channel 454 are concentrically disposed. The plurality of anchors 420 of implant 410 are disposed within anchor-storage zone 422. For some applications, transluminally-advanceable catheter 402 comprises an outer tube. For applications in which catheter 402 comprises the outer tube, the dispenser is positioned between and slidable with respect to the outer tube and channel 454.

Anchor-storage zone 422 has a distal end that is disposed proximally to the distal opening of catheter 402. In such a manner, the distal portion of catheter 402 is devoid of anchors 420 to enable flexibility and manipulation of the end portion of catheter 402. The distal end of anchor-storage zone 422 is often at a distance of no more than 90 cm from the distal opening, such as no more than 25 cm, to maintain the comfort and stability level of the user. For some applications, the distal end of zone 422 is at a distance of between 3 cm and 25 cm from the distal opening, such as between 5 cm and 25 cm.

For some applications, and as shown, eyelet 440 is mounted to be revolvable around axis ax2. For example, head 480 can comprise a ring 484 on which eyelet 440 is mounted. Ring 284 circumscribes and is rotatable about axis ax2, e.g., by being rotatably coupled to tissue-engaging element 430, such as by being rotatably coupled to another component of head 480 (e.g., driver interface 482) that is fixedly coupled to the tissue-engaging element.

For some applications, and as shown, eyelet 440 is mounted to be stationary with respect to axis ax2. For such applications, head 480 does not comprise ring 484, but rather, eyelet 440 is mounted directly on to head 480.

For some applications, each tissue anchor 420 comprises a protrusion 241 (described hereinabove with reference to Figs. 1A-B and 2A-B) coupled to or is shaped and extends from eyelet 440, e.g., from the structural element of eyelet 440.

Dispenser 460 comprises a structural element comprising a proximal detent 462 and a distal detent 464. Detents 462 and 464 are transitionable between states in which they protrude into the lumen of channel 454 toward catheter axis 416 and in which they are collapsed away from catheter axis 426 to free the lumen of channel 456 from their obstruction. For some applications, a tubular structure 495 surrounds at least a portion of channel 454 and is slidable with respect to channel 454. Tubular structure 495 is coupled to dispenser 460 in a manner in which movement of tubular structure 495 longitudinally distally and proximally along channel 454 facilitates collapsing of proximal and distal detents 462 and 464 and extending of the proximal and distal detents 462 and 464 into the lumen. The tubular wall of channel 454 is shaped so as to define a lateral window 490 out of the lumen. Lateral window 490 has a proximal edge 491 and a distal edge 493. Tubular structure 495 surround the at least the portion of channel 454 at lateral window 490. For some applications, catheter 402 comprises outer tube disposed around tubular structure 495 in a manner in which tubular structure 495 is disposed between the outer tube and channel 454.

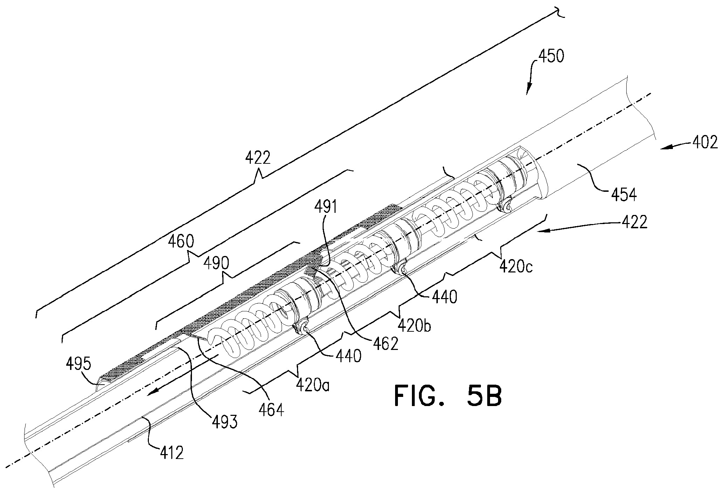

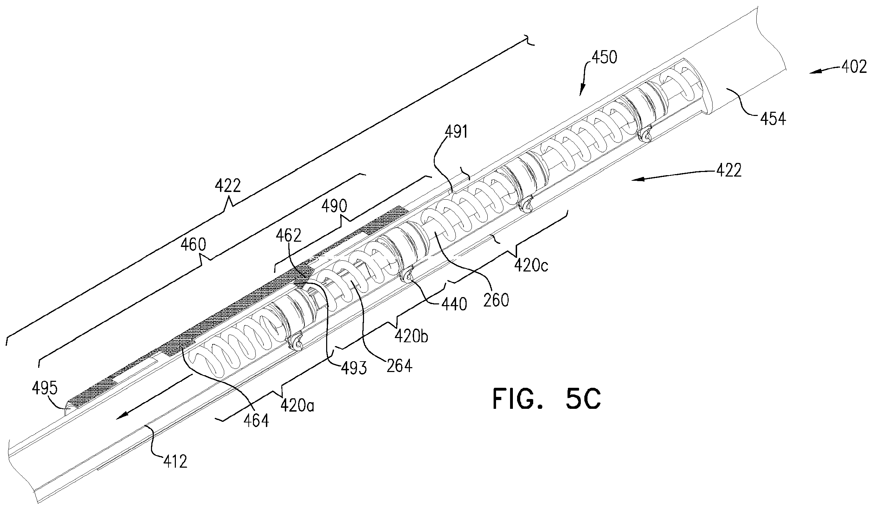

Dispenser 460 is operably coupled to the extracorporeal unit so as to be transitionable, via operation of the extracorporeal unit, between (1) a receiving state, in which (i) proximal detent 462 is collapsed away from catheter axis 416, permitting anchor 420a to move distally past proximal detent 462 and into dispenser 460, and (ii) distal detent 464 extends into the lumen, obstructing anchor 420a from moving distally past distal detent 464 and out of dispenser 460, (2) a closed state (Fig. 5B), in which (i) proximal detent 462 extends into the lumen, obstructing anchor 420a from returning proximally past proximal detent 462 and out of dispenser 460, and (ii) distal detent 464 extends into the lumen, obstructing anchor 420a from moving distally past distal detent 464 and out of dispenser 460, and (3) a dispensing state (Fig. 5C), in which (i) the proximal detent 462 extends into the lumen, obstructing anchor 420a from returning proximally past proximal detent 462 and out of dispenser 460, and distal detent 464 is collapsed away from catheter axis 416, permitting anchor 420a to move distally past distal detent 464 and out of dispenser 460.

In Fig. 5A, anchor 420a has not been moved into dispenser 460. Anchor 420a is coupled to anchor driver 260 and is moveable by driver 260 due to sliding movement of driver 260 within the lumen of channel 454. For some applications, anchor 420a is moveable within dispenser 460 by movement of driver 260 distally as driver 260 is coupled to anchor 420. Movement of anchor 420a distally past proximal detent 462 collapses detent 462. Once anchor 420a has been moved past detent 464, detent 464 returns to an extended state within the lumen of channel 454, prohibiting proximal retraction of anchor 420a. For some application, tubular structure 495 is moved proximally with respect to lateral window 490 of channel 454 in order to facilitate collapsing of proximal detent 462 against proximal edge 491 of window 490 such that proximal detent 462 moves away from catheter axis 416 and is retracted, or collapsed, with respect to axis 416 such that detent 462 does not obstruct the lumen of channel 454, and anchor 420a is properly positioned within dispenser 460 in the receiving state and is aligned with window 490. Once anchor 420a is positioned within dispenser 460, tubular structure 495 is moved distally such that proximal detent 462 is not collapsed against proximal edge 491 of window 490 and detent 462 protrudes and extends within the lumen of channel 454, as shown in Fig. 5B.

Distal detent 464 is no longer collapsed and retracted by distal edge 493 of window 490 and also protrudes and extends within the lumen of channel 454 such that dispenser 460 is in the closed state, obstructing anchor 420a from moving distally past distal detent 464 and from moving proximally past proximal detent 462. In such a manner, the operating physician can assess appropriate coupling between driver 260 and anchor 420, in response to lack of movement of anchor 420a in response to pulling on or pushing driver 260 in the closed state of dispenser 460. Additionally, when detents 462 and 464 protrude from their collapsed states, they make a "click" sound, and the operating physician receives tactile feedback from the clicking in to place of detents 462 and 464.

As tubular structure 495 is moved proximally and distally, the proximal handle of anchor driver 260 moves accordingly, and the physician is able to visualize movement of driver 260.

In Fig. 5C, tubular structure 495 is moved distally with respect to lateral window 490 facilitates collapsing of distal detent 464 against distal edge 493 of window 490 such that dispenser 460 assumes the dispensing state in which distal detent 464 moves away from catheter axis 416 while proximal detent 462 extends into the lumen of channel 454 obstructing anchor 420a from returning proximally past proximal detent 462. Driver 260 moves distally together with distal movement of tubular structure 495 in order to advance anchor 420a distally out of dispenser 460 and out of anchor-storage zone 422.

Once anchor 420a is moved distally out of dispenser 460, tubular structure 495 is retracted proximally in order to position the next anchor 420b in sequence within dispenser 460. Dispenser 460 returns to the receiving state in which dispenser 460 permits one additional anchor 420b to move distally past proximal detent 462 (since detent 462 is retracted by proximal edge 491). Dispenser 460 then transitions into the closed state wherein dispenser 460 obstructs the at least one additional anchor 420b from moving distally past distal detent 464 and out of dispenser 460. Subsequently, dispenser 460 transitions to the dispensing state, wherein dispenser 460 permits the at least one additional tissue anchor 420b to move distally past distal detent 464 and out of dispenser 460.

For some applications, catheter 402 comprises a spring (not shown), disposed within a lumen of catheter 402 proximally to at least second tissue anchor 420b, e.g., proximally to the plurality of anchors 420. For some applications, the spring is disposed proximally to anchor-storage zone 422. The spring is configured to facilitate distal movement of tissue anchors 420 and is arranged to apply a distally-directed force to the proximal-most anchor 420 within anchor-storage zone 422, thereby holding anchors 420 within storage zone 422, and helping to facilitate distal advancing of the remaining anchors 420 as each of anchors 420 is separately deployed.

As each anchor 420 moves distally, eyelet 440 advances distally linearly along the slit of channel 454. Each anchor 420 in anchor-storage zone 422 is disposed in a manner in which eyelet 440 is disposed within the slit, while tissue-engaging element 430 and anchor head 480 are disposed within the lumen of channel 454. For some applications, the slit does not extend within anchor-storage zone 422 and the entirety of anchor 420 is disposed within the lumen of channel 454 while disposed within anchor-storage zone 422.

Figs. 5A-C show system 400, which comprises implant 410, and a delivery tool 450 for percutaneous (e.g., transluminal, such as transfemoral) implantation of the implant. Tool 450 comprises flexible anchor driver 260 that is configured to reversibly engage driver interface 482 of anchor 420. Via this engagement, driver 260 is configured to drive tissue-engaging element 430 into tissue, e.g., by rotating (and distally pushing) anchor 420. In some applications, tool 450 further comprises a flexible tube (e.g., a transluminal catheter) via which each anchor 420, engaged with driver 260, is advanceable to the tissue to which the anchor is to be anchored.

Each anchor 420 is delivered to the tissue in a delivery state in which wire 412 extends through and is slidable with respect to the aperture of eyelet 440 while generally parallel to axis ax2.

As subsequent anchors 420 are anchored to the same tissue, wire 412 becomes oriented laterally with respect to the anchors. Due to the configuration of eyelet 440, despite this reorientation of wire 412, the wire can still take a clear straight path through aperture 246 of eyelet 440.

After a desired number of anchors 420 have been anchored, an adjustment tool is introduced (e.g., over and along a proximal portion of wire 412) and is used to facilitate tensioning of the wire. Therefore, the tensioning of wire 412 draws anchors 420 closer together, thereby contracting the tissue to which the anchors are anchored. This is facilitated by eyelets 440 providing smooth sliding of wire 412 through the apertures of eyelets 440 while the wire is orthogonal to the anchors, as described hereinabove. Respective stoppers 214 are coupled to wire 412 to prevent wire 412 from sliding out of anchors 420. Excess wire 412 can then be cut and removed from the patient.

As described hereinabove, for some applications eyelet 440 is mounted to be revolvable around axis ax2. This therefore provides independence between the rotational position of the eyelet and that of tissue-engaging element 430. It is hypothesized that, for applications in which tissue-engaging element 430 is helical, this independence advantageously allows the tissue-engaging element to be screwed into tissue to the extend needed for optimal anchoring, without a requirement for the anchor to finish in a particular rotational orientation. It is further hypothesized that, irrespective of the type of tissue-engaging element 430 used, this independence allows eyelet 440 (and wire 412) to be in an optimal position, with respect to axis ax2 of each anchor 420, for a given application. For example, for an application in which implant 410 is used for annuloplasty, anchors 420 are often anchored in a curve around the valve annulus, and eyelets 440 and wire 412 can be disposed on the inside of the curve relative to axis ax2.

To anchor anchor 420, the anchor is advanced out of a distal end of channel 454 while driver 260 rotates driver interface 482 (and thereby tissue-engaging element 430) with respect to the tube, and while the slit of channel 454 can inhibit rotation of ring 484 with respect to the tube. For some applications, it is advantageous for the distal end of catheter 402 to be disposed (or even pressed) against tissue 10 during anchoring of the anchor. The lateral slit extending proximally from the distal end of catheter 402 allows wire 412, but not anchor 420, to exit channel 454 laterally, proximally from the distal opening of catheter 402. It is believed that this facilitates implantation of implants such as implant 410, comprising multiple anchors coupled to (e.g., threaded on) a wire.

Each anchor 420 is shaped so as to define a respective tissue-anchor-lumen extending through a longitudinal length of anchor 420 from a proximal end of anchor head 480 to a distal end of tissue-engaging element 430. Anchor driver 260, e.g., particularly driver head 264, is dimensioned to be axially slid within the lumen of channel 454 and is actuatable to reversibly engage the driver interface 482 of each tissue anchor within anchor-storage zone 422. Anchor driver 260 is configured to, while driver head 264 is engaged with driver interface 482, advance anchor 420 distally away from anchor-storage zone 422, toward the distal opening of catheter 402, and drive tissue-engaging element 430 along tissue anchor axis ax2 into tissue 10. Driver 260 advances tissue anchor 420 distally together with rotation of thread 20. In the absence of closed state of dispenser 460 as shown in Fig. 5B, anchor driver 260 is prevented from advancing anchor 420 distally.

Each anchor 420 defines the respective tissue-anchor-lumen, and anchor driver 260, e.g., particularly driver head 264, is dimensioned to be axially slid within all of the tissue-anchor-lumens of anchors 420. For example, when the distal-most anchor 420 is engaged by driver 260, driver 260 passes through the tissue-anchor-lumens of all of anchors 420 until it reaches anchors head 480 of the distal-most anchor. Once driver 260 implants the distal-most anchor 420, it is disengaged from anchor 420 and retracted through the lumen of channel 454 and through the tissue-anchor-lumen of the next anchor 420 in sequence that was disposed proximally adjacent to the distal-most anchor. In order to engage anchor head 480 of the next anchor 420 in sequence, driver 260 is retracted through the tissue-anchor-lumen of the next anchor 420 in sequence until it is in line with anchor head 480 to engage head 480 and thereby engage the next anchor 420 in sequence.

As each anchor 420 moves distally, eyelet 440 advance distally linearly along the slit of channel 454. Each anchor 420 in anchor-storage zone 422 is disposed in a manner in which eyelets 440 are disposed opposite dispenser 460. Detents 462 and 464 maintain each anchor 420 fixed in a manner in which eyelet 440 is disposed opposite dispenser 460 so as to prevent rotation of anchor 420 and twisting of wire 312. As such, for some applications, ring 484 is fixed with respect to anchor head 480 and does not revolve around axis ax2. For some applications, the slit of channel 454 extends proximally into anchor-storage zone 422 so as to maintain eyelet 440 within the slit and thereby prevent rotation of anchor 420 and entangling of wire 412.

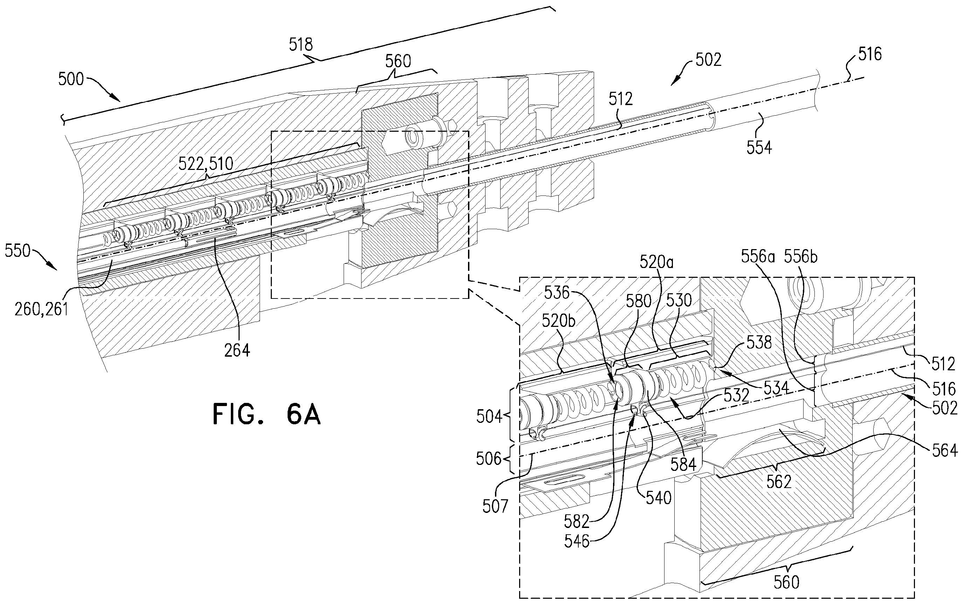

Reference is made to Figs. 6A-E, which are schematic illustrations of examples of a catheter system 500 comprising a transluminally-advanceable catheter 502, at least one tissue anchor 520, and an implant 510 comprising the tissue anchor, and techniques for use therewith, in accordance with some applications. System 500 is a tissue-adjustment system and can be used for adjusting a dimension of a tissue structure. For example, system 500 can be an annuloplasty system, and implant 510 cam be an annuloplasty structure.

System 500 comprises an extracorporeal unit 518 (e.g., a controller and/or a handle), at a proximal portion of system 500. Transluminally-advanceable catheter 502 is coupled to extracorporeal unit 518 at a proximal end of catheter 502 and extends distally therefrom along a longitudinal catheter axis 516 of catheter 502, from unit 518 to a distal opening of catheter 502. Transluminally-advanceable catheter 502 comprises a channel 554, e.g., a flexible tube, having a generally tubular wall that defines a lumen along catheter axis 516. The tubular wall of channel 554 is shaped so as to define a lateral slit extending along at least part of channel 554 at a distal portion thereof. The slit defines a linear slit disposed in parallel with axis 516. The tubular wall of channel 554 defines a longitudinal lumen along channel 554 that is in alignment with catheter axis 516. For the portion the tubular wall of channel 554 that defines the slit, the lumen of channel 554 at the slit is not closed due to the slit.

Extracorporeal unit 518 and channel 554 are shaped so as to define respective lumens which house implant 510 during delivery of implant 510 to tissue 10 of a patient. For applications in which tissue 10 represents tissue of the annulus of a native heart valve, such as the mitral valve, implant 510 is an annuloplasty structure comprising a wire 512 and multiple anchors 520. Each anchor 520 is shaped so as to define a central longitudinal anchor axis ax2. Each anchor 520 comprises a tissue-engaging element 530 and an anchor head 580. Tissue-engaging element 530 has a proximal end 532, a distal end 534, and defines central longitudinal axis ax2 of anchor 520. At distal end 534, tissue-engaging element 530 has a sharpened distal tip 538, and the tissue-engaging element is configured to be driven (e.g., screwed) into tissue of the subject. For some applications, and as shown, tissue-engaging element 530 is helical and defines a central lumen 536 along axis ax2. Optionally, tissue-engaging element 530 can be another type of tissue-engaging element, such as a dart or a staple. Head 580 is coupled to proximal end 532 of tissue-engaging element 530 and comprises a driver interface 582 and an eyelet 540 that defines an aperture 546 therethrough. Driver interface 582 is configured to be reversibly engaged by a flexible anchor driver 260. In some applications, eyelet 540 comprises a structural element that protrudes laterally with respect to tissue-engaging element 530. Driver 260 can comprise an elongate and flexible shaft 261, and a driver head 264 coupled to a distal end of the shaft. Driver head 264 is the component of anchor driver 260 that reversibly engages driver interface 582. Driver interface 582 can be rigidly coupled to tissue-engaging element 530.

For some applications, driver interface 582 comprises a wall is shaped so as to define a recessed portion surrounded by a wall. For some applications, the wall surrounding the recessed portion is shaped as a circular wall, as shown. For some applications, the wall surrounding the recessed portion is square.

In some applications, and as shown, driver interface 582 is disposed on central longitudinal axis ax2, and eyelet 540 is disposed laterally from axis ax2.

As described in more detail hereinbelow, anchor 520 (e.g., eyelet 540 thereof) is configured to facilitate sliding of the anchor along wire 512 (or sliding of the wire through the anchor) while the anchor is aligned with the wire - e.g., while axis ax2 is parallel with the wire. This is hypothesized to facilitate transcatheter advancement of anchor 520 along the wire. As also described in more detail hereinbelow, anchor 520 (e.g., eyelet 540 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 540. This is hypothesized to be useful, inter alia, for applications in which the wire is tensioned after implantation in order to adjust anatomical dimensions, such as annuloplasty.

For some applications, and as shown, eyelet 540 is mounted to be revolvable around axis ax2. For example, head 580 can comprise a ring 584 on which eyelet 540 is mounted. Ring 584 circumscribes and is rotatable about axis ax2, e.g., by being rotatably coupled to tissue-engaging element 530, such as by being rotatably coupled to another component of head 580 (e.g., driver interface 582) that is fixedly coupled to the tissue-engaging element.

For some applications, and as shown, eyelet 540 is mounted to be stationary with respect to axis ax2. For such applications, head 580 does not comprise ring 584, but rather, eyelet 540 is mounted directly on to head 580.

The handle of extracorporeal unit 518 shaped so as to define two sublumens: (1) an anchor-driver sublumen 506 having an anchor-driver-sublumen axis 507 in alignment with the lumen of channel 554 of transluminally-advanceable catheter 502 and along catheter axis 516, and (2) an anchor-storage sublumen 504 offset with respect to the lumen of channel 554 of transluminally-advanceable catheter 502 and offset with respect to anchor-driver sublumen 506. In some applications, sublumens 504 and 506 are in fluid communication and are parallel with respect to each other. Anchor-storage sublumen 504 defines an anchor-storage zone 522 housing a plurality of tissue anchors 520. The plurality of anchors 520 are lined up sequentially within zone 522. Wire 512 and each eyelet 540 of anchors 520 is positioned in a space between anchor-storage sublumen 504 and anchor-driver sublumen 506. That is, when tissue anchor 520 is disposed within anchor storage zone 522 and within anchor-storage sublumen 504, tissue-engaging element 530 and anchor head 580 are disposed within anchor-storage sublumen 504 while eyelet 540 and wire 512 are disposed in the space between sublumens 504 and 506. Wire 512 extends from anchor-driver sublumen distally through the lumen of channel 554 toward the distal opening of catheter 502.

Prior to engaging any of anchors 520, anchor driver 260 is disposed and slidable within anchor-driver sublumen 506, as shown in Fig. 6A.

The handle of extracorporeal unit 518 comprises a revolving stopper 560 at a distal end of the handle. Revolving stopper 560 is shaped so as to define a receiving lumen 562 to receive one tissue anchors 520 at a time. Revolving stopper is revolvable about catheter axis 516 in a manner in which, during a first stopping state, as shown in Fig. 6A, stopper is in a position in which receiving lumen 562 does not align with anchor-storage sublumen 504, but rather, a wall of stopper 560 blocks the distal end of sublumen 504, thereby preventing distal movement of any anchor 520.

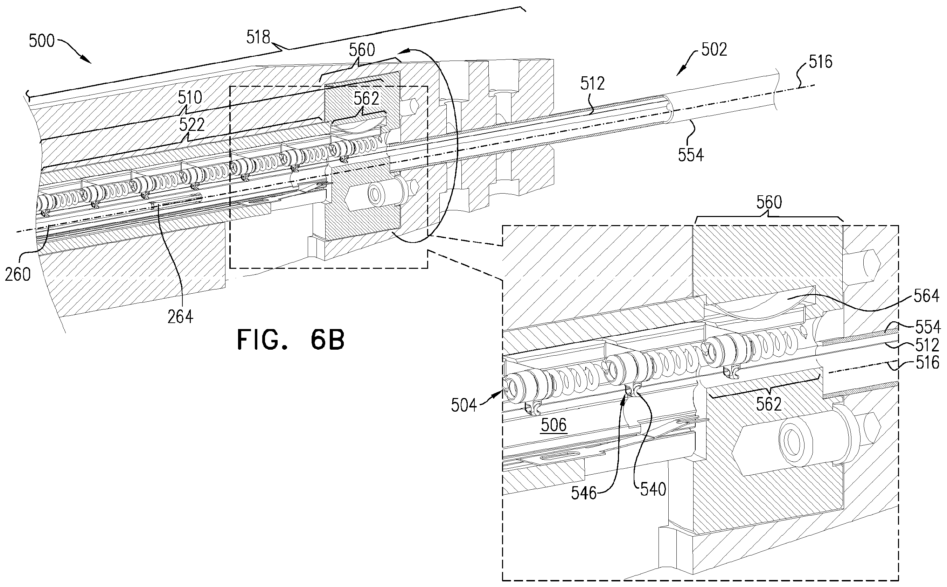

As shown in Fig. 6B, revolving stopper 560 is revolved 180 degrees about central axis 516 of catheter 502 and about wire 512 so as to bring receiving lumen 562 of revolving stopper 560 into alignment with anchor-storage sublumen 504 in order to receive a first tissue anchor 520a anchor-storage zone 522, thereby defining a receiving state of revolving stopper 560. During the revolving of stopper 560, wire 512 remains stationary. Revolving of stopper 560 about wire 512 prevents tangling of wire 512. Stopper 560 comprises a spring 564 disposed within receiving lumen 562 so as to fasten anchor 520a in place during revolving of revolving stopper 560

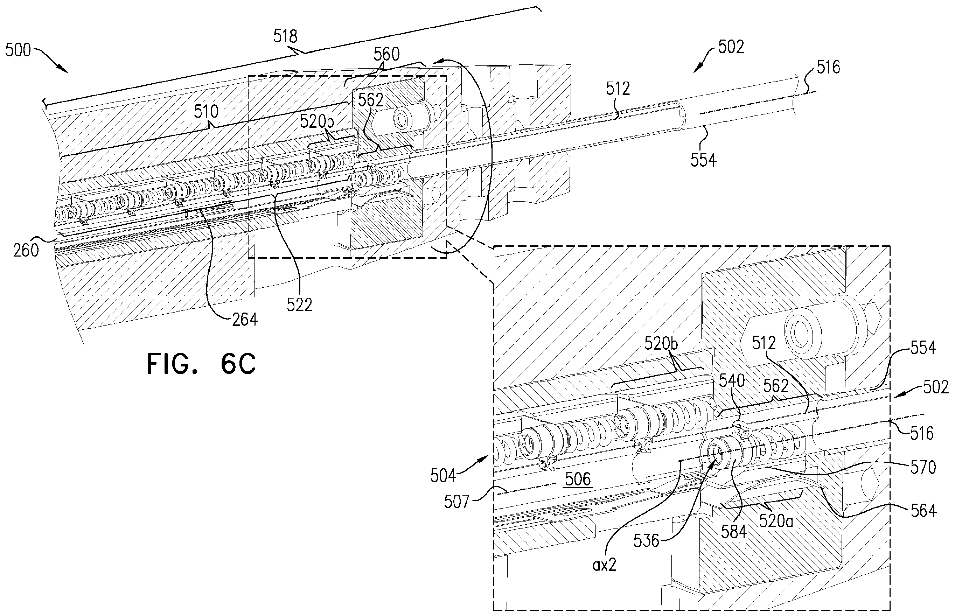

In Fig. 6C, revolving stopper 560 is revolved again about central axis 516 of catheter 502 in order to bring revolving stopper 560 into a loaded state in which receiving lumen 562 of revolving stopper 560 is brought into alignment with anchor-driver sublumen 506 such that tissue anchor 520a is passable through the lumen of channel 554 of transluminally-advanceable catheter 502. For some applications, revolving stopper 560 is revolved an additional 180 degrees in the same direction as described hereinabove with reference to Fig. 6B, i.e., such that revolving stopper completes a full 360 revolution about axis 516 from the stopping state shown in Fig. 6A to the loaded state shown in Fig. 6C. For some applications, revolving stopper 560 is revolved an additional 180 degrees in the opposite direction as described hereinabove with reference to Fig. 6B, i.e., such that revolving stopper toggles back and forth 180 degrees between the stopping state shown in Fig. 6A to the loaded state shown in Fig. 6C.

Since each anchor 520 is slidably coupled to wire 512 via eyelet 540, as revolving stopper revolves about axis 516, tissue anchor 520a disposed within receiving lumen 562 revolves about wire 512. In the loaded state, as shown in Fig. 6C, tissue-engaging element 530 and anchor head 580 are disposed in alignment with anchor-driver sublumen 506 and with the lumen of channel 554. That is, central lumen 536 of tissue-engaging element 530 is disposed (a) along axis ax2 of anchor 520, (b) in alignment with the lumen of channel 554 of transluminally-advanceable catheter 502 along axis 516 of catheter 502, and (c) in alignment with anchor-driver sublumen 506 along anchor-driver-sublumen axis 507.

In the loaded state, the wall of stopper 560 blocks the distal end of anchor-storage sublumen 504, thereby preventing distal movement of any additional anchor 520 and providing a safety mechanism for accidental release of an additional anchor. For some applications, the handle of unit 518 comprises a spring (not shown), disposed within anchor-storage sublumen 504 proximally to the plurality of tissue anchors 520. For some applications, the spring is disposed proximally to anchor-storage zone 522.

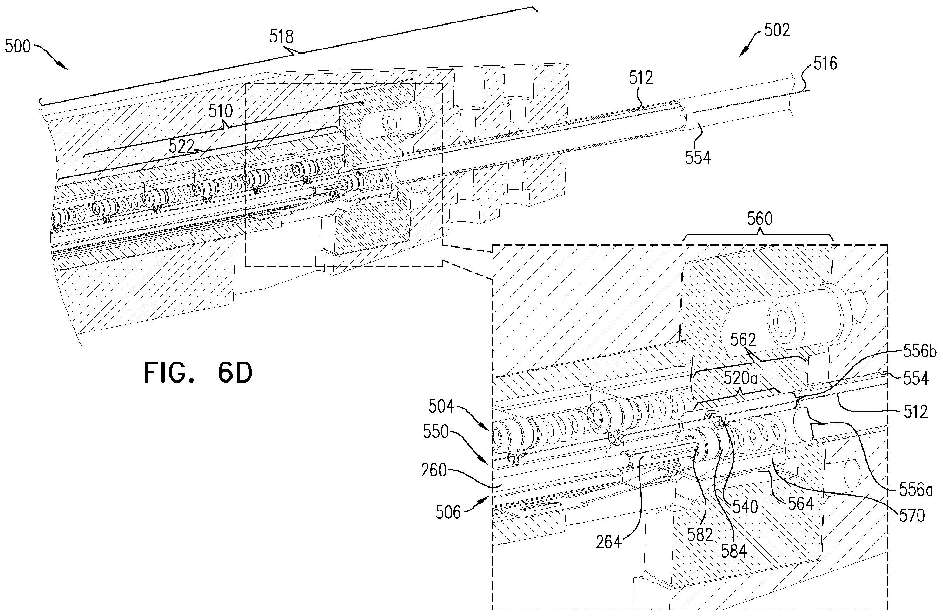

Fig. 6D shows system 500, which comprises implant 510, and delivery tool 550 for percutaneous (e.g., transluminal, such as transfemoral) implantation of the implant. Tool 550 comprises flexible anchor driver 260 that is configured to reversibly engage driver interface 582 of anchor 520. Via this engagement, driver 260 is configured to drive tissue-engaging element 530 into tissue, e.g., by rotating (and distally pushing) anchor 520. While anchor 520a is disposed within receiving lumen 562 and generally held in place due to the force of spring 564, anchor driver 260 is slide distally within anchor-driver sublumen 506 such that driver head 264 approaches and engages with interface 582 of anchor head 580 of tissue anchor 520a.

Channel 554 is shaped to control, during delivery and anchoring, a rotational position of eyelet 540 with respect to axis ax2 and/or tissue-engaging element 530. For some such applications, channel 554 defines a major channel region 556a and a minor channel region 556b. Major channel-region 556a has a larger cross-sectional area than does minor channel region 556b. Anchor 520 is slidable through channel 554 with tissue-engaging element 530 sliding (often snugly) through primary channel region 556a, and eyelet 540 sliding (often snugly) through minor channel region 556b and along wire 512. Rotational control of channel 552 thereby controls the position of eyelet 540, and therefore of wire 512, around axis ax2 of each anchor. While driver interface 582 and tissue-engaging element 530 are rotatable within channel 554, ring 584 and eyelet 540 are not. For some applications, and as shown, channel 554 has a keyhole-shaped orthogonal cross-section.

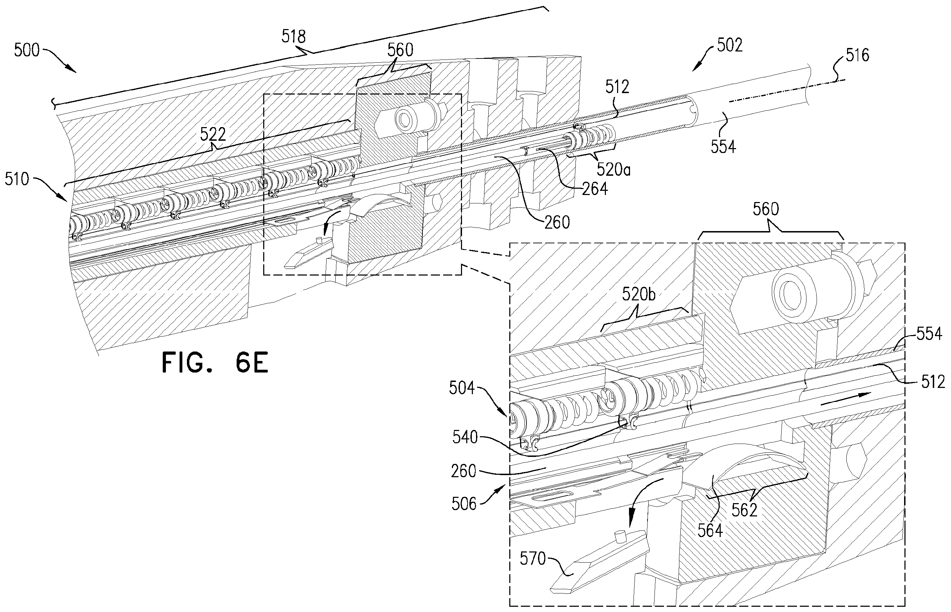

As shown in Fig. 6D, an indicator 570 is coupled to each anchor 520. When anchor 520a is disposed within receiving lumen 562, indicator 570 is disposed between anchor 520 and spring 564 to help stabilize anchor 520a. In Fig. 6E, anchor driver 260 is pushed distally in order to advance anchor 520a distally beyond receiving lumen 562 and into the lumen of channel 554. Due to the curvature of spring 564, in the absence of anchor 520a, spring 564 returns to its resting state, i.e., in which spring 564 assumes a curved state. As anchor 520a is advanced distally and spring 564 assumes its curved state, indicator 570 is expelled from within receiving lumen 562 providing an indication that anchor driver 260 has successfully been coupled to anchor 520a and has advanced anchor 520a distally into the lumen of channel 554 of catheter 502.

Once driver 260 implants tissue anchor 520a, it is disengaged from anchor 520a and retracted through the lumen of channel 554 and through the anchor-driver sublumen 506. Driver head 264 is disposed within sublumen 506 proximally to revolving stopper 560 until revolving stopper 560 is revolved such that it assumes the receiving state as shown in Fig. 6B in order to receive the next anchor 520b in sequence that was disposed proximally adjacent to the distal-most anchor 520a. Revolving stopper 560 is then revolved to assume the loaded state shown in Fig. 6C so that anchor 550b is brought into alignment with anchor-driver sublumen 506 and with the lumen of channel 554 of catheter 502. In the loaded state, anchor driver 260 engages tissue anchor 520b, as described hereinabove with reference to Fig. 6D, and anchor driver 260 pushes anchor 520b distally through the lumen of channel 554, as described in Fig. 6E.

After a desired number of anchors 520 have been anchored, an adjustment tool is introduced (e.g., over and along a proximal portion of wire 512) and is used to facilitate tensioning of the wire. Therefore, the tensioning of wire 512 draws anchors 520 closer together, thereby contracting the tissue to which the anchors are anchored. This is facilitated by eyelets 240 providing smooth sliding of wire 512 through apertures 546 while the wire is orthogonal to the anchors, as described hereinabove. Respective stoppers are coupled to wire 512 to prevent wire 512 from sliding out of anchors 520. Excess wire 512 can then be cut and removed from the patient.

As described hereinabove, for some applications eyelet 540 is mounted to be revolvable around axis ax2. This therefore provides independence between the rotational position of the eyelet and that of tissue-engaging element 530. It is hypothesized that, for applications in which tissue-engaging element 530 is helical, this independence advantageously allows the tissue-engaging element to be screwed into tissue to the extend needed for optimal anchoring, without a requirement for the anchor to finish in a particular rotational orientation. It is further hypothesized that, irrespective of the type of tissue-engaging element 530 used, this independence allows eyelet 540 (and wire 512) to be in an optimal position, with respect to axis ax2 of each anchor 520, for a given application. For example, for an application in which implant 510 is used for annuloplasty, anchors 520 are often anchored in a curve around the valve annulus, and eyelets 540 and wire 512 can be disposed on the inside of the curve relative to axis ax2.

To anchor anchors 520, each anchor is advanced out of a distal end of channel 554 and while driver 260 rotates driver interface 582 (and thereby tissue-engaging element 530) with respect to the channel 554.

Each anchor 520 defines the respective tissue-anchor-lumen extending through a longitudinal length of anchor 520 from a proximal end of anchor head 580 to a distal end of tissue-engaging element 530, and anchor driver 260, e.g., particularly driver head 264, is dimensioned to be axially slid within all of the tissue-anchor-lumens of anchors 520.

For some applications, eyelet 540 comprises or is shaped so as to define protrusion 241 described hereinabove with reference to Figs. 1A-B and 2A-B.

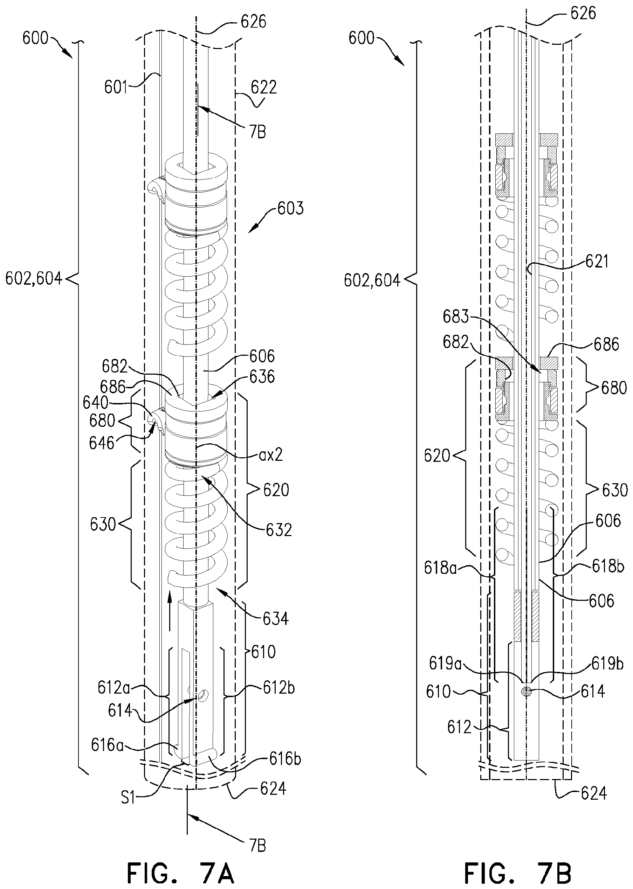

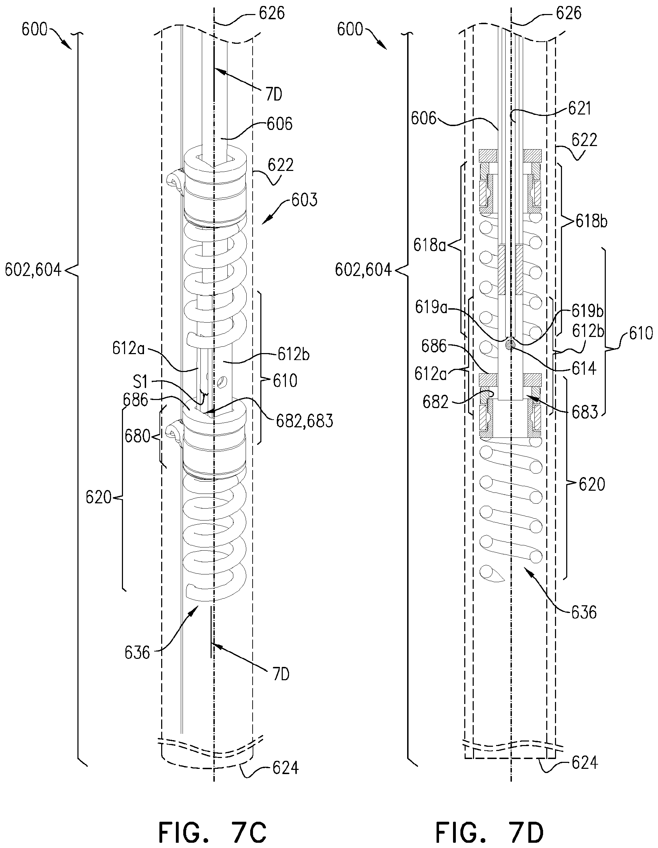

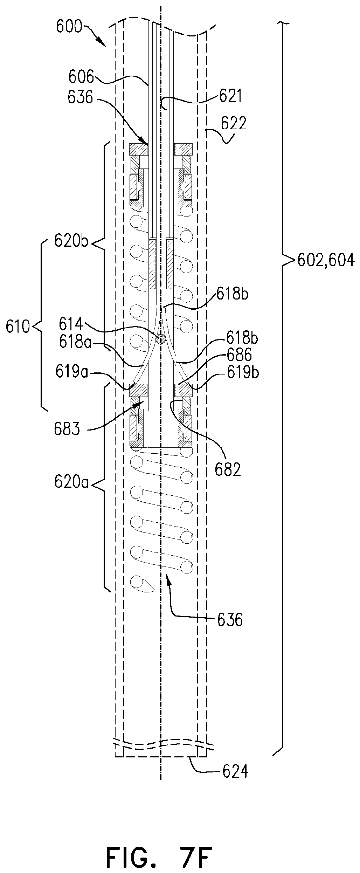

Reference is now made to Figs. 7A-F, which are schematic illustrations of examples of a catheter system 600 comprising a transluminally-advanceable catheter 622, at least one tissue anchor 620, and an implant 603 comprising the tissue anchor, and techniques for use therewith, in accordance with some applications. System 600 is a tissue-adjustment system and can be used for adjusting a dimension of a tissue structure. For example, system 600 can be an annuloplasty system, and implant 603 can be an annuloplasty structure.

Catheter 622 comprises a flexible tube, e.g., a polymer tube, and is coupled to an extracorporeal unit (e.g., unit 18, shown for example, in Fig. 1A) at a proximal end of catheter 622 and extends distally therefrom along a longitudinal catheter axis 626 of catheter 622, from the unit to a distal opening of catheter 622 at a distal end 624 of catheter 622. Transluminally-advanceable catheter 622 comprises a channel shaped so as to define a lumen which houses implant 603 during delivery of implant 603 to tissue 10 of a patient. For applications in which tissue 10 represents tissue of the annulus of a native heart valve, such as the mitral valve, implant 603 is an annuloplasty structure comprising a wire 601 and multiple anchors 620. Each anchor 620 is shaped so as to define a central longitudinal anchor axis ax2. Each anchor 620 comprises a tissue-engaging element 630 and an anchor head 680. Tissue-engaging element 630 has a proximal end 632, a distal end 634, and defines central longitudinal axis ax2 of anchor 620. At distal end 634, tissue-engaging element 630 has a sharpened distal tip, and the tissue-engaging element is configured to be driven (e.g., screwed) into tissue of the subject. For some applications, and as shown, tissue-engaging element 630 is helical and defines a central lumen 636 along axis ax2, which is part of the central lumen of tissue anchor 620 from an opening at a proximal end of anchor head 680 to distal end 634 of tissue-engaging element 630. Optionally, tissue-engaging element 630 can be another type of tissue-engaging element, such as a dart or a staple. Head 680 is coupled to proximal end 632 of tissue-engaging element 630 and comprises a driver interface 682 and an eyelet 640 that defines an aperture 646 therethrough. Driver interface 682 is configured to be reversibly engaged by a flexible anchor driver 604. In some applications, eyelet 640 comprises a structural element that protrudes laterally with respect to tissue-engaging element 630.

System 600 comprises anchor driver 604 which can comprise an elongate and flexible shaft 606, and a driver head 610 coupled to a distal end of the shaft. Driver head 610 is the component of anchor driver 604 that reversibly engages driver interface 682 of anchor 620. Driver interface 682 can be rigidly coupled to tissue-engaging element 630. For some applications, anchor head 680 (e.g., driver interface 682) comprises a proximal rim 686 and a wall extending distally therefrom shaped so as to define and surround a recessed portion 683 which extends distally from rim 686. For some applications, the wall surrounding recessed portion 683 is square, as shown. For some applications, the wall surrounding recessed portion 683 is shaped as a circular wall.

In some applications, and as shown, driver interface 682 is disposed on central longitudinal axis ax2, and eyelet 640 is disposed laterally from axis ax2.

Anchor 620 (e.g., eyelet 640 thereof) is configured to facilitate sliding of the anchor along wire 601 (or sliding of the wire through the anchor) while the anchor is aligned with the wire - e.g., while axis ax2 is parallel with the wire. This is hypothesized to facilitate transcatheter advancement of anchor 620 along the wire. Anchor 620 (e.g., eyelet 640 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 640. This is hypothesized to be useful, inter alia, for applications in which the wire is tensioned after implantation in order to adjust anatomical dimensions, such as annuloplasty.

For some applications, and as shown, eyelet 640 is mounted to be revolvable around axis ax2. For example, head 680 can comprise a ring 684 on which eyelet 640 is mounted. Ring 684 circumscribes and is rotatable about axis ax2, e.g., by being rotatably coupled to tissue-engaging element 630, such as by being rotatably coupled to another component of head 680 (e.g., driver interface 682) that is fixedly coupled to the tissue-engaging element.

Each anchor 620 is shaped so as to define a central lumen extending from proximal rim 686 to distal end 634 of tissue-engaging element 630. Flexible shaft 606 and driver head 610 of driver 604 are dimensioned to be slidable within the respective central lumens of anchors 620. Anchor driver 604, e.g., particularly driver head 610, is dimensioned to be axially slid within the lumen of catheter 622 and is actuatable to reversibly engage the driver interface 682 of each tissue anchor. Anchor driver 604 is configured to, while driver head 610 is engaged with driver interface 682, advance anchor 620 distally toward the distal opening at end 624 of catheter 622, and drive tissue-engaging element 630 along tissue anchor axis ax2 into tissue 10.

As shown in Figs. 7A-B, driver head 610 is disposed distally to a distal-most anchor 620, while shaft 606 is disposed within the central lumen of anchor 620, by way of illustration and not limitation, whereas in Figs. 7C-F, a distal end portion of driver head 610 is positioned within anchor head 680 after driver head 610 has been slid proximally though the central lumen of anchor 620 by pulling on shaft 606.

Reference is now made to Figs. 7A-B. It is to be noted that Fig. 7B is a vertical cross-section of delivery tool 602. Driver head 610 is shaped so as to define first and second legs 612a and 612b having a resting state (shown in Figs. 7A-D) in which first and second legs 612a and 612b define a first space S1 between each other having a first distance and in which first and second legs 612a and 612b do not contact and/or do not firmly engage with the wall of tissue anchor 620 surrounding recessed portion 683. Anchor driver 604 comprises a connecting bar 614 disposed at least in part between first and second legs 612a and 612b. Legs 612a and 612b are each shaped so as to define respective windows for coupling of each leg 612a and 612b to respective ends of bar 614. The windows of legs 612a and 612b are configured to enable legs 612a and 612b, in an expanded state, to slide along bar 614 and facilitate expansion of legs 612a and 612b away from central axis 626. The distal portions of each legs 612a and 612b are each shaped so as to define respective engaging bulbs 616a and 616b which are configured to engage the wall of recessed portion 683, as is described hereinbelow.

Anchor driver 604 additionally comprises first and second expander elements 618a and 618b (shown in Fig. 7B) which are disposed at or coupled to a distal end of an advancing element 621. For some applications, first and second expander elements 618a and 618b are formed from advancing element 621. For some applications, first and second expander elements 618a and 618b comprise respective arms or wires. First and second expander elements 618a and 618b and advancing element 621 are slidable with respect to flexible shaft 606 and with respect to first and second legs 612a and 612b. First and second expander elements 618a and 618b are advanced distally responsively to pushing on advancing element 621 and are advanceable proximally in response to pulling on advancing element 621. The first and second expander elements can be configured in a variety of ways, e.g., including one or more of wires, arms, rods, latches, levers, extensions, expandable rings, expandable helixes, etc. The advancing element can also be configured in a variety of ways, e.g., to include one or more of a wire, rod, shaft, hypotube, extension, etc.

As shown in Figs. 7A-D, first and second expander elements 618a and 618b are disposed upstream of connecting bar 614 in a collapsed state in the resting state of first and second legs 612a and 612b of anchor driver 604. As such, driver head 610 remains in a position in which it does not fixedly engage any part of anchor 620, and driver head 610 is slidable axially with respect to anchor 620 within its central lumen. In Figs. 7A-B, driver head 610 is positioned distally to a distal-most tissue anchor 620. Since legs 612a and 612b are in a resting state, anchor driver 604 is slidable proximally, e.g., by pulling on shaft 606, in order to properly position driver head 610 with respect to anchor 620, as shown in Figs. 7C-D, so that driver head 610 can engage and maintain coupling with anchor head 680. As shown, prior to engaging anchor head 680, the distal portions of legs 612a and 612b are disposed within recessed portion 683 in a manner in which engaging bulbs 616a and 616b of legs 612a and 612b are positioned within recessed portion 683 and in alignment with the wall surrounding recessed portion 683 of driving interface 682.

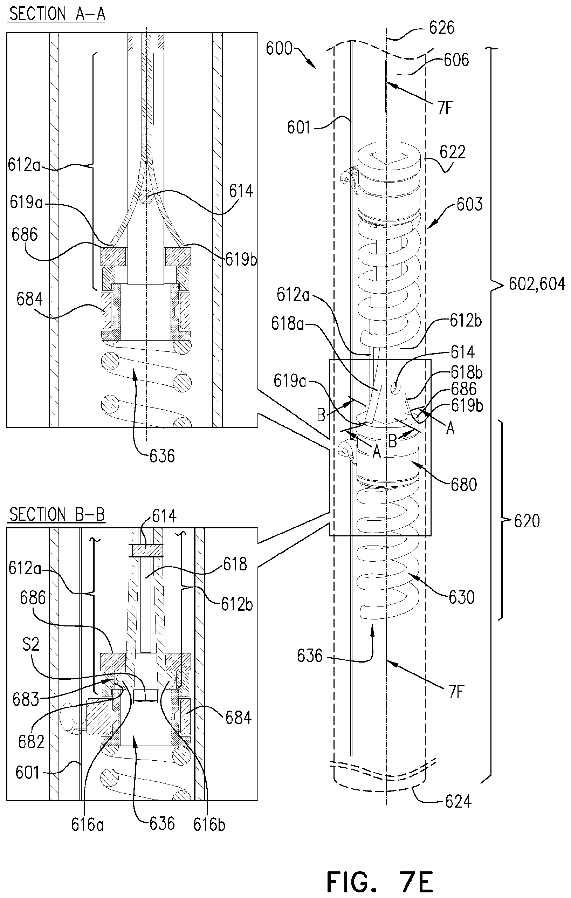

Figs. 7E-F show first and second legs 612a and 612b of driver head 610 in an engaged state in which engaging bulbs 616a and 616b of legs 612a and 612b firmly engage the wall surrounding recessed portion 683 of driving interface 682. In order to transition legs 612a and 612b into the engaged state, advancing element 621 is pushed distally in order to advance first and second expander elements 618a and 618b expand around connecting bar 614 away from central axis 626 and into the space between first and second legs 612a and 612b. As first and second expander elements 618a and 618b expand within the space between legs 612a and 612b, first and second expander elements 618a and 618b expand first and second legs 612a and 612b away from central axis 626 such that first and second legs 612a and 612b assume the engaged state in which the first and second legs define a second space S2 between each other having a second distance greater than the first distance of first space S1 (shown in Figs. 7A and 7C). As legs 612a and 612b expands outward, engaging bulbs 616a and 616b of first and second legs 612a and 612b contact and engage the wall of tissue anchor 620 surrounding recessed portion 683.

As shown, as first and second expander elements 618a and 618b expand around connecting bar 614, expander elements 618a and 618b expand along a first plane shown in Section A-A that is at a nonzero angle, e.g., perpendicular, with respect to a second plane shown in Section B-B along which first and second legs 612a and 612b expand, by way of illustration and not limitation.

First and second expander elements 618a and 618b have respective distal surfaces 619a and 619b at the respective end of each element 618a and 618b. In the engaged state of first and second legs 612a and 612b as shown in Figs. 7E-F, surfaces 619a and 619b engage with and push against proximal rim 686 of tissue anchor 620 in order to facilitate pushing of tissue anchor 620 distally in the engaged state of the first and second legs. Therefore, system 600 is particularly advantageous because expander elements 618a and 618b facilitate firm and reversible coupling between legs 612a and 612b and tissue anchor 620 as well as pushing of tissue anchor 620 by expander elements 618a and 618b. In particular, expansion and collapsing of expander elements 618a and 618b is controlled by advancing element 621. In such a manner, anchor driver 604 is able to (1) slide proximally and distally through a series of anchors without firmly engaging any of them during the sliding, (2) engage any of the anchors in a controlled manner by controlling expansion and collapsing of expander elements 618a and 618b, and (3) access any anchor in a series of anchors, whether the anchor is disposed proximal or distal to driver head 610.

Driver 604 is used to implant tissue anchor 620 by rotating tissue anchor 620 along axis ax2. Expander elements 618a and 618b prevent collapsing of legs 612a and 612b during the driving of anchor 620 into tissue as torque is applied to anchor 620 by driver 604. Additionally, expander elements 618a and 618b enable legs 612a and 612b to pull proximally on anchor 620 if necessary.

It is to be noted that system 600 provides a reversible engaging between anchor driver 604 and anchor 620. For example, once anchor 620 is implanted in tissue, driver 604 is disconnected from tissue anchor 620 by pulling proximally on advancing element 621 such that expander elements 618a and 618b and retracted proximally from bar 614 and collapse together to the collapsed state shown in Figs. 7A- which allows first and second legs 612a and 612b to assume their resting state in which the first space S1 is defined between legs 612a and 612b. The reversible engaging of driver 604 with any tissue anchor 620 by the expansion and collapsing of legs 612a and 612b by expander elements 618a and 618b is particularly useful if the operating physician mistakenly engages the incorrect anchor in a series of anchors 620. In such an event, the engaging of the incorrect anchor can be reversed by retracting advancing element 621 to retract expander elements 618a and 618b, and thereby, anchor driver 604 can be retracted. Anchor driver 604 can then be repositioned to engage the correct anchor in a manner as described hereinabove.

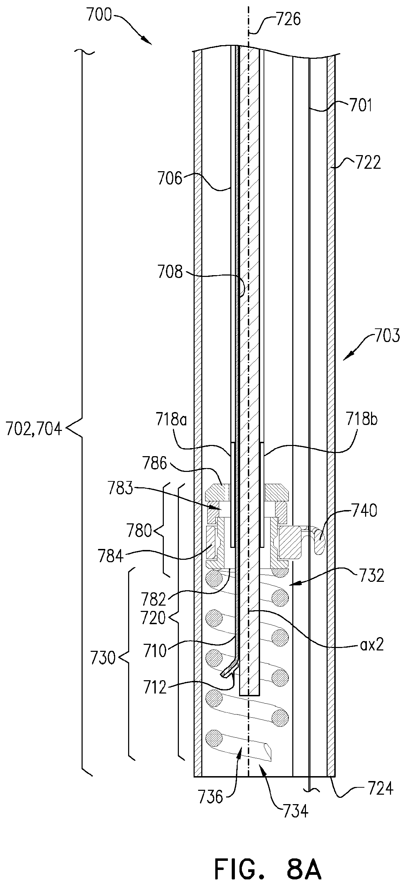

Reference is now made to Figs. 8A-C, which are schematic illustrations of examples of a catheter system 800 comprising a transluminally-advanceable catheter 722, at least one tissue anchor 720, and an implant 703 comprising the tissue anchor, and techniques for use therewith, in accordance with some applications. System 700 is a tissue-adjustment system and can be used for adjusting a dimension of a tissue structure. For example, system 700 can be an annuloplasty system, and implant 703 can be an annuloplasty structure.

Catheter 722 comprises a flexible tube, e.g., a polymer tube, and is coupled to an extracorporeal unit (e.g., unit 18, shown for example, in Fig. 1A) at a proximal end of catheter 722 and extends distally therefrom along a longitudinal catheter central axis 726 of catheter 722, from the unit to a distal opening of catheter 722 at a distal end 724 of catheter 722. Transluminally-advanceable catheter 722 comprises a channel shaped so as to define a lumen which houses implant 703 during delivery of implant 703 to tissue 10 of a patient. For applications in which tissue 10 represents tissue of the annulus of a native heart valve, such as the mitral valve, implant 703 is an annuloplasty structure comprising a wire 701 and multiple anchors 720.

For some applications, each anchor 720 is shaped so as to define a central longitudinal anchor axis ax2. For some applications, each anchor 720 comprises a tissue-engaging element 730 and an anchor head 780. Tissue-engaging element 730 has a proximal end 732, a distal end 734, and defines central longitudinal axis ax2 of anchor 720. For some applications, at distal end 734, tissue-engaging element 730 has a sharpened distal tip, and the tissue-engaging element is configured to be driven (e.g., screwed) into tissue of the subject. For some applications, and as shown, tissue-engaging element 730 is helical and defines a central lumen 736 along axis ax2, which is part of the central lumen of tissue anchor 720 from an opening at a proximal end of anchor head 780 to distal end 734 of tissue-engaging element 730. Optionally, tissue-engaging element 730 can be another type of tissue-engaging element, such as a dart or a staple. Head 780 is coupled to proximal end 732 of tissue-engaging element 730 and comprises a driver interface 782 and an eyelet 740 that defines an aperture 746 therethrough. Driver interface 782 is configured to be reversibly engaged by flexible anchor driver 704. In some applications, eyelet 740 comprises a structural element that protrudes laterally with respect to tissue-engaging element 730.

System 700 comprises an anchor driver 704 which can comprise an elongate and flexible shaft 710, and a driver head 712 coupled to a distal end of the shaft. Driver head 712 comprises a deflectable protrusion and is the component of anchor driver 704 that reversibly engages driver interface 782 of anchor 720. Driver interface 782 can be rigidly coupled to tissue-engaging element 730 and defines an inner surface thereof. In some applications, as shown, driver interface 782 comprises a section of anchor 720 at the junction between head 780 and tissue-engaging element 730, by way of illustration and not limitation. It is to be noted that driver interface 782 may comprise any portion of anchor 720. For some applications, anchor head 780 comprises a proximal rim 786 and a wall extending distally therefrom shaped so as to define and surround a recessed portion 783 which extends distally from rim 786. For some applications, the wall surrounding recessed portion 783 is square, as shown. For some applications, the wall surrounding recessed portion 783 is shaped as a circular wall.

In some applications, and as shown, driver interface 782 is disposed on central longitudinal axis ax2, and eyelet 740 is disposed laterally from axis ax2.

Anchor 720 (e.g., eyelet 740 thereof) is configured to facilitate sliding of the anchor along wire 701 (or sliding of the wire through the anchor) while the anchor is aligned with the wire - e.g., while axis ax2 is parallel with the wire. This is hypothesized to facilitate transcatheter advancement of anchor 720 along the wire. Anchor 720 (e.g., eyelet 740 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 740. This is hypothesized to be useful, inter alia, for applications in which the wire is tensioned after implantation in order to adjust anatomical dimensions, such as annuloplasty.

For some applications, and as shown, eyelet 740 is mounted to be revolvable around axis ax2. For example, head 780 can comprise a ring 784 on which eyelet 740 is mounted. Ring 784 circumscribes and is rotatable about axis ax2, e.g., by being rotatably coupled to tissue-engaging element 730, such as by being rotatably coupled to another component of head 780 (e.g., driver interface 782) that is fixedly coupled to the tissue-engaging element.

Each anchor 720 is shaped so as to define a central lumen extending from proximal rim 786 to distal end 734 of tissue-engaging element 730. Flexible tube 706 and driver head 712 of driver 704 are dimensioned to be slidable within the respective central lumens of anchors 720. Anchor driver 704, e.g., particularly driver head 712, is dimensioned to be axially slid within the lumen of catheter 722 and is actuatable to reversibly engage the driver interface 782 of each tissue anchor. Anchor driver 704 is configured to, while driver head 712 is engaged with driver interface 782, advance anchor 720 distally toward the distal opening at end 724 of catheter 722, and drive tissue-engaging element 730 along tissue anchor axis ax2 into tissue 10.

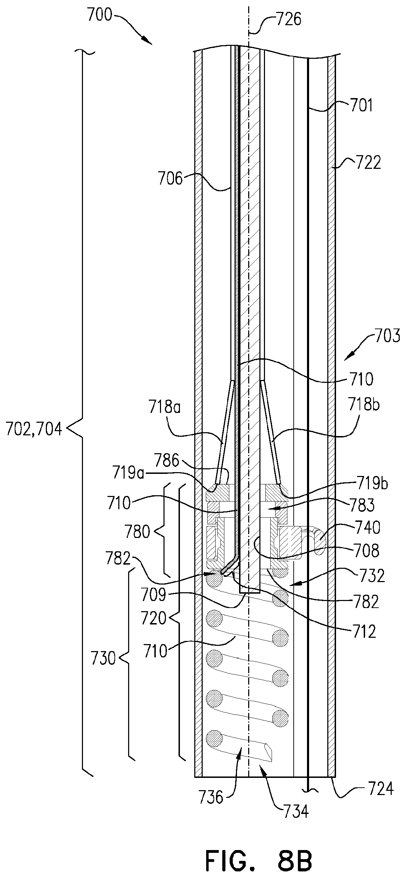

As shown in Fig. 8A, driver head 712 is disposed distally to driver interface 782 of a distal-most anchor 720, while tube 706 is disposed within the central lumen of anchor 720, by way of illustration and not limitation, whereas in Fig. 8B, driver head 712 is positioned to firmly engage within driver interface 782 after driver head 712 and shaft 710 have been slid proximally though the central lumen of anchor 720 by pulling on shaft 710. For some applications, driver interface 782 defines a groove in which driver head 712 fits snugly in order to firmly engage driver interface 782. For some applications, driver interface 782 defines a wall of anchor 720 surrounding recessed portion 783.

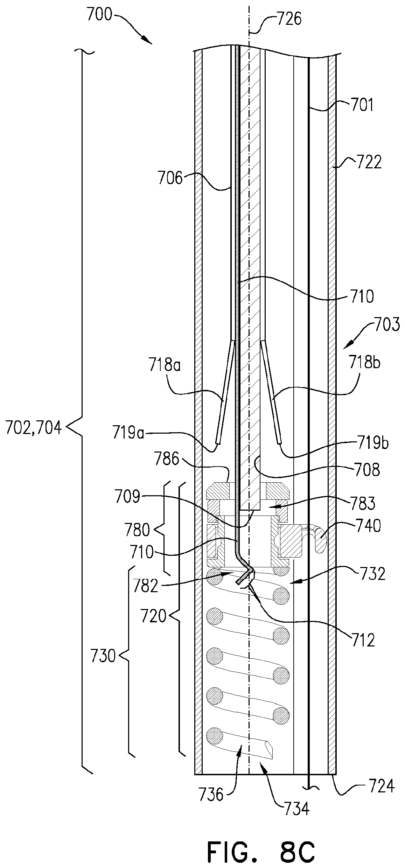

Anchor driver 704 comprises a flexible tube 706 having a central axis defined along central axis 726 of catheter 722. The protrusion of driver head 712 is deflectable from (1) a resting state in which the protrusion of head 712 assumes a collapsed state (Fig. 8C) toward the central axis defined by flexible tube 706 (i.e., toward axis 726) and does not contact the tissue anchor, e.g., a wall of anchor 720 surrounding recessed portion 783, and (2) to an engaged state of the deflectable protrusion of head 712 in which the deflectable protrusion moves away from the central axis defined by flexible tube 706 (i.e., toward axis 726) and engages with tissue anchor 720 at driver interface 782 (Fig. 8B).

Anchor driver 704 comprises a shaft 708 disposed within a lumen of flexible tube 706 and slidable with respect to tube 706. Shaft 708 is engageable with the deflectable protrusion of driver head 712 such that when shaft 708 slides alongside the deflectable protrusion of head 712 and a distal end 709 is proximal to driver head 712, shaft 708 deflects the deflectable protrusion from the collapsed state (as shown in Fig. 8C), away from the central axis of tube 706 (i.e., axis 726), and into an engaged state of the deflectable protrusion of head 712 in which the deflectable protrusion engages with tissue anchor 720 at interface 782 (as shown in Fig. 8B). Anchor driver 704 is dimensioned to be axially slid through a lumen defined by tissue anchor 720 and to selectively and reversibly engage tissue anchor 720 responsively to movement of shaft 708. Distal end 709 of shaft 708 is retractable away from the deflectable protrusion of head 712 to facilitate deflecting of the deflectable protrusion from the engaged state (Fig. 8B), toward central axis 726 and into the collapsed state (Fig. 8C) to disengage anchor driver 704 from tissue anchor 720.

Reference is now made to Figs. 8A-C. It is to be noted that Figs. 8A-C are vertical cross-sections of delivery tool 702.

Anchor driver 704 additionally comprises first and second expander elements 718a and 718b which are disposed at or coupled to a distal end of tube 706, e.g., at an outer surface of tube 706. For some applications, first and second expander elements 718a and 718b are formed from tube 706. For some applications, first and second expander elements 718a and 718b are controlled by a separate mechanism. For some applications, first and second expander elements 718a and 718b comprise respective wires. First and second expander elements 718a and 718b and tube 706 are slidable with respect to flexible shaft 708 and with respect to shaft 710 and driver head 712. First and second expander elements 718a and 718b are advanced distally responsively to pushing on tube 706 and are advanceable proximally in response to pulling on tube 706.

As shown in Fig. 8A, first and second expander elements 718a and 718b are collapsed within the lumen of anchor 720. For some applications, first and second expander elements 718a and 718b comprise shape-memory material which enable first and second expander elements 718a and 718b to expand in the absence of force applied thereto, as shown in Fig. 8B which shows first and second expander elements 718a and 718b in their expanded state. For some applications, first and second expander elements 718a and 718b do not comprise a shape-memory material, and expansion of first and second expander elements 718a and 718b is either controlled by tube 706 or by some external mechanism.

To transition from Fig. 8A in which driver head 712 is in a position in which it does not fixedly engage any part of anchor 720 to Fig. 8B, shaft 708 is retracted proximally alongside shaft 710 to collapse the deflectable protrusion of driver head 712 toward axis 726. Shaft 710 and head 712 as well as shaft 708 are retracted proximally to position driver head 712 at interface 782. Additionally, tube 706 is retracted proximally to expose first and second expander elements 718a and 718b from within the lumen of anchor 720. For some applications, movement of shaft 708 and tube 706 are connected such that movement of shaft 708 facilitates movement of tube 706. In Fig. 8B, driver head 712 firmly engages with driver interface 782 and maintains coupling with anchor 720. During the firm coupling distal end 709 of shaft 708 is positioned distally to driver head 712 such that a distal portion of shaft 708 applies a force outward from axis 726 against driver head 712 to maintain driver head 712 in a position in which it is deflected away from axis 726 are remains engaged with interface 782.

As shown in Fig. 8B, as first and second expander elements 718a and 718b expand their respective distal surfaces 719a and 719b at the respective end of each element 718a and 718b engage with and push against proximal rim 786 of tissue anchor 720 in order to facilitate pushing of tissue anchor 720 distally in the engaged state of the deflectable protrusion of driver head 712. Therefore, system 700 is particularly advantageous because driver head 712 facilitates firm and reversible coupling to tissue anchor 720, and expander elements 718a and 718b facilitate pushing of tissue anchor 720. In particular, collapsing of expander elements 718a and 718b is controlled by tube 706.

Driver 704 is used to implant tissue anchor 720 by rotating tissue anchor 720 along axis ax2.

It is to be noted that system 700 provides a reversible engaging between anchor driver 704 and anchor 720. For example, once anchor 720 is implanted in tissue, driver 704 is disconnected from tissue anchor 720 by pulling proximally on shaft 708 such the deflectable protrusion of head 712 collapses toward axis 726 (Fig. 8C) and tube 706 is pulled proximally such that expander elements 718a and 718b are retracted proximally anchor 720 and surface 719a and 719b do not contact rim 786. Expander elements 718a and 718b can then collapse together to the collapsed state when driver 704 is retracted through a proximally-disposed anchor in the series of anchors 720. The reversible engaging of driver 704 with any tissue anchor 720 by the expansion and collapsing of legs 712a and 712b by expander elements 718a and 718b is particularly useful if the operating physician mistakenly engages the incorrect anchor in a series of anchors 720. In such an event, the engaging of the incorrect anchor can be reversed by retracting shaft 708 to retract collapse head 712, and thereby, anchor driver 704 can be retracted. Anchor driver 704 can then be repositioned to engage the correct anchor in a manner as described hereinabove.

It is to be noted that Fig. 8C shows disengaging of driver 704 from anchor 720 while anchor 720 is within the lumen of catheter 722 by way of illustration and not limitation. In the same manner as described for disengaging driver 704 from anchor 720 while anchor 720 is within the lumen of catheter 722, driver 704 is disengageable from anchor 720 once anchor 720 has been implanted in tissue 10.

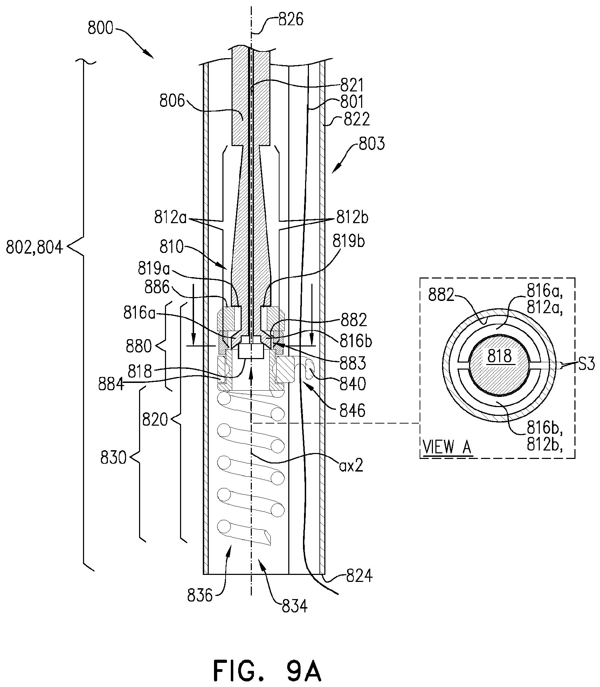

Reference is now made to Figs. 9A-B, which are schematic illustrations of examples of a catheter system 800 comprising a transluminally-advanceable catheter 822, at least one tissue anchor 820, and an implant 803 comprising the tissue anchor, and techniques for use therewith, in accordance with some applications. System 800 is a tissue-adjustment system and can be used for adjusting a dimension of a tissue structure. For example, system 800 can be an annuloplasty system, and implant 803 can be an annuloplasty structure.

Catheter 822 comprises a flexible tube, e.g., a polymer tube, and is coupled to an extracorporeal unit (e.g., unit 18, shown for example, in Fig. 1A) at a proximal end of catheter 822 and extends distally therefrom along a longitudinal catheter axis 826 of catheter 822, from the unit to a distal opening of catheter 822 at a distal end 824 of catheter 822. Transluminally-advanceable catheter 822 comprises a channel shaped so as to define a lumen which houses implant 803 during delivery of implant 803 to tissue 10 of a patient. For applications in which tissue 10 represents tissue of the annulus of a native heart valve, such as the mitral valve, implant 803 is an annuloplasty structure comprising a wire 801 and multiple anchors 820.

For some applications, each anchor 820 is shaped so as to define a central longitudinal anchor axis ax2. For some applications, each anchor 820 comprises a tissue-engaging element 830 and an anchor head 880. Tissue-engaging element 830 has a proximal end 832, a distal end 834, and defines central longitudinal axis ax2 of anchor 820. For some applications, at distal end 834, tissue-engaging element 830 has a sharpened distal tip, and the tissue-engaging element is configured to be driven (e.g., screwed) into tissue of the subject. For some applications, and as shown, tissue-engaging element 830 is helical and defines a central lumen 836 along axis ax2, which is part of the central lumen of tissue anchor 820 from an opening at a proximal end of anchor head 880 to distal end 834 of tissue-engaging element 830. Optionally, tissue-engaging element 830 can be another type of tissue-engaging element, such as a dart or a staple. Head 880 is coupled to proximal end 832 of tissue-engaging element 830 and comprises a driver interface 882 and an eyelet 840 that defines an aperture 846 therethrough. Driver interface 882 is configured to be reversibly engaged by a flexible anchor driver 804. In some applications, eyelet 840 comprises a structural element that protrudes laterally with respect to tissue-engaging element 830.

System 800 comprises anchor driver 804 which can comprise an elongate and flexible advancing element 806, and a driver head 810 coupled to a distal end of advancing element 806. Driver head 810 is the component of anchor driver 804 that reversibly engages driver interface 882 of anchor 820. Driver interface 882 can be rigidly coupled to tissue-engaging element 830. For some applications, anchor head 880 (e.g., driver interface 882) comprises a proximal rim 886 and a wall extending distally therefrom shaped so as to define and surround a recessed portion 883 which extends distally from rim 886. For some applications, the wall surrounding recessed portion 883 is square. For some applications, the wall surrounding recessed portion 883 is shaped as a circular wall.

In some applications, and as shown, driver interface 882 is disposed on central longitudinal axis ax2, and eyelet 840 is disposed laterally from axis ax2.

Anchor 820 (e.g., eyelet 840 thereof) is configured to facilitate sliding of the anchor along wire 801 (or sliding of the wire through the anchor) while the anchor is aligned with the wire - e.g., while axis ax2 is parallel with the wire. This is hypothesized to facilitate transcatheter advancement of anchor 820 along the wire. Anchor 820 (e.g., eyelet 840 thereof) is configured to facilitate sliding of the anchor along the wire (or sliding of the wire through the anchor) while the anchor is oriented orthogonal to the wire - i.e., while axis ax2 is orthogonal to the wire. This is achieved at least partly due to the shape and dimensions of eyelet 840. This is hypothesized to be useful, inter alia, for applications in which the wire is tensioned after implantation in order to adjust anatomical dimensions, such as annuloplasty.

For some applications, and as shown, eyelet 840 is mounted to be revolvable around axis ax2. For example, head 880 can comprise a ring 884 on which eyelet 840 is mounted. Ring 884 circumscribes and is rotatable about axis ax2, e.g., by being rotatably coupled to tissue-engaging element 830, such as by being rotatably coupled to another component of head 880 (e.g., driver interface 882) that is fixedly coupled to the tissue-engaging element.

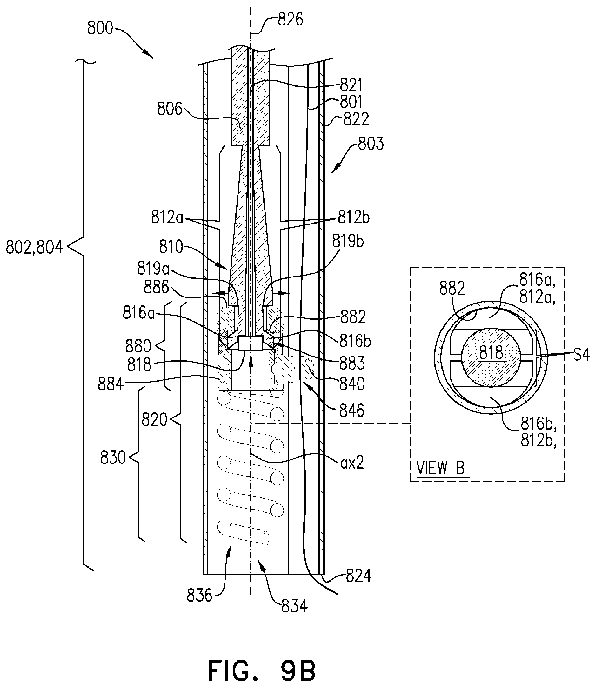

Each anchor 820 is shaped so as to define a central lumen extending from proximal rim 886 to distal end 834 of tissue-engaging element 830. Flexible advancing element 806 and driver head 810 of driver 804 are dimensioned to be slidable within the respective central lumens of anchors 820 when driver 804 is in its resting state as shown in Fig. 9B. Anchor driver 804, e.g., particularly driver head 810, is dimensioned to be axially slid within the lumen of catheter 822 and is actuatable to reversibly engage the driver interface 882 of each tissue anchor. Anchor driver 804 is configured to, while driver head 810 is engaged with driver interface 882, advance anchor 820 distally toward the distal opening at end 824 of catheter 822, and drive tissue-engaging element 830 along tissue anchor axis ax2 into tissue 10.

It is to be noted that Figs. 9A-B are vertical cross-sections of delivery tool 802. Driver head 810 is shaped so as to define first and second legs 812a and 812b having a resting state (shown in Fig. 9A) in which first and second legs 812a and 812b define a first space S3 between each other having a first distance and in which first and second legs 812a and 812b do not contact and/or do not firmly engage with the wall of tissue anchor 820 surrounding recessed portion 883.

Anchor driver 804 comprises an expander element 818 coupled to a displacing element 821, e.g., a shaft, rod, wire, hypotube, line, wedge, extension, etc., which facilitates distal and proximal sliding of expander element 818. Displacing element 821 is disposed within a lumen of and slidable with respect to advancing element 806 (which can be the same as or similar to other advancing elements herein) such that expander element is slidable with respect to advancing element 806 and with respect to legs 812a and 812b. In the resting state of legs 812a and 812b, expander element 818 is positioned at a distal surface of legs 812a and 812b, as shown in Fig. 9A. In the resting state, as shown in View A, legs 812a and 812b collectively assume a circular profile.

As shown in Fig. 9B, expander element 818 is pulled proximally by pulling on displacing element 821 such that expander element 818 is pulled into position between legs 812a and 812b and fits snugly within the space between legs 812a and 812b in order to facilitate expansion of legs 812a and 812b away from central axis 826 into an engaged state of legs 812a and 812b (Fig. 9B). In the engaged state of legs 812a and 812b, legs 812a and 812b define a second space S4 between each other having a second distance greater than the first distance of first space S3. The distal portions of each legs 812a and 812b are each shaped so as to define respective engaging bulbs 816a and 816b which are configured to engage the wall of recessed portion 883. For some applications, the wall of recessed portion 883 is shaped so as to define a groove for snug coupling of bulbs 816a and 816b in the engaged state of legs 812a and 812b. In the engaged state, as shown in View B, legs 812a and 812b collectively assume an elliptical profile.

For some applications, expander element 818 comprises a circular disc, as shown, having a diameter that is larger than first space S3 between first and second legs 812a and 812 in their resting state, such first and second legs 812a and 812b expand to assume the engaged state when expander element 818 is positioned between legs 812a and 812b.

For some applications, expander element 818 comprises an elliptical disc having a major axis diameter that is larger than first space S3 between first and second legs 812a and 812 in their resting state, such first and second legs 812a and 812b expand to assume the engaged state when expander element 818 is positioned between legs 812a and 812b.

Legs 812a and 812b of advancing element 806 are shaped so as to define pushing surfaces 819a and 819b which are disposed proximally to proximal rim 868. In the engaged state of first and second legs 812a and 812b as shown in Fig. 9B, surfaces 819a and 819b engage with and push against proximal rim 886 of tissue anchor 820 in order to facilitate pushing of tissue anchor 820 distally in the engaged state of the first and second legs. Therefore, system 800 is particularly advantageous because expander element 818 facilitates firm and reversible coupling between legs 812a and 812b and tissue anchor 820, and legs 812a and 812b provide pushing of tissue anchor 820 by pushing surfaces 819a and 819b. In particular, expansion and collapsing of legs 812a and 812b is controlled by displacing element 821 and expander element 818. In such a manner, anchor driver 804 is able to (1) slide proximally and distally through a series of anchors without firmly engaging any of them during the sliding, (2) engage any of the anchors in a controlled manner by controlling movement of expander element 818, and (3) access any anchor in a series of anchors, whether the anchor is disposed proximal or distal to driver head 810.

Driver 804 is used to implant tissue anchor 820 by rotating tissue anchor 820 along axis ax2. Expander element 818 prevents collapsing of legs 812a and 812b during the driving of anchor 820 into tissue as torque is applied to anchor 820 by driver 804. Additionally, expander element 818 enables legs 812a and 812b to pull proximally on anchor 820 if necessary.

It is to be noted that system 800 provides a reversible engaging between anchor driver 804 and anchor 820. For example, once anchor 820 is implanted in tissue, driver 804 is disconnected from tissue anchor 820 by pulling pushing distally on displacing element 821 such that expander element 818 is pushed distally which allows first and second legs 812a and 812b to collapse toward axis 826 and assume their resting state in which the first space S3 is defined between legs 812a and 812b. The reversible engaging of driver 804 with any tissue anchor 820 by the expansion and collapsing of legs 812a and 812b by expander elements 818a and 818b is particularly useful if the operating physician mistakenly engages the incorrect anchor in a series of anchors 820. In such an event, the engaging of the incorrect anchor can be reversed by pushing displacing element 821 to displace and move distally expander element 818, and thereby, anchor driver 804 can be retracted. Anchor driver 804 can then be repositioned to engage the correct anchor in a manner as described hereinabove.

Reference is now made to Figs. 7A-F, 8A-C, and 9A-B. It is to be noted that any of anchor drivers 604, 704, and 804 can be used to deliver any of tissue anchors 220, 320, 420, and 520 described hereinabove with reference to Figs. 1A-B, 2A-B, 3A-B, 4A-B, 5A-C, and 6A-E.

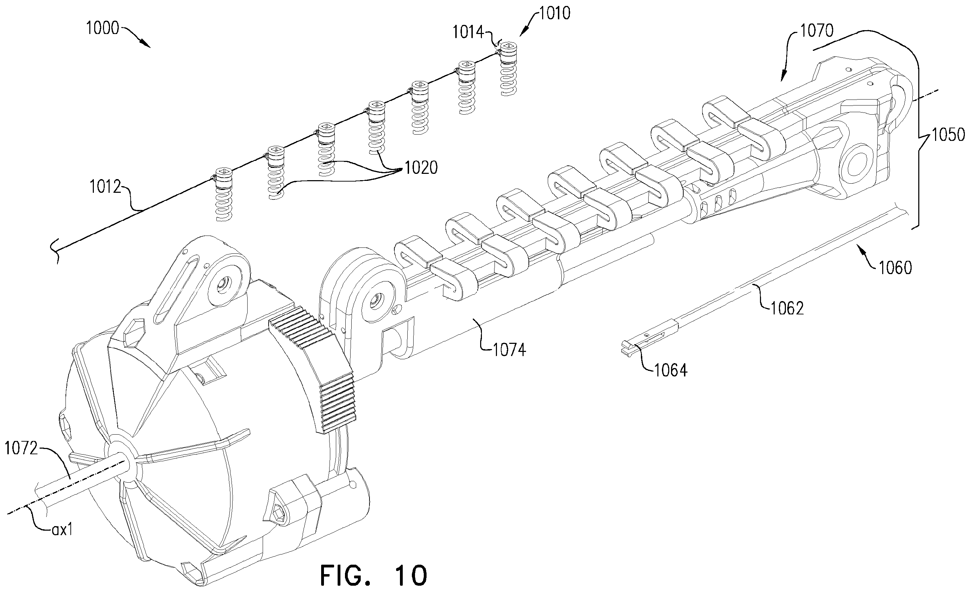

Reference is made to Figs. 10, 11A-B, 12A-B, 13A-E, 14A-E, and 15A-B, which are schematic illustrations of a system 1000 for use with a subject, in accordance with some applications. Fig. 10 shows an overview of system 1000, which comprises an implant 1010, and a delivery tool 1050 that comprises an anchor driver 1060 and a catheter device 1070. Implant 1010 comprises multiple tissue anchors 1020 and a tether (e.g., a wire or cord) 1012 on which the tissue anchors are threaded. As described in more detail hereinbelow, during implantation only a distal portion of tether 1012 remains implanted in the subject, while a proximal portion of the tether remains attached to delivery tool 1050. Nonetheless, for the sake of simplicity, implant 1010 is described herein as comprising the tether.

Tissue anchors 1020 are distributed in a series along tether 1012, and delivery tool 1050 can be used to implant implant 1010 by anchor driver 1060 being used, for each of anchors 1020 consecutively, to advance the anchor distally into the subject and to anchor the anchor to internal tissue of the subject. For example, and as shown, implant 1010 can be an annuloplasty implant, implanted by distributing anchors 1020 around at least a portion of an annulus of a native heart valve of the subject, such as the mitral or tricuspid valve. Further in some applications, a distal end of tether 1012 can be advanced distally into the subject along with the first anchor, and subsequent anchors can be advanced by sliding them distally along the tether. For some applications, system 1000 and/or techniques described for use therewith are used in combination with one or more of the systems and/or techniques described in US Provisional Patent Application 62/949,392 to Kasher et al., filed on December 17, 2019, and entitled ANNULOPLASTY AND TISSUE ANCHOR TECHNOLOGIES". For example, system 1000 can be used to deliver implant 210 of system 200 or implant 210 of system 600 described in US 62/949,392, mutatis mutandis. For example, anchor driver 1060 of system 1000 can be (or can be used as) the anchor driver of system 200 or 600 of US 62/949,392, and/or catheter 1072 of system 100 can be (or can be used as) the flexible tube of US 62/949,392, mutatis mutandis.

Device 1070 comprises a distal portion or a distal member, such as a catheter 1072, configured to be advanced into the subject. For some applications in which implant 1010 is an annuloplasty implant, and as shown, catheter 1072 is a transluminally (e.g., transfemorally) advanceable catheter. Device 1070 further comprises an extracorporeal unit (e.g., an extracorporeal unit) 1074, configured to remain outside the body of the subject. For some applications, extracorporeal unit 1074 defines, or is coupled to, a handle of device 1070.

Extracorporeal unit 1074 comprises a tensioner that comprises a winch 1080 that facilitates reducing slack on tether 1012 during implantation of implant 1010, e.g., during sliding of anchors distally along the tether. It is hypothesized that reducing slack advantageously reduces a likelihood of tether 1012 becoming twisted or entangled, or of inadvertent engagement of the tether with the anchor being delivered. It is further hypothesized that reducing slack using a winch, rather than by a human operator manually pulling on a proximal end of the tether, advantageously provides greater control over the magnitude and consistency of tension applied to the tether, and may further advantageously reduce the number of human operators required.

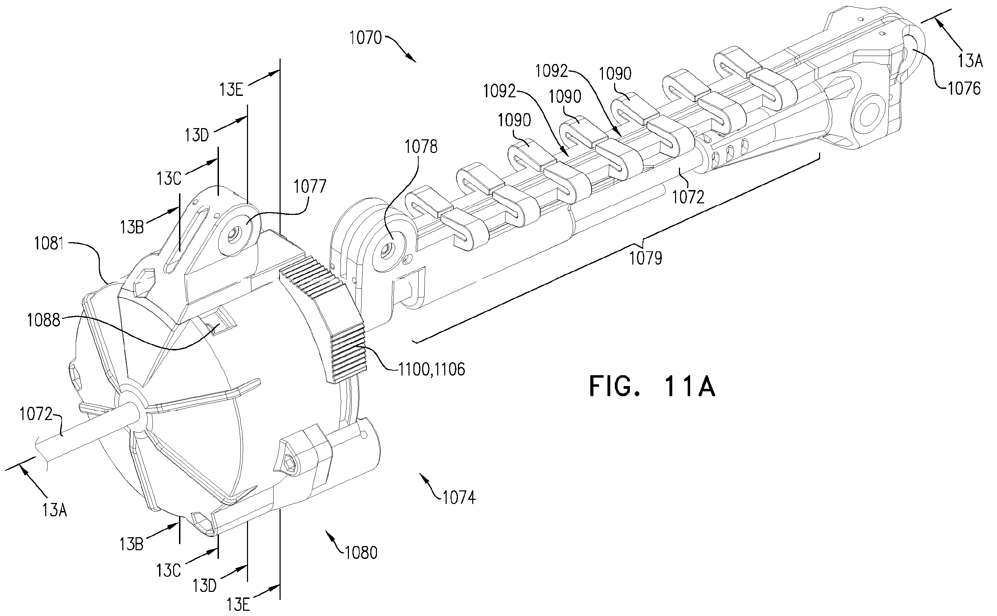

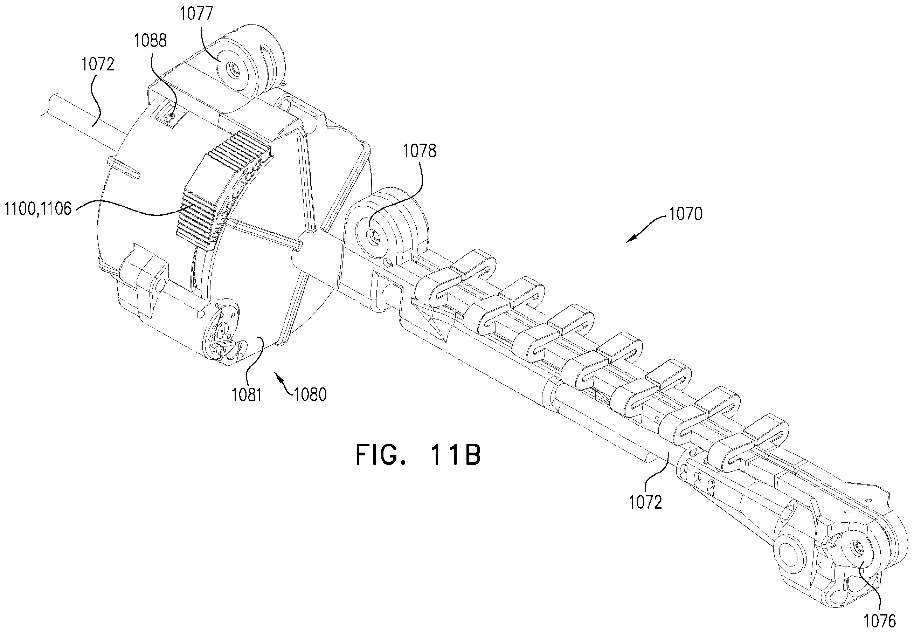

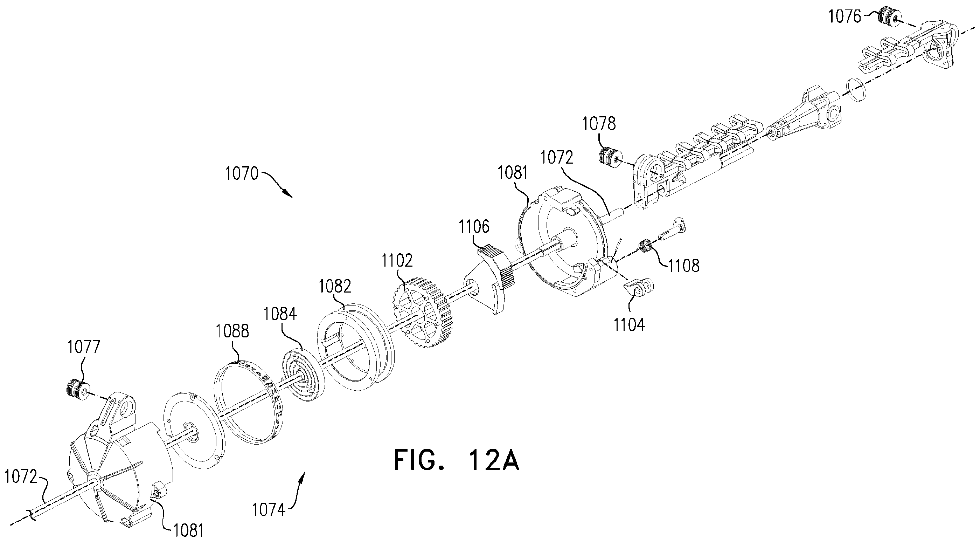

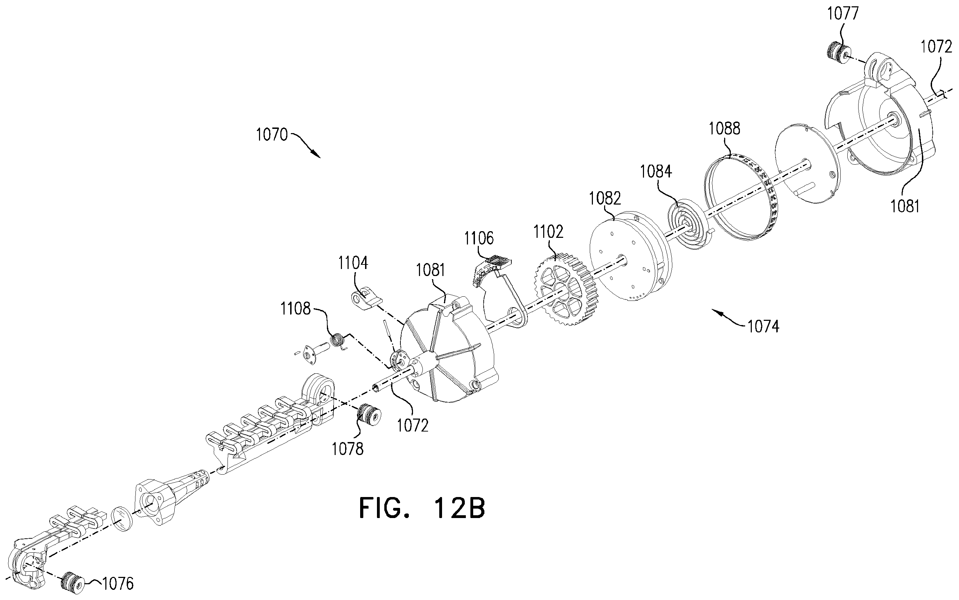

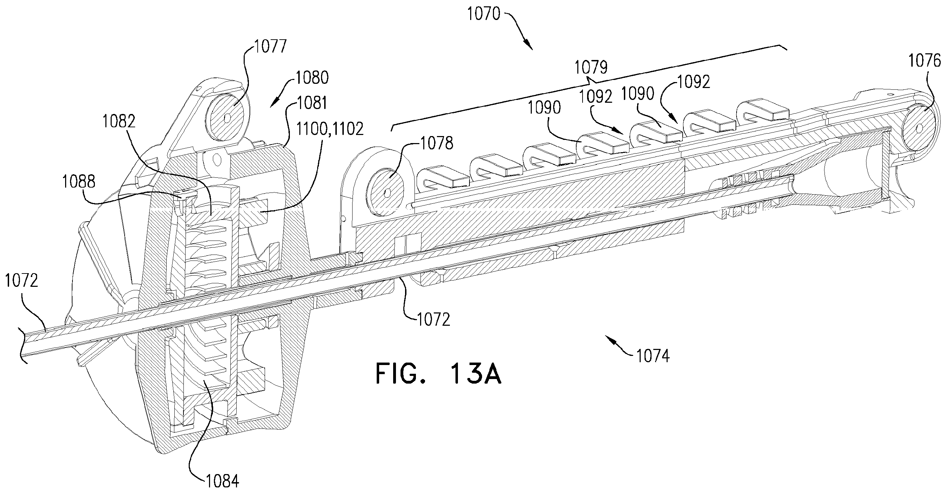

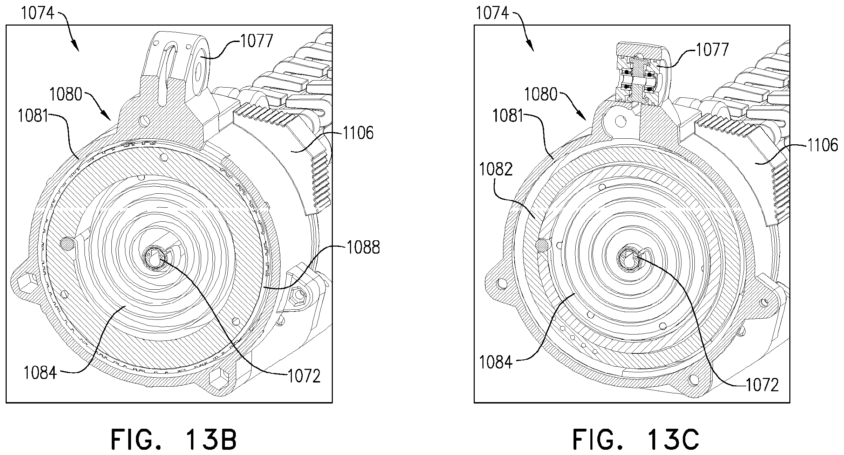

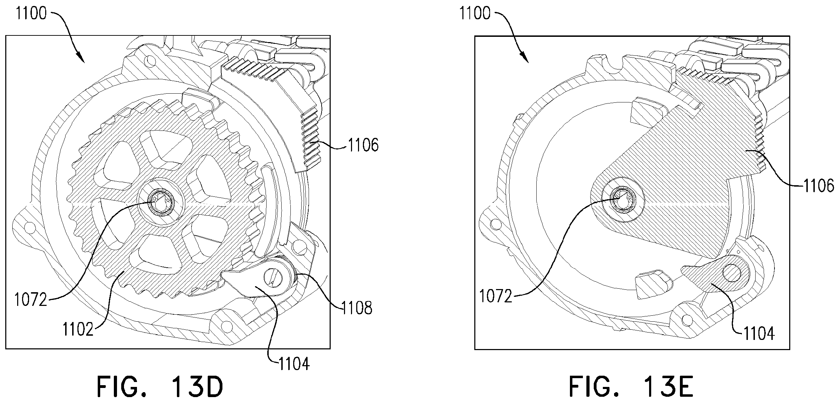

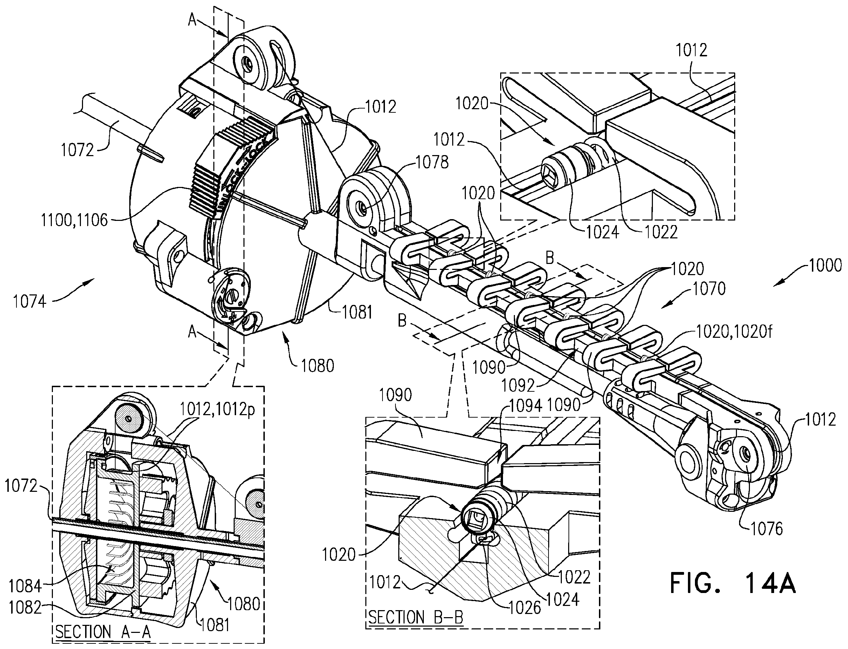

Figs. 11A-B show detailed views of catheter device 1070 (e.g., extracorporeal unit 1074 thereof) in the absence of implant 1010, Figs. 12A-B show exploded views of the catheter device, and Figs. 13A-E show cross-sections of the catheter device. Figs. 14A-E show system 1000 with implant 1010 mounted/loaded on delivery tool 1050, and the delivery tool being used with the implant. Fig. 14A shows a similar view of extracorporeal unit 1074 to that shown by Fig. 11A, but in the presence of implant 1010.

Winch 1080 comprises a spool 1082, and a spring 1084. Spool 1082 and spring 1084 can be housed in a housing 1081. Spool 1082 is coupled to spring 1084 such that rotation of the spool in a first rotational direction applies stress to the spring, which therefore pushes back in a second rotational direction, that is opposite to the first rotational direction.

Tether 1012 has a proximal portion 1012p that includes a proximal end of the tether, and a distal portion 1012d that includes a distal end of the tether. As shown in Fig. 14A, tether 1012 (e.g., proximal portion 1012p) is wound around spool 1082 such that pulling of the tether (e.g., distal portion 1012d) away from the winch unwinds the tether from the spool by rotating the spool in the first rotational direction. Such pulling of tether 1012 would typically occur during implantation of implant 1010, as anchors 1020, threaded on the tether, are consecutively advanced distally toward the implantation site. Because of the resulting stress on spring 1084, the spring pushes spool 1082 back in the second rotational direction, such that winch 1080 pulls in a proximal direction on tether 1012. Winch 1080 (e.g., spring 1084 thereof) can be configured such that the pulling force applied by the winch on tether 1012 is sufficiently great to take up slack between the winch and the distal end of the tether, but sufficiently small to allow manipulation of the tether necessary for implantation of implant 1010, e.g., as described hereinbelow. For example, the force can be (i) sufficiently small to allow anchor driver 1060 to advance, position, and anchor anchors 1020 to the target tissue site while the anchors are threaded on tether 1012, but (ii) significantly great to take up slack in the tether thereby reducing a likelihood of the tether becoming tangled or ensnared during this advancing, positioning, and anchoring of the anchors.

In some applications, spring 1084 is configured to provide a generally constant force over its range of motion (e.g., is a constant-force spring), such that the pulling force applied to tether 1012 is generally consistent, irrespective of how much the tether has been let out from winch 1080.

In some applications, and as shown, spring 1084 is a torsion spring, such as a spiral torsion spring. For example, spring 1084 can be a mainspring.

In some applications, each anchor 1020 comprises a tissue-engaging element 1022, and a head 1024 that defines an eyelet 1026 that is threaded onto tether 1012. As shown in Fig. 14A, multiple tissue anchors are distributed in a series along tether 1012, between winch 1080 (e.g., spool 1082 thereof) and the distal end of the tether. For example, and as shown, system 1000 can be provided with anchors 1020 distributed in a series on extracorporeal unit 1074 (e.g., a handle).

For some applications, and as shown (e.g., in Fig. 14A), the portion of tether 1012 immediately outside of winch 1080 extends proximally with respect to device 1070, and then turns to extend distally with respect to device 1070. It is to be noted that, despite this arrangement, anchors 1020 are distributed in a series along tether 1012 between winch 1080 and the distal end of the tether - meaning that, with respect to the tether (irrespective of the disposition of the tether with respect to device 1070 as a whole), the anchors are distributed along the tether in a series between the winch and the distal end of the tether.

For some applications, and as shown, this arrangement is facilitated by device by extracorporeal unit 1074 comprising a bearing (e.g., a pulley) 1076 (e.g., a proximal bearing) around which tether 1012 turns. For some such applications, device 1070 is provided with the distal end of tether 1012 already disposed beyond bearing 1076 - i.e., with the tether extending from winch 1080 proximally along extracorporeal unit 1074, and around bearing 1076, with the distal end of the tether disposed at least slightly distal to the bearing, e.g., aligned with and/or disposed opposite or inside an open proximal end (i.e., a proximal opening) of catheter 1072. For some such applications, the distal end of tether 1012 is inhibited from moving proximally from this position by a stopper 1014 that is fixedly attached to the distal end of the tether. Alternatively, catheter device 1070 is provided with the "distal end" of tether 1012 being the most proximally-positioned part of the tether with respect to the catheter device - e.g., extending proximally from winch 1080 and ending generally at a first anchor 1020f of implant 1010.

It is hypothesized that configuring device 1070 as described hereinabove, with tether 1012 extending proximally and then distally, advantageously positions anchors 1020 in a position, proximal to winch 1080, that is particularly accessible to the operator.

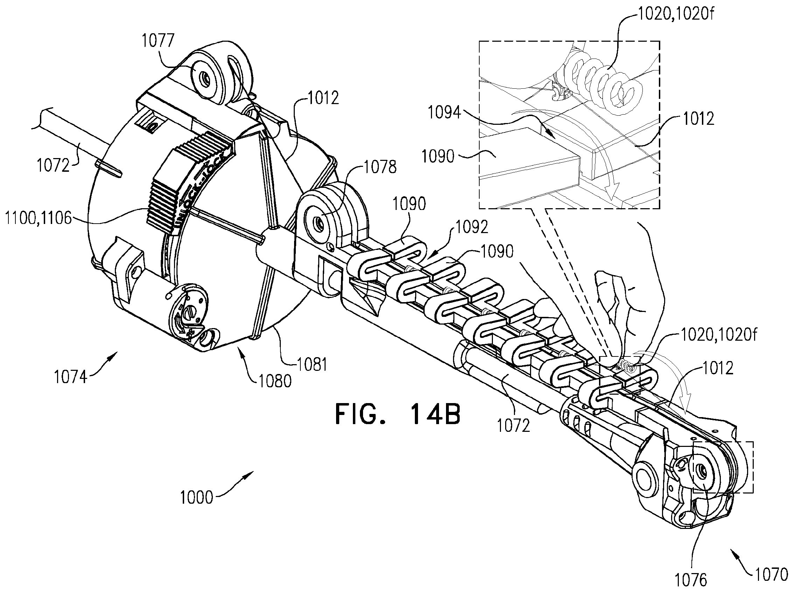

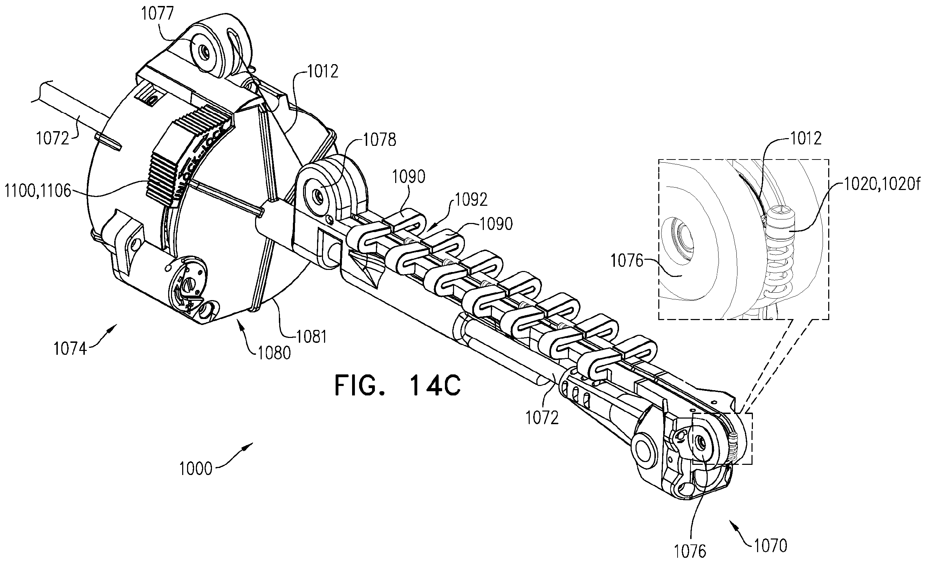

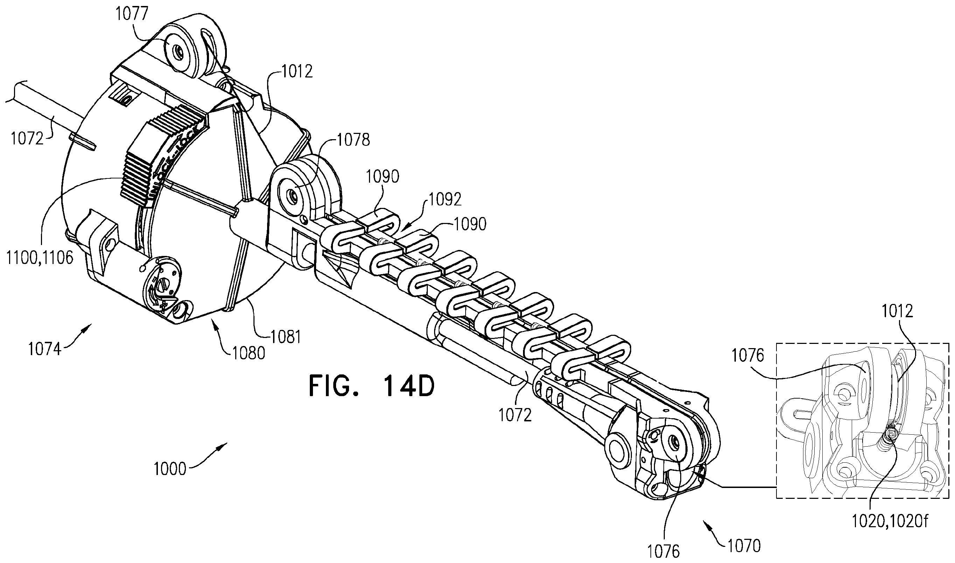

To deploy implant 1010, each anchor 1020, in series, is reversibly coupled to an anchor driver that advances the implant to a respective site within the body of the subject, such as a respective site on the annulus of a heart valve of the subject. Figs. 14B- show this being performed for first anchor 1020f of implant 1010. Anchor 1020f is advanced along tether 1012 toward the distal end of the tether. Because of the disposition of tether 1012 with respect to device 1070, this movement of anchor 1020f comprises moving the anchor proximally with respect to device 1070 (Fig. 14B), around bearing 1076 (Fig. 14C), and then distally (Fig. 14D).

For some applications, and as shown, the steps shown in Figs. 14B-D are performed manually by the human operator grasping and moving tissue anchor 1020 by hand. For some applications, modifications and/or additions to system 1000 eliminate the necessity for anchors 1020 to be grasped directly by hand, e.g., as described hereinbelow for system 1200 and/or system 1400, *mutatis mutandis.*

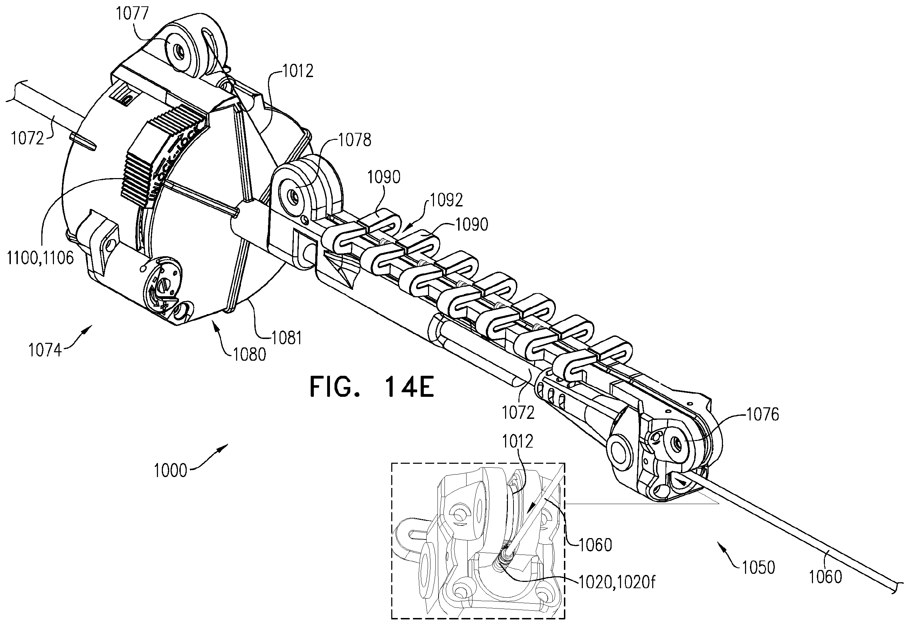

It is to be noted that each anchor 1020 is therefore initially oriented with its tissue-engaging element 1022 pointing proximally with respect to catheter device 1070, and subsequently becomes oriented with its tissue-engaging element pointing distally with respect to the catheter device. Once anchor 1020f is pointing distally (e.g., aligned with and/or disposed opposite or inside an open proximal end of catheter 1072), anchor driver 1060 is reversibly coupled to the anchor (e.g., to head 1024 thereof) (Fig. 14E), and is subsequently used to advance the anchor distally through catheter 1072 and to anchor the anchor to tissue of the subject. Anchor driver 1060 can comprise an elongate and flexible shaft 1062, and a driver head 1064 coupled to the distal end of the shaft.

For some applications, advancement of first anchor 1020f also pushes the distal end of tether 1012 through catheter 1072 to the tissue of the subject, e.g., by virtue of stopper 1014 (visible in Figs. 10, and 15A-B) fixedly attached to the distal end of the tether. For each of the subsequent anchors 1020, performing the same steps, mutatis mutandis, slides the anchor distally along tether 1012 toward the distal end of the tether and the first anchor.

15A-B show the implantation site after three anchors 1020 of implant 1010 have already been anchored to tissue 10 (e.g., tissue of the annulus of a heart valve) of the subject, and a fourth anchor is being delivered by anchor driver 1060, in accordance with some applications.

Fig. 15A illustrates that, when implanting an implant that is generally similar to implant 1010, in the absence of winch 1080, slack in the implant's tether may present a challenge, e.g., by increasing a likelihood of the tether becoming tangled, ensnared with another element of the implant and/or delivery system, and/or trapped between an anchor and the tissue into which the anchor has been driven. This is illustrated in Fig. 15A by slack in tether 1012 allowing distal portion 1012d of the tether (1) to form a bend 1013 that might become ensnared with a previously-anchored anchor (e.g., might loop around the most recently anchored anchor), and (2) to drift across the lumen and/or open distal end of catheter 1072 (indicated by reference numeral 1015) in a manner than increases a risk of the tether becoming ensnared with the tissue-engaging element 1022 of the anchor that is currently being delivered, and/or becoming sandwiched between the head 1024 of that anchor and the tissue.

Fig. 15B illustrates the same procedure, but with winch 1080 maintaining tension on tether 1012 such that slack is reduced (e.g., is eliminated), thereby reducing the likelihood of the undesirable outcomes described with reference to Fig. 15A.

For some applications, and as shown, spool 1082 circumscribes catheter 1072 (e.g., is concentric with the catheter). For some applications, and as shown, spool 1082 is mounted such that, at the longitudinal site of catheter 1072 at which winch 1080 is disposed, the rotation axis of the spool is parallel or coaxial with a lumen axis ax1 defined by the lumen of the catheter (which for some applications may be considered to be a central longitudinal axis of device 1070). It is hypothesized that such a configuration of winch 1080 advantageously provides increased weight balance of device 1070 (e.g., compared to winch 1080 being positioned laterally to catheter 1071), thereby facilitating its manipulation and stability.

For some applications, the position and orientation of winch 1080 (e.g., spool 1082 thereof) is facilitated by one or more bearings in addition to proximal bearing 1076. For example, and as shown, tether 1012 leaves spool 1082 laterally, and a bearing (e.g., a pulley) 1077 redirects the tether proximally (and often also medially). It is to be noted that, in this context, the terms "lateral," "laterally," "medial," and "medially" denote positions and directions with respect to the central longitudinal axis of device 1070. For some applications another bearing (e.g., another pulley) 1078 redirects the tether to be generally parallel with catheter 1072 until it reaches proximal bearing 1076. Therefore, for some applications, bearings 1076 and 1078 together define therebetween a region 1079 in which tether 1012 is generally parallel with catheter 1072 (and with the central longitudinal axis of device 1070). In some applications, and as shown in Fig. 14A, system 1000 is provided with anchors 1020 disposed within region 1079.

For some applications, extracorporeal unit 1074 of catheter device 1070 comprises a plurality of separators 1090 that define anchor-storage zones 1092 therebetween, each of the anchor-storage zones can be dimensioned to store a single anchor 1020. Separators 1090 allow the storage, on extracorporeal unit 1074, of anchors 1020 threaded in series on tether 1012. Separators 1090 can be shaped and/or positioned to obstruct anchors 1020 while not obstructing tether 1012. For example, and as shown, tether 1012 can extend through region 1079 on a given line (e.g., at a given lateral distance from axis ax1), and separators 1090 can be disposed slightly away (e.g., further laterally) from that line, such that anchors 1020, which protrude from the tether on which they are threaded, reach separators 1090. For applications in which device 1070 comprises bearings 1076 and 1078, the line on which tether 1012 extends through region 1079 is often determined by the position of these bearings.

For some applications, each separator 1090 can define a slot 1094 therein through which tether 1012 can be moved. For example, the grasping and moving of an anchor 1020 out of its anchor-storage zone 1092 can include lifting the anchor over one or more separators 1090, which may be facilitated by the slot 1094 of the one or more separators allowing tether 1012 to move through the slot (e.g., as shown in Fig. 14B). For some applications, this lifting of anchor 1020, along with tether 1012 on which it is threaded, is further facilitated by winch 1080 temporarily allowing some of the tether to unspool responsively the tether being pulled, and subsequently respooling the tether.

For some applications, winch 1080 comprises a reversibly-activatable ratchet 1100. While activated (i.e., in an activated state), ratchet 1100 allows spool 1082 to rotate in the first rotational direction (e.g., allows pulling of tether 1012 to unspool the tether), and inhibits rotation of the spool in the second rotational direction (e.g., inhibits spring 1084 from respooling the tether). While deactivated (i.e., in a deactivated state), ratchet 1100 allows spool 1082 to rotate in both the first rotational direction and the second rotational direction. In some applications, ratchet 1100 comprises a gear 1102, a pawl 1104, and a switch 1106. Gear 1102 is coupled to spool 1082 such that rotation of the spool rotates the gear (e.g., the gear and the spool are rotationally locked). Pawl 1104 is reversibly engageable with gear 1102, such that when the pawl is engaged with the gear, ratchet 1100 is activated, and when the pawl is disengaged from the gear, the ratchet is deactivated. Switch 1106 is configured to switch ratchet 1100 between being activated and deactivated by moving pawl 1104 between being engaged with gear 1102 and being disengaged from the gear. For example, and as shown, switch 1106 can define a cam that pushes pawl 1104 out of engagement with gear 1102. For some such applications, pawl 1104 can be biased toward a position in which it is engaged with gear 1102, e.g., by a ratchet spring 1108.

As described hereinabove, while activated, ratchet 1100 allows the operator to cause unspooling of tether 1012 (e.g., by advancing the distal end of the tether away from winch 1080), but inhibits the winch from respooling the tether. Therefore, activation of ratchet 1100 somewhat disables winch 1080. As described hereinabove, during advancement of the first anchor 1020, the first anchor may pull with it the distal end of tether 1012 all the way through catheter 1072. It is hypothesized that, for some applications, activating ratchet 1100 advantageously facilitates this advancement by allowing the operator to distally advance the anchor and tether gradually and/or stepwise, without competing with winch 1080 pulling the anchor and tether proximally. It is further hypothesized that ratchet 1100 provides the operator with the ability to temporarily introduce slack into tether 1012 should the operator wish to do so, e.g., in order to manually lift a subsequent anchor 1020 and move it into position for engagement with driver 1060, or while making fine adjustments to the position of the distal end of catheter 1072, with which tension in tether 1012 might interfere. Slack may be introduced into tether by activating ratchet 1100, and then manually pulling tether 1012 in order to unspool an amount of the tether from spool 1082. Subsequently, slack may be removed by deactivating the ratchet.

It is to be noted that winch 1080 can be configured (e.g., by configuring the spring strength of spring 1084) such that the tension that the winch applies to tether 1012 is insufficient to cause premature contraction of implant 1010. For example, for applications in which implant 1010 is an annuloplasty implant, spring 1084 can be insufficiently strong to cause significant contraction of the valve annulus into which anchors 1020 are driven. For some applications, spring 1084 is only just strong enough to remove slack from tether 1012.

Fig. 15A is described hereinabove as resulting from implanting an implant in the absence of winch 1080. However, it is to be noted that the state shown in Fig. 15A may also be achieved by implanting an implant with ratchet 1100 activated.

For some applications, winch 1080 further comprises an indicator 1088 that indicates how much tether 1012 has been unspooled from spool 1082, e.g., by indicating how much the spool has rotated. For example, and as shown, indicator 1088 can be fixedly coupled to spool 1082, and can indicate turning/unspooling through a static window. For some applications, and as shown, indicator 1088 can indicate turning/unspooling numerically. In some applications, other symbols or representations, such as color, can be used. As shown, indicator 1088 can be a ring that is rotationally locked with spool 1082.

Reference is now made to Figs. 16A-F, which are schematic illustrations of a system 1200 for use with a subject, in accordance with some applications. System 1200 comprises implant 1010, and a delivery tool 1250 that comprises an anchor driver (e.g., driver 1060) and a catheter device 1270. System 1200 can be identical to system 1000, except for the addition of a series of cartridges 1220, and adaptations of the catheter device (e.g., the extracorporeal unit thereof) to facilitate the use of the cartridges as described herein. Therefore, for some applications, catheter device 1270 (e.g., an extracorporeal unit 1274 thereof) can be identical to catheter device 1070 (e.g., extracorporeal unit 1074 thereof) except for adaptations for use with cartridges 1220, such as those described hereinbelow.

For some applications, extracorporeal unit 1274 comprises a bearing (e.g., a pulley) 1276 (e.g., a proximal bearing) around which tether 1012 turns. Bearing 1276 can be similar or identical to bearing 1076, described hereinabove.

Each cartridge 1220 holds (e.g., cradles) a respective tissue anchor 1020, and facilitates the operator manipulating the tissue anchor in a manner that is generally similar to that described for system 1000, mutatis mutandis. However, it is hypothesized that, for some applications, cartridges 1220 provide increased control and/or safety.

Fig. 16A shows an initial state of device 1270, with each of cartridges 1220 coupled to extracorporeal unit 1274 in a respective initial position. This is analogous to the state shown in Fig. 14A for system 1000.

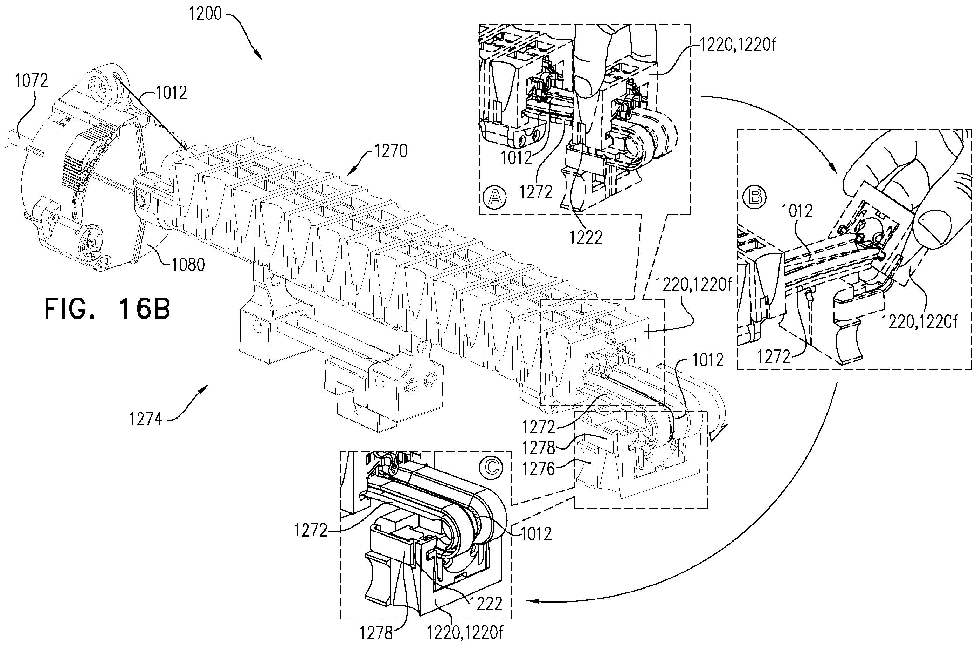

Extracorporeal unit 1274 comprises or defines at least one track 1272 (e.g., a groove, as shown, or a rail) along which each cartridge 1220 is moveable (e.g., slidable), while remaining coupled to the extracorporeal unit, from the respective initial position to a deployment position in which the cartridge holds its tissue anchor 1020 opposite or inside the open proximal end of catheter 1072. An example of such movement is shown in Fig. 16B, which shows, for a first cartridge 1220f (which holds first anchor 1020f), the initial position (inset A), an intermediate position (inset B), and the deployment position (inset C and main picture). This can be performed manually by the operator, who grasps the cartridge by hand. It is hypothesized that cartridges 1220 are more easily grasped and manually moved than are anchors 1020 alone.

In some applications, each cartridge 1220 is configured to lock to extracorporeal unit 1274 upon arriving at the deployment position. Such a configuration can be achieved, for example, using a latch mechanism, e.g., with extracorporeal unit 1274 comprising one or more latches 1278, and each cartridge 1220 being correspondingly shaped to be locked to by the one or more latches, for example, by each cartridge defining a corresponding one or more recesses 1222. Latches 1278 can be elastic or spring-loaded, such that they transiently flex (e.g., outward) responsively to the arrival of cartridge 1220, and then automatically lock to recesses 1222 upon cartridge 1220 becoming fully positioned at the deployment position (e.g., snapping/clicking into place).

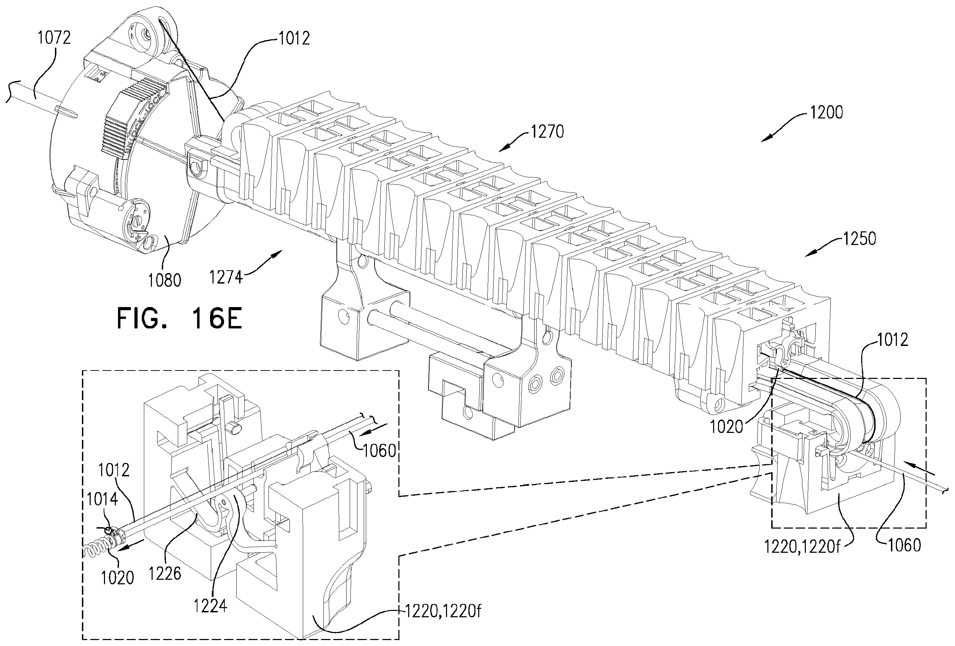

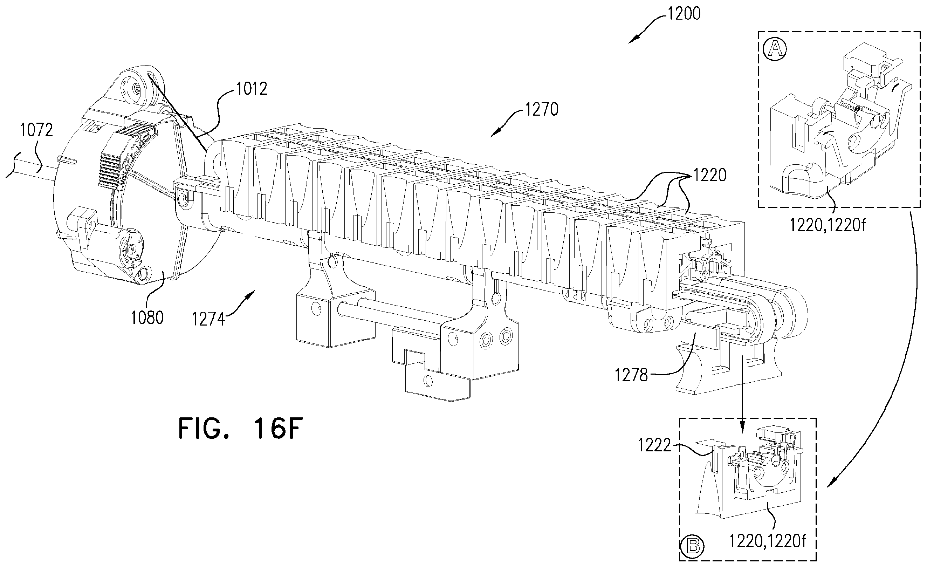

While cartridge 1220 is disposed in the deployment position, anchor driver 1060 is used to advance the anchor 1020 distally out of the cartridge (Fig. 16E) and through catheter 1072, e.g., in order to anchor the anchor to the target tissue site. Anchor driver 1060 is subsequently decoupled from anchor 1020 and can be withdrawn from device 1270. Cartridge 1220 is subsequently removable from the deployment position (e.g., entirely removable from extracorporeal unit 1274) such that the deployment position is vacant for a successive cartridge in the series (Fig. 16F). For example, and as shown, recesses 1222 can be open at one end in order to facilitate slipping out of the cartridge (e.g., a downward direction as shown) without requiring flexion of latches 1278.

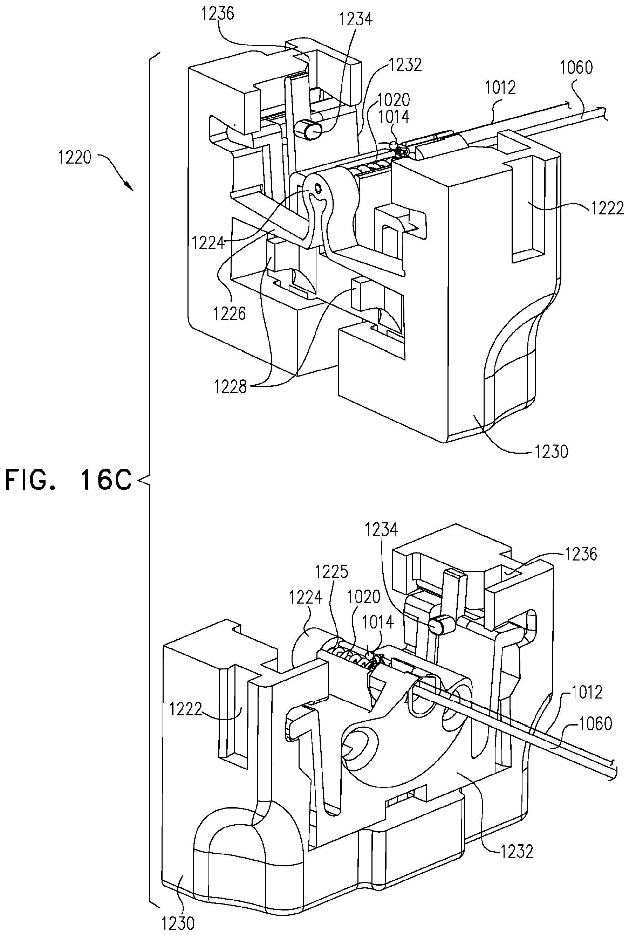

Figs. 16C-D illustrate a testing feature of system 1200, in accordance with some applications. This testing feature tests (or facilitates testing of) the coupling of anchor driver 1060 to anchor 1020 and allows distal advancement of the anchor only if this testing is successful. It is hypothesized that this advantageously reduces a likelihood of inadvertently distally advancing an anchor that is imperfectly coupled to the anchor driver (e.g., merely pushing the anchor distally). Such imperfect coupling might result in an anchor being advanced but with the anchor driver being unable to screw the anchor into the tissue or to proximally withdraw the anchor.

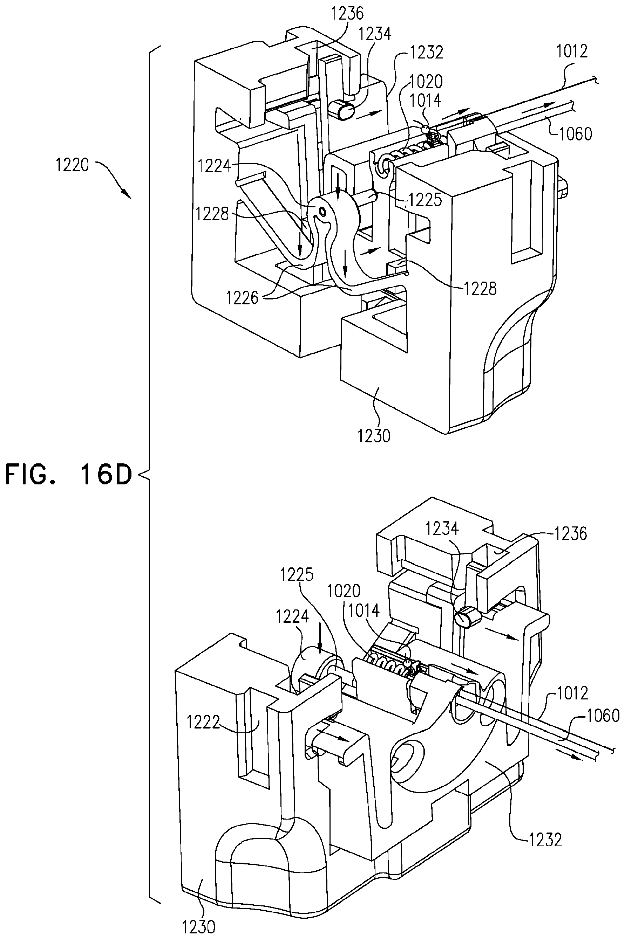

For applications in which system 1200 comprises this testing feature, each cartridge 1220 comprises a displaceable barrier 1224 that obstructs anchor 1020 from being advanced distally out of the cartridge until testing is successful (Fig. 16C). Cartridge 1220 can be provided with barrier 1224 in this obstructing position. For such applications, each cartridge can further comprise a displacement mechanism 1226 that, upon actuation of the displacement mechanism, displaces barrier 1224 such that the barrier ceases to obstruct anchor 1020 from being advanced distally out of the cartridge. Once driver 1060 has been coupled to anchor 1020, the coupling is tested by applying a force to the anchor, for example, by the operator pulling proximally on driver 1060 such that the driver pulls the anchor proximally (Fig. 16D). Cartridge 1220 is configured such that this pulling actuates (or allows actuation of) displacement mechanism 1226 only if driver 1060 is properly coupled to anchor 1020 and can therefore apply sufficient pulling force to the anchor. For example, if the coupling is imperfect, driver 1060 may become decoupled from anchor 1020 before sufficient pulling force is applied.

For some applications, displacement mechanism 1226 is spring-loaded, e.g., comprising an elastic element that is provided in a constrained state (Fig. 16C), and that becomes released by the force applied to anchor 1020 (Fig. 16D). For example, cartridge 1220 can comprise one or more detents 1228 that constrain displacement mechanism 1226 (Fig. 16C), and that are moved away from the displacement mechanism when sufficient pulling force is applied to anchor 1020, such that they cease to constrain the displacement mechanism, and the displacement mechanism responsively moves barrier 1224 such that the barrier ceases to obstruct the anchor from being advanced distally out of the cartridge (Fig. 16D).

For some applications, and as shown, each cartridge 1220 comprises a first piece 1230 that comprises or defines barrier 1224, and a second piece 1232 that holds (e.g., cradles) anchor 1020. For some such applications, displacement mechanism 1226 is configured to be actuated by anchor driver 1060 pulling anchor 1020 proximally with sufficient force that the anchor pulls second piece 1230 proximally with respect to first piece 1232 such that detents 1228 cease to constrain the displacement mechanism (Fig. 16D).

For some applications, and as shown, second piece 1232 comprises or defines detent(s) 1228, and first piece 1230 comprises or defines displacement mechanism 1226 (and optionally also barrier 1224).

For some applications, detent functionality can alternatively or additionally be provided by a detent 1225 (e.g., a post) that is a component of first piece 1230, e.g., coupled to or defined by barrier 1224. In the obstructing position of barrier 1224, detent 1225 extends into the central lumen defined by the helical tissue-engaging element of anchor 1020. Upon application of sufficient pulling force to anchor 1020, the helical tissue-engaging element of the anchor slides off of detent 1225, allowing barrier 1224 to be displaced.

For some applications, first piece 1230 is a first monolithic structure made from a single piece of material. For some applications, second piece 1232 is a second monolithic structure made from a single piece of material.

For some applications, and as shown, second piece 1232 couples cartridge 1220 to extracorporeal unit 1274, e.g., by being slidably engaged with track 1272. As shown, for applications in which track 1272 is a groove, second piece 1232 can define a tongue 1234 that couples the first piece (and thereby cartridge 1220 as a whole) to extracorporeal unit 1274 by engaging the groove. For the sake of clarity, extracorporeal unit 1274 and track 1272 are not shown in Figs. 16C-D.

For some applications, cartridge 1220 is configured such that the movement of second piece 1232 proximally with respect to first piece 1230 reconfigures the cartridge into a removable state that facilitates removal of the cartridge from the deployment position. For example, and as shown in Fig. 16C, before the pulling, the position of tongue 1234 with respect to first piece 1232 is such that the first piece obstructs the tongue from disengaging from track 1272. In the example shown, each track 1272 is disposed medially from its respective tongue 1234, and the disengagement would comprise the tongue moving laterally outward. As shown in Fig. 16D, the pulling repositions tongue 1234 such that the first piece no longer obstructs such movement. For example, and as shown, the pulling may align tongue 1234 (or a portion of first piece 1230 that supports the tongue) with a cavity 1236 defined by first piece 1230 (Fig. 16D). Once thus aligned, cartridge 1220 can be moved away from track 1272 by pulling the cartridge away from the track (e.g., by the operator grasping the cartridge by hand), because this alignment allows tongue 1234 to deflect outward (e.g., transiently) responsively to the pulling, and thereby disengage from the track (Fig. 16F). The deployment position is thereby made vacant for a successive cartridge to be used.

It is to be noted that, as described hereinabove, for some applications cartridge 1220 is configured such that successful verification of the coupling between anchor driver 1060 and anchor 1020 reconfigures the cartridge to allow both (i) distal advancement of the anchor out of the cartridge into catheter 1072, and (ii) removal of the cartridge in order to vacate the deployment position for a successive cartridge.

For some applications, cartridge 1220 is configured to be removed while driver 1060 remains extended into catheter 1072. For some applications, cartridge 1220 is configured to be removed only after driver 1060 has been withdrawn from catheter 1072.

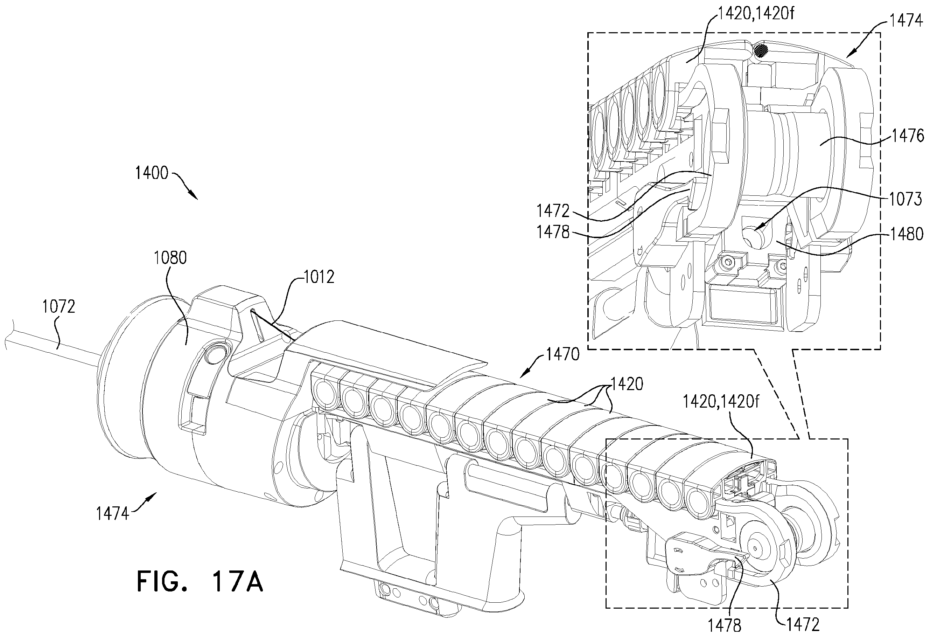

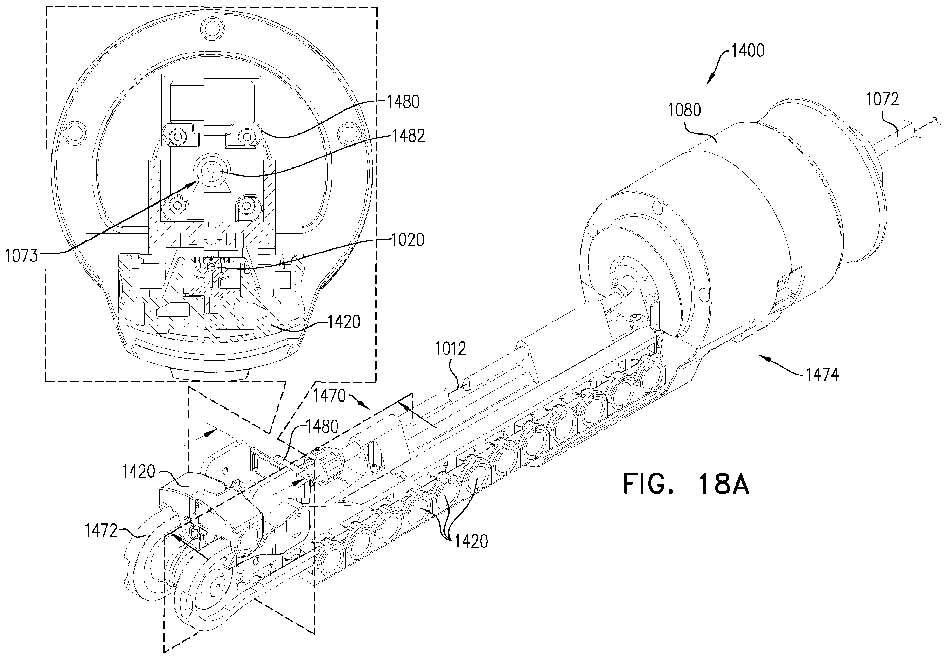

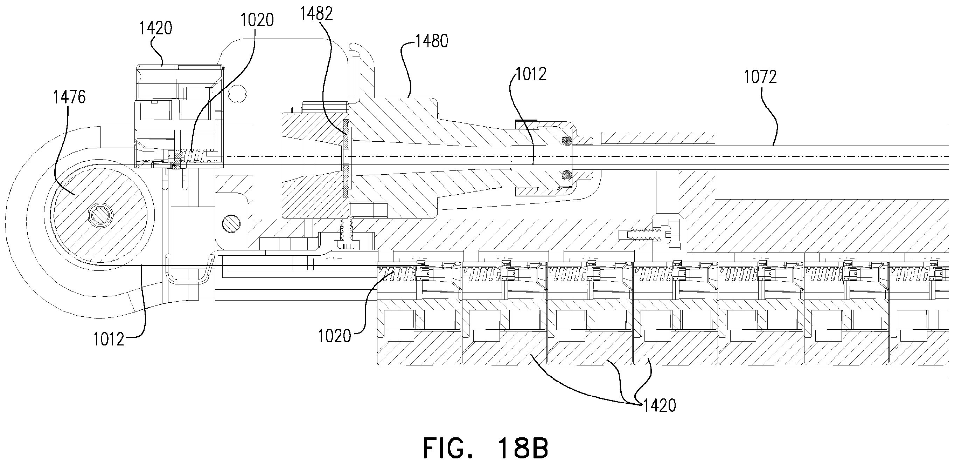

Reference is now made to Figs. 17A-F and 18A-B which are schematic illustrations of an example system 1400 for use with a subject, in accordance with some applications. System 1400 comprises implant 1010, and a delivery tool 1450 that comprises an anchor driver (e.g., driver 1060) and a catheter device 1470. System 1400 can be identical, *mutatis mutandis,* to system 1000, except for the addition of a series of cartridges 1220, and adaptations of the catheter device (e.g., of the extracorporeal unit thereof) to facilitate the use of the cartridges as described herein. For some applications, system 1400 can be as described for system 1200, except for the use of cartridges 1420 instead of cartridges 1220, and corresponding adaptations of the catheter device (e.g., of the extracorporeal unit thereof). Therefore, for some applications, catheter device 1470 (e.g., an extracorporeal unit 1474 thereof) can be identical to catheter device 1070 (e.g., extracorporeal unit 1074 thereof) and/or to catheter device 1270 (e.g., extracorporeal unit 1274 thereof) except for adaptations for use with cartridges 1420, such as those described hereinbelow.

Although tensioner (e.g., winch) 1080 is illustrated differently in system 1400, it can have the same or similar componentry and functionality as described hereinabove.

For some applications, extracorporeal unit 1474 comprises a bearing (e.g., a pulley) 1476 (e.g., a proximal bearing) around which tether 1012 turns. Bearing 1476 can be similar or identical to bearing 1076 and/or 1276, described hereinabove.

Each cartridge 1420 holds (e.g., cradles) a respective tissue anchor 1020, and facilitates the operator manipulating the tissue anchor in a manner that is generally similar to that described for system 1200, *mutatis mutandis.*

Fig. 17A shows an initial state of device 1470, with each of cartridges 1420 coupled to extracorporeal unit 1474 in a respective initial position. This is analogous to the state shown in Fig. 16A for system 1200.

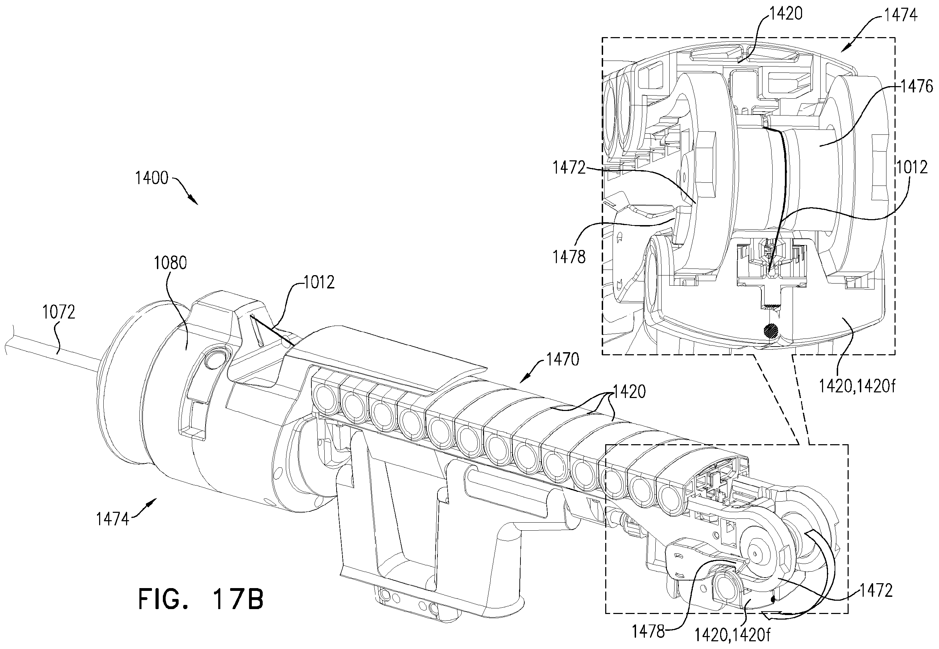

In some applications, extracorporeal unit 1474 comprises or defines at least one track 1472 (e.g., a groove, as shown, or a rail) along which each cartridge 1420 is moveable (e.g., slidable), while remaining coupled to the extracorporeal unit, from the respective initial position to a deployment position in which the cartridge holds its tissue anchor 1020 opposite or inside the open proximal end (i.e., proximal opening) 1073 of catheter 1072. An example of such movement is shown in Fig. 17B, in which a curved arrow indicates movement of a first cartridge 1420f (which holds first anchor 1020f), from its initial position to its deployment position, e.g., similarly to as described for cartridge 1220 with reference to Fig. 16B, *mutatis mutandis.* This can be performed manually by the operator, who grasps the cartridge by hand. It is hypothesized that cartridges 1420 are more easily grasped and manually moved than are anchors 1020 alone.

In some applications, each cartridge 1420 is configured to lock to extracorporeal unit 1474 upon arriving at the deployment position. Such a configuration can be achieved, for example, using a latch mechanism - e.g., with extracorporeal unit 1474 comprising one or more latches 1478, and each cartridge 1420 being correspondingly shaped to be locked to by the one or more latches. Latches 1478 can be elastic or spring-loaded, such that they transiently flex (e.g., upward) responsively to the arrival of cartridge 1420, and then automatically lock to the cartridge upon the cartridge becoming fully positioned at the deployment position (e.g., snapping/clicking into place).

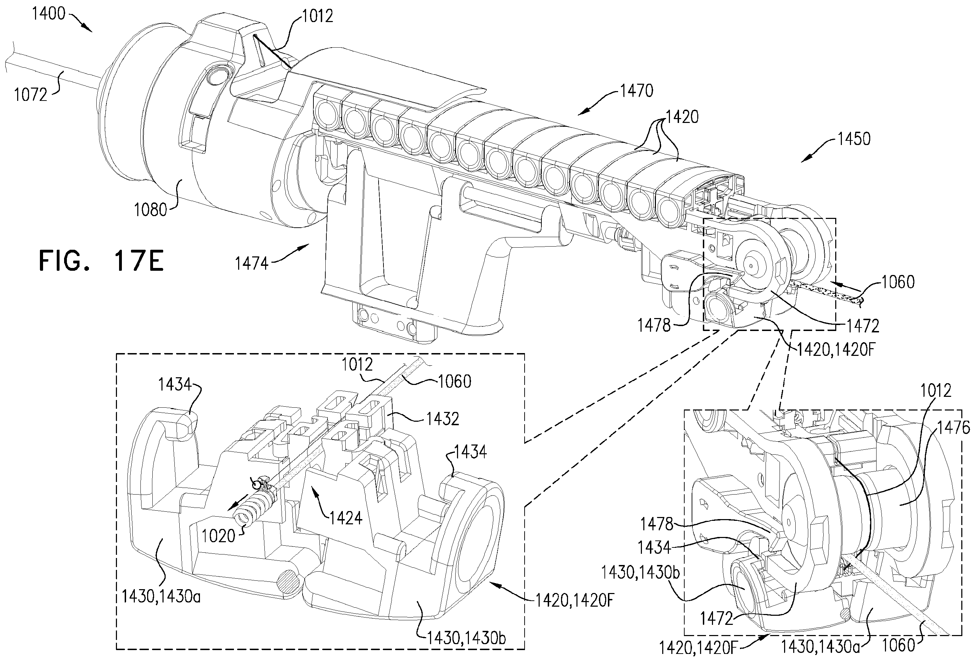

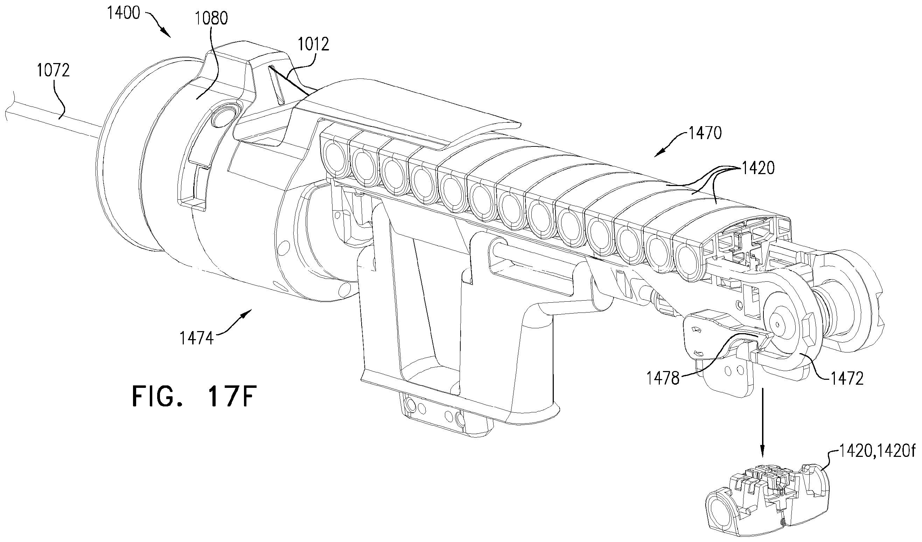

In some applications, while cartridge 1420 is disposed in the deployment position, anchor driver 1060 is used to advance the anchor 1020 distally out of the cartridge (Fig. 17E) and through catheter 1072, e.g., in order to anchor the anchor to the target tissue site. Anchor driver 1060 is subsequently decoupled from anchor 1020 and can be withdrawn from device 1470. Cartridge 1420 is subsequently removable from the deployment position (e.g., entirely removable from extracorporeal unit 1474) such that the deployment position is vacant for a successive cartridge in the series (Fig. 17F). For example, and as shown, cartridge 1420 can be shaped to facilitate slipping out of the cartridge (e.g., in a downward direction as shown) without requiring flexion of latches 1478.

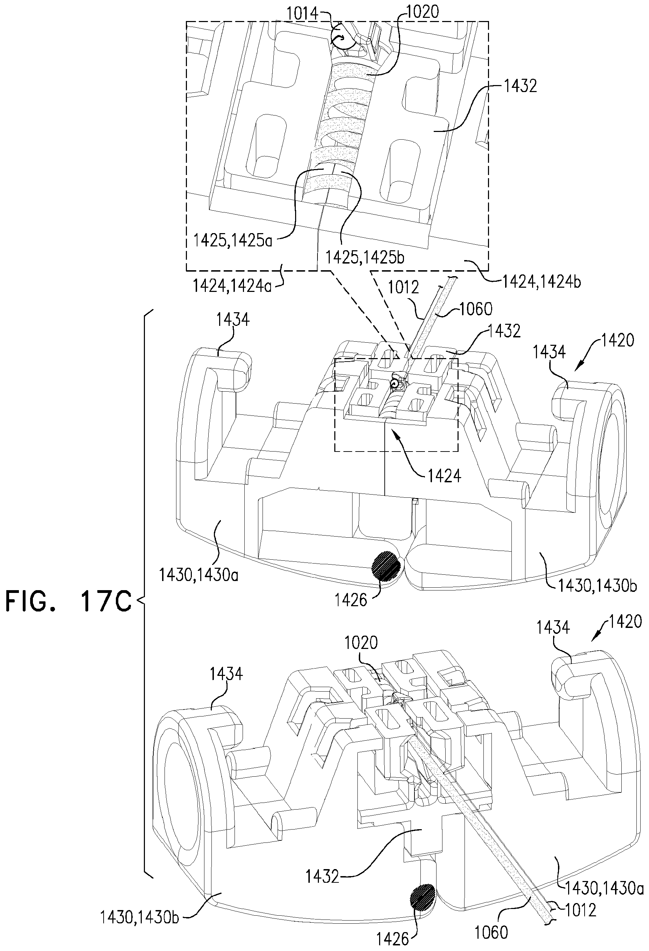

Figs. 17C-D illustrate an example testing feature of system 1400, in accordance with some applications. This testing feature tests (or facilitates testing of) the coupling of anchor driver 1060 to anchor 1020 and allows distal advancement of the anchor only if this testing is successful. It is hypothesized that this advantageously reduces a likelihood of inadvertently distally advancing an anchor that is imperfectly coupled to the anchor driver (e.g., merely pushing the anchor distally). Such imperfect coupling might result in an anchor being advanced but with the anchor driver being unable to screw the anchor into the tissue or to proximally withdraw the anchor.

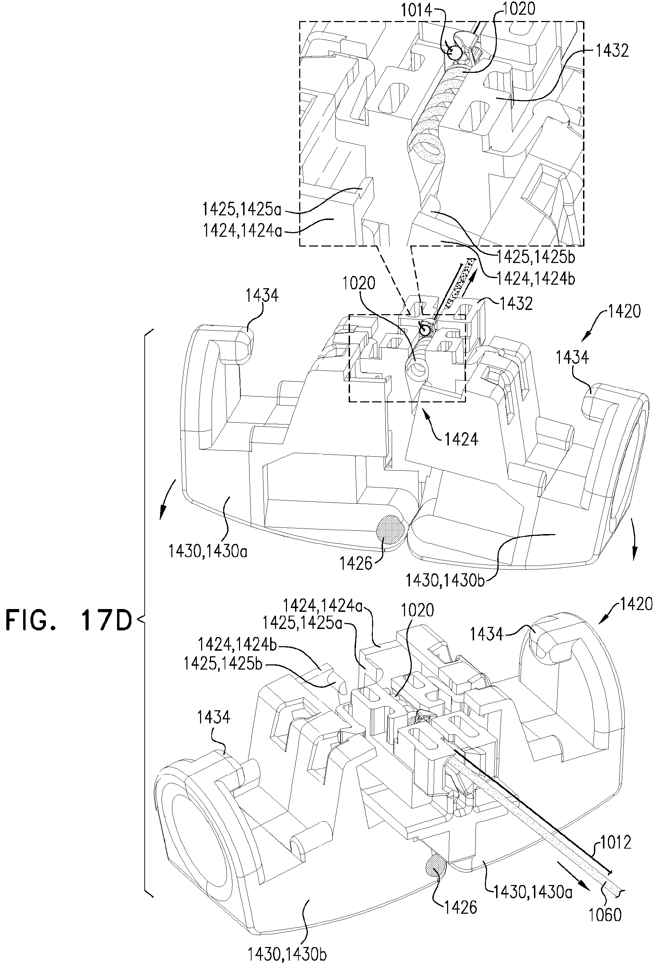

For applications in which system 1400 comprises this testing feature, each cartridge 1420 comprises a displaceable barrier 1424 that obstructs anchor 1020 from being advanced distally out of the cartridge until testing is successful (Fig. 17C). Cartridge 1420 can be provided with barrier 1424 in this obstructing position (Fig. 17C). For such applications, each cartridge can further comprise a displacement mechanism 1426 that, upon actuation of the displacement mechanism, displaces barrier 1424 such that the barrier ceases to obstruct anchor 1020 from being advanced distally out of the cartridge. Once driver 1060 has been coupled to anchor 1020, the coupling is tested by applying a force to the anchor, for example, by the operator pulling proximally on driver 1060 such that the driver pulls the anchor proximally (Fig. 17D). Cartridge 1220 is configured such that this pulling actuates (or allows actuation of) displacement mechanism 1426 only if driver 1060 is properly coupled to anchor 1020 and can therefore apply sufficient pulling force to the anchor. For example, if the coupling is imperfect, driver 1060 may become decoupled from anchor 1020 before sufficient pulling force is applied.

For cartridge 1220, described hereinabove, displacement mechanism 1226 can displace barrier 1224 by linear displacement of the barrier. In contrast, for cartridge 1420, displacement mechanism 1426 can displace barrier 1424 by deflection of the barrier. For example, and as shown, displacement mechanism 1426 can comprise a hinge about which barrier 1424 deflects. For some applications, and as shown, the displacement of barrier 1424 can involve the barrier separating into (e.g., two) barrier subcomponents 1424a and 1424b that deflect in different directions away from each other.

Cartridge 1420 can comprise one or more detents 1425 that inhibit (e.g., constrain) displacement mechanism 1426 from moving barrier 1424 (Fig. 17C), and that are moved away when sufficient pulling force is applied to anchor 1020, such that they cease to inhibit (e.g., constrain) the displacement mechanism, thereby allowing (or triggering) actuation of the displacement mechanism.

For some applications, and as shown, detents 1425 are coupled to or defined by barrier 1424. In the obstructing position of barrier 1424, detents 1425 extend into the central lumen defined by the helical tissue-engaging element of anchor 1020. Upon application of sufficient pulling force to anchor 1020, the helical tissue-engaging element of the anchor slides off of detents 1425, allowing barrier 1424 to be displaced.

For some applications, and as shown, each barrier subcomponent can have its own detent 1425, e.g., barrier subcomponent 1424a has a detent 1425a, and barrier subcomponent 1424b has a detent 1425b, both of the detents extending into the central lumen defined by the helical tissue-engaging element of anchor 1020 while in the obstructing position. For some such applications, anchor 1020 itself (e.g., the tissue-engaging element thereof) thereby inhibits the deflection of barrier subcomponents 1424a and 1424b away from each other.

For some applications, and as shown, in the obstructing position of barrier 1424 multiple detents 1425 can collectively define a post, e.g., for applications in which barrier 1424 has two barrier subcomponents, each detent can be semicylindrical such that the two detents collectively form a cylindrical post (see inset of Fig. 17C).

For some applications, displacement mechanism 1426 is spring-loaded, e.g., comprising an elastic element such as a spring that is provided in a constrained state, and that becomes released by the force applied to anchor 1020. For applications in which displacement mechanism 1426 is spring-loaded, the displacement mechanism automatically moves barrier 1424 responsively to the movement away from detents 1425 such that the barrier ceases to obstruct the anchor from being advanced distally out of the cartridge (Fig. 17D).

For some applications, and as shown, each cartridge 1420 comprises a first piece 1430 that comprises or defines barrier 1424, and a second piece 1432 holds (e.g., cradles) anchor 1020. For some such applications, displacement mechanism 1426 is configured to be actuated (or is allowed to become actuated) by anchor driver 1060 pulling anchor 1020 proximally with sufficient force that the anchor pulls second piece 1432 proximally with respect to first piece 1430 such that detents 1425 cease to inhibit (e.g., constrain) the displacement mechanism (Fig. 16D).

For some applications, and as shown, first piece 1430 comprises or defines detents 1425 and displacement mechanism 1426. For some such applications, and as shown, first piece 1430 comprises two sub-pieces 1430a and 1430b, hingedly coupled to each other by displacement mechanism 1426, and each sub-piece comprises a respective barrier subcomponent and a respective detent 1425.

For some applications, each of sub-pieces 1430a and 1430b is a monolithic structure made from a single piece of material. For some applications, second piece 1432 is a monolithic structure made from a single piece of material.

For some applications, first piece 1430 couples cartridge 1420 to extracorporeal unit 1474, e.g., by being slidably engaged with track 1472. For example, and as shown, first piece 1430 can define at least one tongue 1434 (e.g., one tongue per sub-piece 1430a and 1430b) that couples the first piece (and thereby cartridge 1420 as a whole) to extracorporeal unit 1474 by slidably engaging track 1472. For the sake of clarity, extracorporeal unit 1474 and track 1472 are not shown in Figs. 17C-D.

For some applications, and as shown, cartridge 1420 is shaped to be locked to by latches 1478 by the latches latching onto tongues 1434. That is, for such applications, tongues 1434 both (i) slidably couple cartridge 1420 to track 1472, and (ii) facilitate locking of the cartridge in the deployment position.

For some applications, cartridge 1420 is configured such that the movement of second piece 1432 proximally with respect to first piece 1430 reconfigures the cartridge (or allows the cartridge to be reconfigured) into a removable state that facilitates removal of the cartridge from the deployment position. For example, and as shown in Fig. 17C, before the pulling, each tongue 1434 is positioned so as to slidably engage track 1472, obstructing cartridge 1420 from disengaging from track 1472. As shown in Fig. 16D, the pulling of second piece 1432 proximally disengages each tongue 1434 (or allows the tongue to be disengaged) from track 1472, e.g., by the tongues moving laterally, thereby allowing the cartridge to be pulled away from the track and, in some applications, from extracorporeal unit 1474 as a whole (Fig. 17F). The deployment position is thereby made vacant for a successive cartridge to be used.

For some applications, and as shown, it is the same deflection between sub-pieces 1430a and 1430b that both (i) displaces barrier 1424 by deflecting barrier subcomponents 1424a and 1424b with respect to each other, and (ii) disengages tongues 1434 from track 1472. For such applications, both this displacement and this disengagement are therefore facilitated (or triggered) by the proximal pulling of anchor 1020, with sufficient pulling force, by driver 1060.

It is therefore to be noted that, as described hereinabove, for some applications cartridge 1420 is configured such that successful verification of the coupling between anchor driver 1060 and anchor 1020 reconfigures the cartridge to allow both (i) distal advancement of the anchor out of the cartridge into catheter 1072, and (ii) removal of the cartridge in order to vacate the deployment position for a successive cartridge.

For some applications, cartridge 1420 is configured such that even once tongues 1434 are disengaged from track 1472, the cartridge remains in place until manually removed, e.g., by being grasped and pulled by the operator.

For some applications, cartridge 1420 is configured to be removed while driver 1060 remains extended into catheter 1072. For some applications, cartridge 1420 is configured to be removed only after driver 1060 has been withdrawn from catheter 1072.

Figs. 18A-B show system 1400 after (i) the anchor 1020 of first cartridge 1420f (i.e., the first anchor) has been advanced (with tether 1012 coupled thereto) through catheter 1072 and anchored to tissue, (ii) driver 1060 has been withdrawn, (iii) the first cartridge has been removed, and (iv) a subsequent cartridge 1420 has been moved into the deployment position. Tether 1012 extends proximally from the first anchor, back through catheter 1072. Thus, when the subsequent anchor is advanced through catheter 1072 it will be slid over and along tether 1012.

Reference is again made to Figs. 16A-19B. As described hereinabove, for some applications each of cartridges 1220 and 1420 can comprise (i) a first piece that defines the barrier of the cartridge, and (ii) a second piece that holds anchor 1020, and that is pullable proximally with respect to the first piece so as to move the barrier in order to allow subsequent advancement of the anchor into tube 1072. It is to be noted that in system 1200, cartridge 1220 is slidably coupled to the track of the proximal unit by the first piece (i.e., the piece that holds the anchor), whereas in system 1400, cartridge 1420 is slidably coupled to the track of the proximal unit by the second piece (i.e., the piece that defines the barrier).

It is to be noted that, although systems 1200 and 1400 are shown hereinabove as comprising tensioner (e.g., winch) 1080, aspects of these systems - in particular the use of cartridges 1220 and 1420 - can be used independently of tensioner 1080 (or of any tensioner). Thus, the scope of the present disclosure includes variants of systems 1200 and 1400 that do not comprise tensioner 1080, and that may not comprise any tensioner.

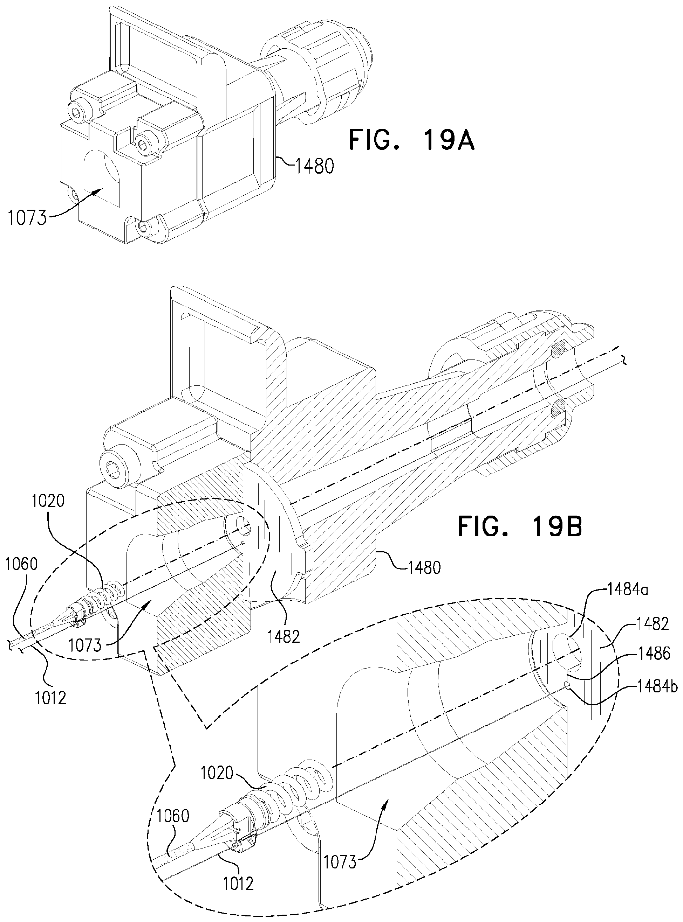

Reference is again made to Figs. 17A-18B, and reference is further made to Figs. 19A-B, which are schematic illustrations of a port 1480, in accordance with some applications. For some applications, a port 1480 is disposed at proximal opening 1073 of catheter 1072. Although this is shown as a component of system 1400, port 1480 can be used in other systems described herein, *mutatis mutandis.* Port 1480 can have a tapered lumen that facilitates smooth advancement of anchors 1020 into catheter 1072.

Port 1480 can comprise a membrane 1482 that provides hemostatic sealing during the implantation procedure. Membrane 1482 can be formed from a silicone. The material (e.g., the silicone) from which membrane 1482 is formed can have a hardness of 38-42 (e.g., 40) Shore A. Membrane 1482 can be about 1 mm thick. Membrane 1482 can be oriented substantially transversely to the proximal end of tube 1072.

Membrane 1482 can be shaped to define two apertures 1484 therethrough (e.g., a first aperture 1484a and a second aperture 1484b), connected by a closed slit 1486. In some applications, first aperture 1484a is larger (e.g., at least twice as large, e.g., at least three times larger, e.g., 3-10 times larger, such as at least 4 times larger) in diameter than second aperture 1484b. For example, first aperture 1484a can be 1.5-2.5 mm (e.g., 1.7-2.2 mm, e.g., 1.8-2.0 mm, such as 1.9 mm) in diameter, whereas second aperture 1484b can be 0.2-0.7 mm (e.g., 0.2-0.6 mm, e.g., 0.3-0.5 mm, such as 0.4 mm) in diameter.

As shown, port 1480 (e.g., membrane 1482 thereof) can be oriented such that first aperture 1484a lies on the axis along which driver 1060 and the tissue-engaging element of anchor 1020 is advanced. This "anchor-advancement axis" is indicated by a broken line. While a cartridge 1420 is in the deployment position, the tissue-engaging element of its tissue anchor 1020 can be aligned with first aperture 1484a, thereby defining the anchor-advancement axis from the tissue anchor, through the first aperture, and through tube 1072.

As also shown, second aperture 1484b typically lies on the axis along which tether 1012 is advanced. Each anchor can be advanced through membrane 1482 with (i) its central longitudinal axis and/or tissue-engaging element aligned with first aperture 1484a, and (ii) its eyelet, which is threaded onto tether 1012, aligned with second aperture 1484b.

It is to be noted that typically neither aperture 1484a (and thereby the anchor-advancement axis) nor aperture 1484b is aligned centrally with respect to catheter 1072. Rather, the center of first aperture 1484a is disposed on one side of the central axis of the catheter, and the center of second aperture 1484b is disposed on the opposite side of the central axis of the catheter. However, for applications in which first aperture 1484a is sufficiently large, the first aperture may overlap the central axis of the catheter (despite nonetheless not being centered on the central axis of the catheter).

As the anchor passes membrane 1482 distally, apertures 1484 and slit 1486 responsively open or widen transiently and then close or re-narrow behind the anchor.

In some applications, aperture 1484a is dimensioned to seal around driver 1060 (e.g., the shaft thereof), which can be narrower than the head of anchor 1020. For example, for some applications the diameter of aperture 1484a is 80-120 percent (e.g., 90-110 percent) the thickness of the shaft of driver 1060.

In some applications, aperture 1484b is dimensioned to seal around tether 1012, which is narrower than the eyelet of anchor 1020. For example, for some applications the diameter of aperture 1484b is 50-200 (e.g., 80-120 percent, such as 90-110 percent) the thickness of tether 1012.

When driver 1060 is withdrawn proximally through membrane 1482, tether 1012 remains extended through second aperture 1484b.

It is hypothesized that the double-aperture configuration of membrane 1482 advantageously provides better hemostatic sealing for the implantation procedure compared to other configurations, such as single larger aperture or slit. For example, during anchoring of an anchor 1012, at which time tether 1012 and driver 1060 extend through membrane 1482, slit 1486 is typically closed.

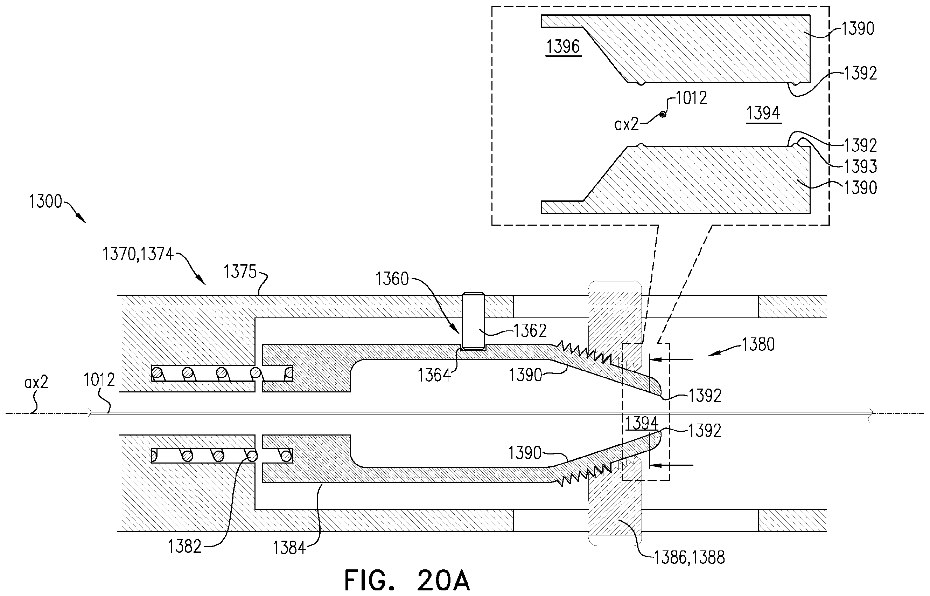

Reference is made to Figs. 20A-H, which are schematic illustrations of a system 1300, for use with a subject, in accordance with some applications. System 1300 comprises a catheter device 1370 that comprises a distal portion or distal member, such as catheter 1072, and an extracorporeal unit 1374 that comprises a housing 1375 and a tensioner 1380. System 1300 also comprises an implant such as implant 1010, and an anchor driver such as anchor driver 1060. Catheter device 1370 can be similar to catheter device 1070 and/or catheter device 1270, except for the presence of tensioner 1380 (e.g., instead of tensioner / winch 1080), mutatis mutandis.

In some applications, as described hereinabove, implant 1010 comprises tether 1012 and anchors 1020. Tether 1012 extends from extracorporeal unit 1374, distally through catheter device 1370 to a distal portion of the tether, the distal portion being disposed distally from the tensioner and including a distal end of the tether. As described in more detail hereinbelow, tensioner 1380 is configured to pull proximally on tether 1012. Although the way in which this proximal pulling is achieved is different from that described for system 1000, the advantage hypothesized is similar - reducing slack on tether 1012 during implantation (or at least during certain steps of implantation).

In some applications, in systems 1000 and 1200, anchors 1020 of implant 1010 are threaded onto tether 1012 distally from the tensioner (winch) - i.e., the anchors are threaded onto the tether between the tensioner and the distal end of the tether. Therefore, in order to take up slack at the distal portion of the tether, tension applied by the tensioner must pass through the portion of the tether on which as-yet unused anchors are threaded, such that slack slides through the as-yet unused anchors. In contrast, in system 1300, anchors 1020 are threaded onto tether 1012 proximally from tensioner 1380. It is hypothesized that, at least for some applications, such a configuration provides certain advantages, such as enabling these as-yet unused anchors to be stored in an arrangement that is convenient or ergonomic but that is less conducive to sliding of tether 1012 therethrough. For example, while the as-yet unused anchors of systems 1000 and 1200 are stored in a linear arrangement, those of system 1300 might be stored in a non-linear arrangement, such as in a ring.

Anchor driver 1060 is configured be reversibly coupled to each of the anchors consecutively, and to advance the current anchor along the tether, distally past (e.g., through) tensioner 1380. Figs. 20A-H illustrate at least some steps in advancement of one of the anchors. As described hereinbelow, system 1300 has certain features that facilitate the positioning of tensioner 1380 between the stored as-yet unused anchors and the distal end of the tether. These features facilitate anchor driver 1060 advancing anchor 1020 distally past (e.g., through) tensioner 1380, without the anchor or the driver (e.g., shaft 1062 thereof) inhibiting, or being inhibited by, the tensioner.

Tensioner 1380 can comprise a spring 1382 that is coupled to housing 1375, and that is reversibly lockable to tether 1012 such that, while the spring is under stress and is locked to the tether, the spring causes the tensioner to pull proximally on the tether. For some applications, and as shown, spring 1382 is a compression spring, is configured to be stressed by compression, and is configured to pull proximally on the tether by extending. Optionally, spring 1382 can be a tension spring, or another suitable type of spring.

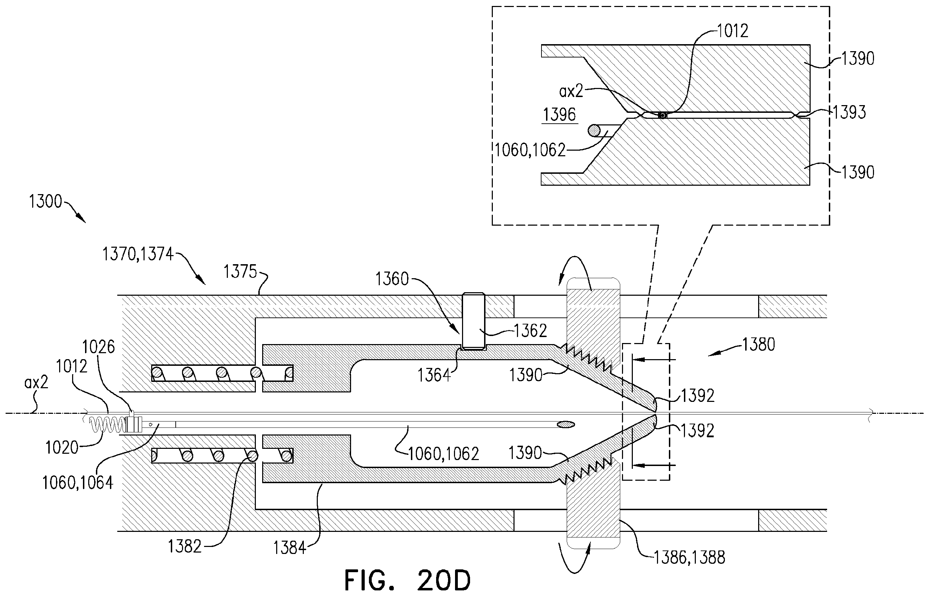

Tensioner 1380 can further comprise a clamp 1384, coupled to spring 1382, and reversibly transitionable between an unclamped state (Figs. 20A-C) and a clamped state (Figs. 20D-F). Tether 1012 extends distally through catheter device 1370 such that the tether extends through clamp 1384 in a manner in which, when the clamp is in the clamped state, spring 1382 is locked to the tether. For example, and as shown, a tether axis ax2, along which tether 1012 extends through tensioner 1380, may pass between jaws 1390 of clamp 1384.

Tensioner 1380 can further comprise an actuator 1386, actuation of the actuator transitioning clamp 1384 between the unclamped state and the clamped state. For some applications, and as shown, actuator 1386 comprises a ring 1388 that circumscribes the clamp, rotation of the ring transitioning the clamp between the unclamped state and the clamped state. For example, clamp 1384 and ring 1388 may collectively function as a chuck, e.g., with the clamp biased toward assuming its unclamped state, and rotation of the ring squeezing the clamp closed. For example, an inner screw thread 1389 defined by an inner surface of ring 1388, and a reciprocal outer screw thread 1385 defined by an outer surface of clamp 1384, may interact such that rotation of the ring screws the ring over the clamp. For some applications, the inner surface of ring 1388 and/or the outer surface of clamp 1384 is tapered such that axial movement of the ring with respect to the clamp (e.g., screwing of the ring over the clamp) squeezes the clamp toward its clamped state. In some applications, ring 1388 is configured to be gripped and rotated by hand.

In some applications, clamp 1384 defines first and second clamp surfaces 1392, configured to engage tether 1012 when the clamp is in its clamped state. For example, clamp 1384 can comprise first and second clamp jaws 1390, each of the clamp jaws defining a respective clamp surface 1392, and being deflectable by squeezing, e.g., by actuator 1386. In the unclamped state, clamp 1384 defines a gap 1394 between clamp surfaces 1392, tissue anchors 1020 being dimensioned to be advanced, by anchor driver 1060, along tether 1012, distally through the gap. For example, a greatest transverse width of each anchor 1020 (measured transverse to the longitudinal axis of the anchor, along which the anchor is driven into tissue) can be smaller than gap 1394. In some applications, anchor driver 1060 (e.g., driver head 1064 and shaft 1062) is also narrower than gap 1394.

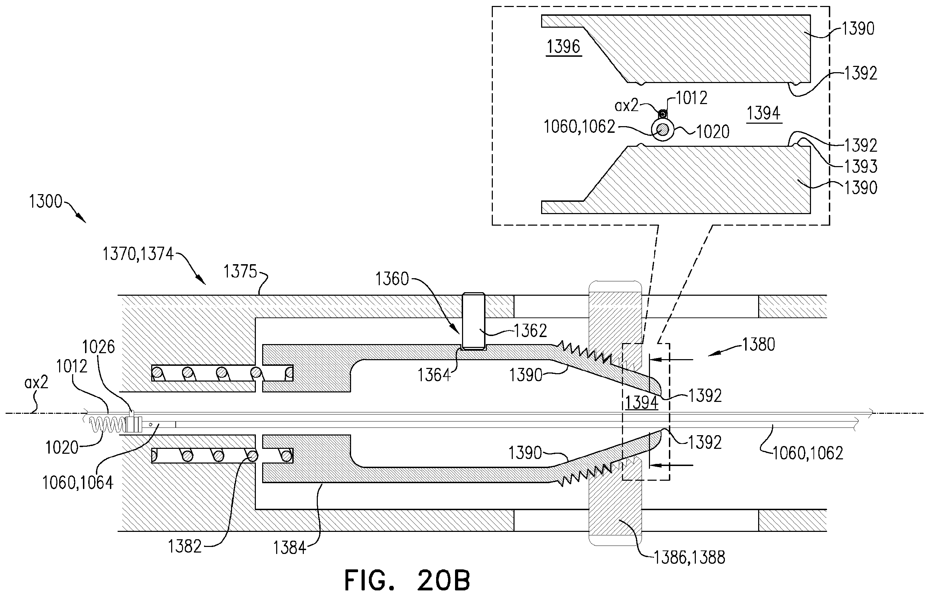

Fig. 20A shows tensioner 1380 with tether 1012 extending therethrough, from a proximal side of the tensioner (right-side of the figure) distally past (e.g., through) the tensioner, e.g., extending through and out of catheter 1072, e.g., after a first tissue anchor has already been anchored to tissue, and prior to a second tissue anchor being advanced. Fig. 20B shows anchor driver 1060, with driver head 1064 reversibly coupled to anchor 1020, having been used to advance the anchor distally past (e.g., through) tensioner 1380. The inset transverse cross-section of Fig. 20B illustrates that both tether 1012, and shaft 1062 of driver 1060, are disposed in gap 1394 between clamp surfaces 1392 of jaws 1390.

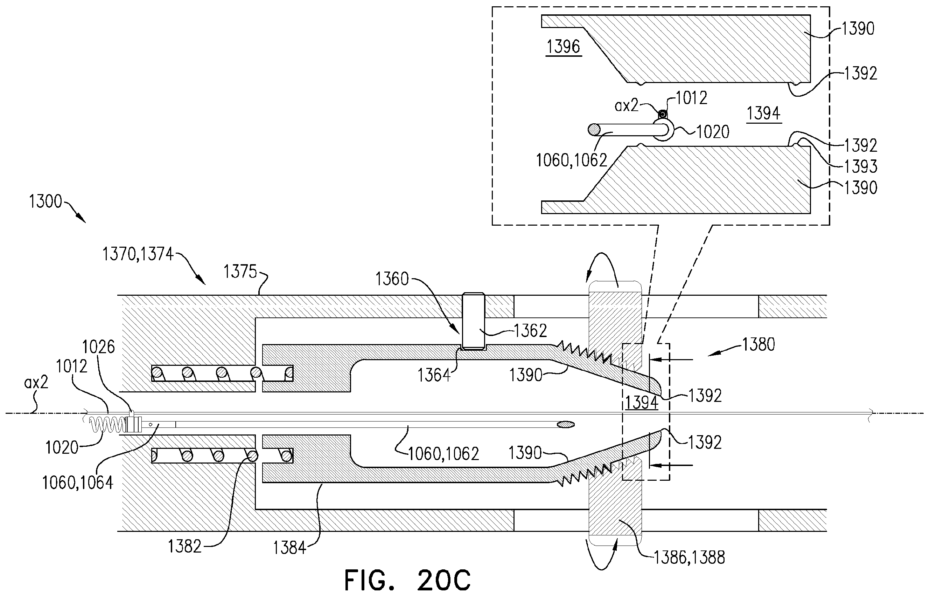

Prior to transitioning clamp 1384 into its clamped state, at least part of shaft 1062 of driver 1060 is shifted laterally away from tether axis ax2 (and thereby from tether 1012), into a non-clamping zone 1396 defined by catheter device 1370 (e.g., by extracorporeal unit 1374 thereof) (Fig. 20C). This is performed while driver head 1064 remains coupled to anchor 1020, which itself remains threaded on tether 1012, and therefore often involves a degree of deflection and/or bending of shaft 1062. This is most clearly illustrated in the inset transverse cross-section, the plane of which is at gap 1394, at which shaft 1062 is disposed within non-clamping zone 1396, laterally from gap 1394. This is also represented in the main longitudinal cross-section by shaft 1062 coming out of the plane of the cross-section, and therefore being "cut" by the cross section at a point distal from clamp surfaces 1392.

Therefore, non-clamping zone 1396 is shaped and dimensioned to accommodate shaft 1062 being shifted laterally after driver 1060 has advanced anchor 1020 along tether 1012, distally past tensioner 1380, such that the shaft extends distally toward the anchor, bypassing clamp 1384.

For some applications, catheter device 1370 (e.g., extracorporeal unit 1374 thereof) provides non-clamping zone 1396 by clamp surfaces 1392 being eccentric with respect to tether axis ax2 - e.g., as shown in the inset transverse cross-section.

While shaft 1062 remains in non-clamping zone 1396, actuator 1386 is actuated (e.g., ring 1388 is rotated), thereby transitioning clamp 1384 into the clamped state, such that the clamp clamps tether 1012 between clamp surfaces 1392 of jaws 1390, thereby locking the clamp (and therefore spring 1382) to the tether (Fig. 20D). It is to be noted that, as described hereinabove, the position of shaft 1062 in non-clamping zone 1396 allows clamp 1384 to clamp tether 1012 without clamping shaft 1062, and without the shaft obstructing the clamping of the tether.

For some applications, and as shown, at least one of jaws 1390 is shaped to define one or more bulges 1393, which limit the proximity of clamp surfaces 1392 to each other in the clamped state, e.g., in order to reduce a likelihood of clamp 1384 damaging and/or cutting tether 1012 by clamping too tightly.

In Figs. 20A-D, spring 1382 is in a stressed state in which the spring is under stress and is locked in that state. In some applications, this locking is provided by a lock 1360 that can be a component of extracorporeal unit 1374. As shown in the transition between Fig. 20C to Fig. 20D, clamp 1384 can be transitionable into its clamped state while lock 1360 remains locked.

For some applications, and as shown, lock 1360 locks spring 1382 in its stressed state by locking clamp 1384 to housing 1375. For some applications, and as shown, lock 1360 comprises a detent 1362 and a recess 1364, protrusion of the detent into the recess locking the lock. For some such applications, and as shown, clamp 1384 defines recess 1364, and housing 1375 comprises the detent.

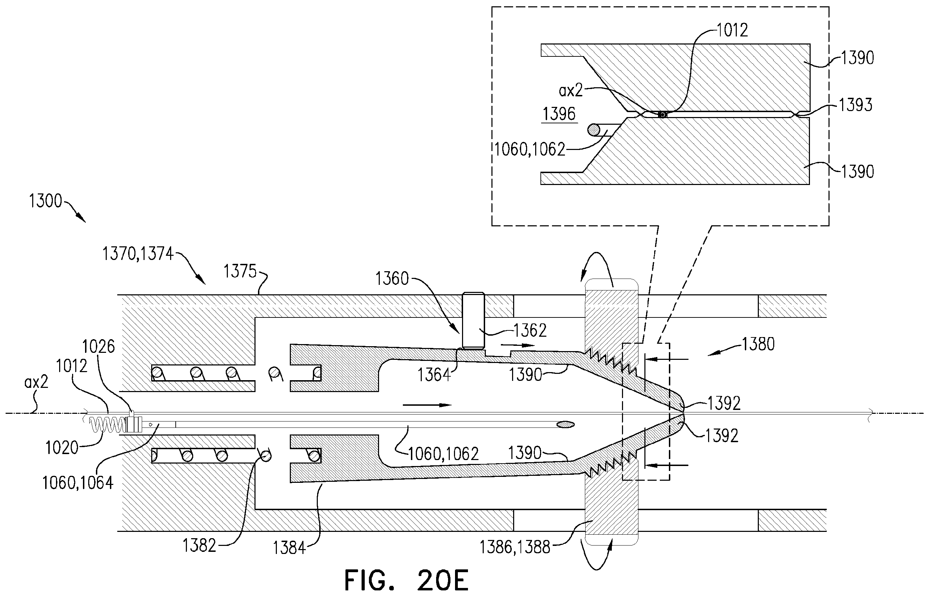

While clamp 1384 remains clamped to tether 1012, lock 1360 is unlocked, thereby triggering spring 1382 to cause tensioner 1380 to pull proximally on the tether, e.g., by the spring forcing the clamp proximally (Fig. 20E).

For some applications, and as shown, extracorporeal unit 1374 (e.g., lock 1360 thereof) is configured such that, after clamp 1384 has been squeezed into its clamped state, further squeezing of the clamp unlocks the clamp from housing 1375 by unlocking the lock, thereby triggering spring 1382 to cause tensioner 1380 to pull proximally on the tether. For example, once actuator 1386 has been actuated sufficiently (e.g., once ring 1388 has been rotated sufficiently) to transition clamp 1384 into its clamped state, further actuation of the actuator (e.g., further rotation of the actuator) unlocks the clamp from housing 1375 by unlocking lock 1360. This is illustrated by the transition between Fig. 20D and 20E, showing that ring 1388 has been screwed further over jaws 1390, squeezing the jaws sufficiently that recess 1364 is retracted from detent 1362, triggering spring 1382 to push clamp 1384 (as well as ring 1388) proximally, thereby pulling tether 1012 proximally, thereby reducing slack in the tether.

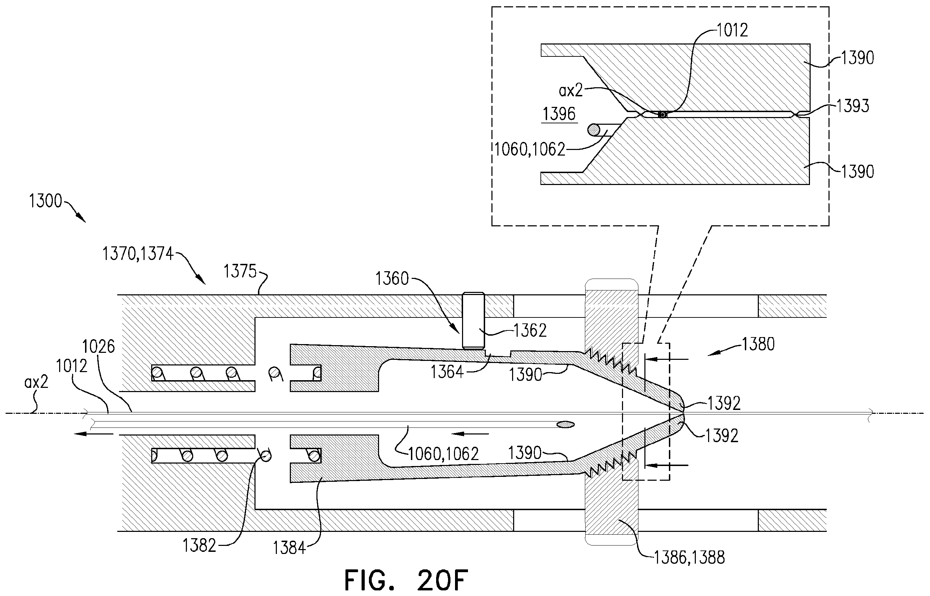

Subsequently, and while tensioner 1380 continues to maintain tension on tether 1012, driver 1060 is used to advance anchor 1020 further through catheter device 1370, along the tether, toward the tissue to which the anchor is to be anchored (Fig. 20F), e.g., similarly to as described for system 1000, mutatis mutandis.

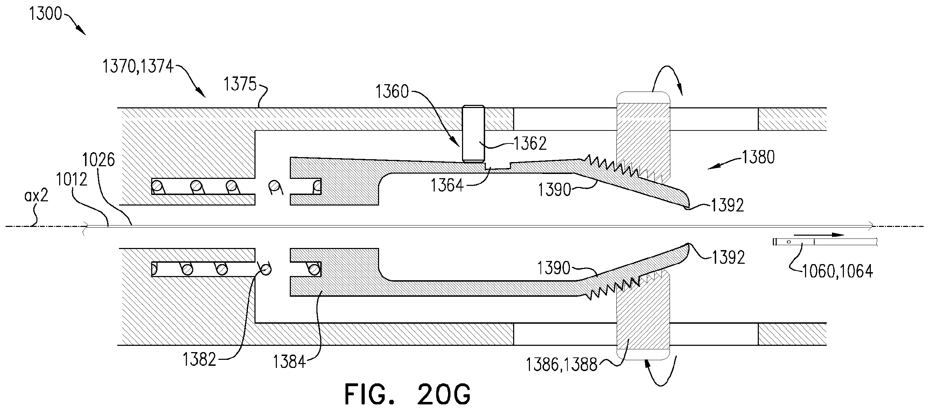

After anchor 1020 has been anchored, clamp 1384 is unclamped from tether 1012 (e.g., by reverse-actuating actuator 1386, such as by unscrewing ring 1388), and driver 1060 is withdrawn proximally (Fig. 20G).

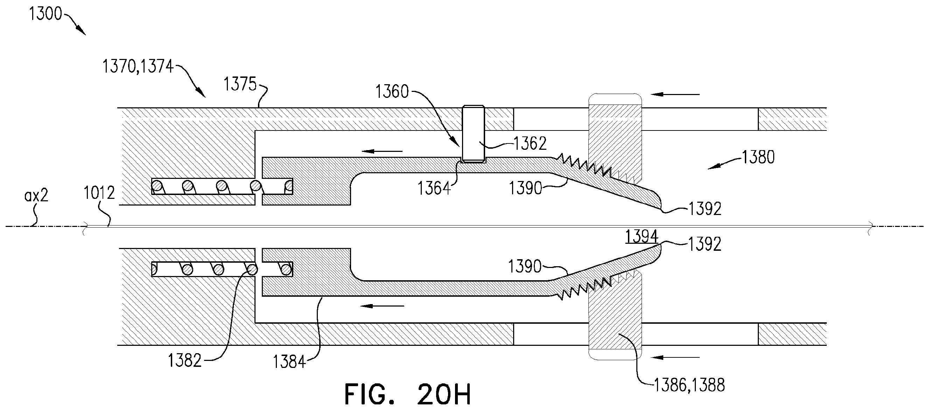

Tensioner 1380 is then returned to its initial position in which spring 1382 is locked in its stressed state (Fig. 20H). For example, and as shown, clamp 1384 can be slid linearly distally by sliding actuator 1386 linearly distally, e.g., without actuating the actuator (e.g., without rotating ring 1388), until lock 1360 locks, e.g., until detent 1362 is reenters recess 1364. For some applications in which clamp 1384 is biased toward assuming its unclamped state (e.g., due to spring-loading or shape-setting), lock 1360 automatically locks (e.g., click-locks) when the clamp reaches the appropriate axial position, e.g., with the clamp's bias causing detent 1362 to enter recess 1364. At this point, tensioner 1380, and catheter device 1370 as a whole, is ready for the next anchor to be advanced.

Reference is now made to Figs. 1A-20H. For some applications, tissue anchors 220, 320, 420, 520, 620, 720, 820, 1020 are interchangeable with each other, *mutatis mutandis.* For some applications, components of systems 1000, 1200, and 1400 are interchangeable with each other, *mutatis mutandis.* For some applications, techniques described with reference to one of systems 1000, 1200, and 1400 can be used, *mutatis mutandis,* with another of these systems.

For some applications, tissue anchors 220, 320, 420, 520, 620, 720, 820, 1020, implants 210, 310, 410, 510, 603, 703, 803, 1010, and/or systems 1000, 1200, 1300, 1400 can be used in combination with apparatuses, systems, and/or implanted using methods/techniques, described in one or more of the following references *mutatis mutandis,* including (but not limited to) by substituting corresponding components:
US Patent Application 14/437,373 to Sheps et al., which published as US 2015/0272634 (now US Patent 9,949,828)
US Patent Application 15/782,687 to Iflah et al., which published as US 2018/0049875
US Patent Application 16/534,875 to Brauon et al., which published as US 2020/0015971
US Patent Application 17/145,258 to Kasher et al., which published as US 2021/0145584
US Provisional Patent Application 63/162,443 to Shafigh et al.

Similarly, tissue anchors, implants, delivery tools, systems, and components thereof described in the above references can be used in combination with the apparatuses, systems, and/or methods/techniques described herein, including (but not limited to) by substituting corresponding components.

It will be appreciated by persons skilled in the art that the present invention is not limited to what has been particularly shown and described hereinabove. Rather, the scope of the present invention is defined by the appended claims. Further, the treatment techniques, methods, operations, steps, etc. described or suggested herein can be performed on a living animal or on a non-living simulation, such as on a cadaver, cadaver heart, simulator (e.g., with the body parts, tissue, etc. being simulated), etc.

## Claims

1. A system for use with a subject, comprising:
a catheter device (1470), comprising:
a tube (1072) that has a proximal opening (1073), and
an extracorporeal controller (1474) that comprises a track (1472) that leads to a deployment position;
a first cartridge (1420f) holding a first tissue anchor (1020), the first cartridge (1420f) shaped to be grasped by hand by a human operator and being coupled to the controller (1474) and, while remaining coupled to the controller (1474), being moveable along the track (1472) by hand by the human operator from a first initial position to the deployment position such that the first cartridge (1420f) holds the first tissue anchor (1020) opposite the proximal opening (1073); and
a second cartridge (1420) holding a second tissue anchor (1020), the second cartridge (1420) shaped to be grasped by hand by the human operator and being coupled to the controller (1474) and, while remaining coupled to the controller (1474), being moveable along the track (1472) by hand by the human operator from a second initial position to the deployment position such that the second cartridge (1420) holds the second tissue anchor (1020) opposite the proximal opening (1073).

2. The system according to claim 1, wherein the first tissue anchor (1020) comprises a first tissue-engaging element (1022) and a first head (1024) comprising a first eyelet (1026), and the second tissue anchor (1020) comprises a second tissue-engaging element (1022) and a second head (1024) comprising a second eyelet (1026).

3. The system according to claim 2, further comprising a tether (1012) threaded through the first eyelet (1026) and the second eyelet (1026), the tether (1012) having a proximal portion (1012p) that includes a proximal end of the tether (1012) and having a distal portion (1012d) that includes a distal end of the tether (1012), the distal end of the tether (1012) being advanceable distally through the tube (1072) into the subject while the proximal end of the tether (1012) remains outside of the subject;
preferably wherein an anchor driver (1060) is configured to advance the first anchor distally out of the first cartridge (1420f), through the proximal opening (1073), and through the tube (1072), while the first eyelet (1026) of the first anchor remains threaded on the tether (1012), and wherein the anchor driver (1060) is configured to advance the second anchor distally out of the second cartridge (1420), through the proximal opening (1073), and through the tube (1072), while the second eyelet (1026) of the second anchor remains threaded on the tether (1012).

4. The system according to claim 3, wherein the catheter device (1470) further comprises a tensioning device (1080) configured to apply tension to the tether (1012);
preferably wherein the tensioning device (1080) comprises a spring (1084) and a spool (1084), the spool (1084) coupled to the spring (1084) such that rotation of the spool (1084) in a first direction applies stress to the spring (1084), and wherein the proximal portion (1012p) of the tether (1012) is wound around the spool (1084) such that advancing of the distal portion (1012d) of the tether (1012) distally through the tube (1072) rotates the spool (1084) in the first direction.

5. The system according to any one of claims 1 to 4, wherein each of the first cartridge (1420f) and the second cartridge (1420) is configured to lock to the controller (1474) upon arriving at the deployment position.

6. The system according to any one of claims 1 to 5, further comprising an anchor driver (1060) that is couplable to the first anchor while the first anchor is held by the first cartridge (1420f) opposite the proximal opening (1073) and is configured to advance the first anchor distally out of the first cartridge (1420f) through the proximal opening (1073) and through the tube (1072), and that is couplable to the second anchor while the second anchor is held by the second cartridge (1420) opposite the proximal opening (1073) and is configured to advance the second anchor distally out of the second cartridge (1420) through the proximal opening (1073) and through the tube (1072);
preferably wherein each of the first cartridge (1420f) and the second cartridge (1420) is removable from the deployment position by being removed from the controller (1474).

7. The system according to any one of claims 1 to 6, wherein the first cartridge (1420f) comprises a first displaceable barrier (1224) that inhibits the first anchor from being advanced distally out of the first cartridge (1420f), and wherein the second cartridge (1420) comprises a second displaceable barrier (1224) that inhibits the second anchor from being advanced distally out of the second cartridge (1420).

8. The system according to claim 7, wherein the first cartridge (1420f) comprises a first displacement mechanism (1226) that, upon actuation of the first displacement mechanism (1226), displaces the first displaceable barrier (1224) such that the first displaceable barrier (1224) ceases to obstruct the first anchor from being advanced distally out of the first cartridge (1420f), and wherein the second cartridge (1420) comprises a second displacement mechanism (1226) that, upon actuation of the second displacement mechanism (1226), displaces the second displaceable barrier (1224) such that the second displaceable barrier (1224) ceases to obstruct the second anchor from being advanced distally out of the second cartridge (1420).

9. The system according to claim 8, wherein the first displacement mechanism (1226) and the second displacement mechanism (1226) are each spring-loaded.

10. The system according to claim 8, wherein the first displacement mechanism (1226) is configured to be actuated by a force applied to the first anchor, and wherein the second displacement mechanism (1226) is configured to be actuated by a force applied to the second anchor.

11. The system according to claim 10, wherein the first displacement mechanism (1226) is configured to be actuated by an anchor driver (1060) pulling the first anchor proximally, and wherein the second displacement mechanism (1226) is configured to be actuated by the anchor driver (1060) pulling the second anchor proximally.

12. The system according to claim 11, wherein the first cartridge (1420f) comprises a first piece (1230) that comprises the barrier, and a second piece (1232) that couples the first cartridge (1420f) to the extracorporeal unit and holds the first anchor.

13. The system according to claim 12, wherein the second piece (1232) defines a detent (1238) that constrains the first displacement mechanism (1226), and wherein the first displacement mechanism (1226) is configured to be actuated by the anchor driver (1060) pulling the first anchor proximally with sufficient force that the first anchor pulls the second piece (1232) proximally with respect to the first piece (1230) such that the detent (1238) ceases to constrain the first displacement mechanism (1226);
preferably wherein the second piece (1232) slidably couples the first cartridge (1420f) to the track (1472);
more preferably wherein the first cartridge (1420f) is configured such that the anchor driver (1060) pulling the first anchor proximally with sufficient force that the first anchor pulls the second piece (1232) proximally with respect to the first piece (1230) reconfigures the first cartridge (1420f) into a removable state that facilitates removal of the first cartridge (1420f) from the deployment position.

14. The system according to claim 12, wherein the second piece (1232) is mounted inside the first piece (1230), and the first piece (1230) is shaped to be grasped by hand by the human operator.

15. The system according to any one of claims 1 to 14, further comprising a third cartridge (1420) holding a third tissue anchor (1020) and being coupled to the controller (1474) and, while remaining coupled to the controller (1474), being moveable along the track (1472) from a third initial position to the deployment position such that the third cartridge (1420) holds the third tissue anchor (1020) opposite the proximal opening (1073).

## Patentansprüche

1. System zur Verwendung in einem Subjekt, umfassend:
eine Kathetervorrichtung (1470), umfassend:
einen Schlauch (1072), der eine proximale Öffnung aufweist (1073),
und eine extrakorporale Steuereinheit (1474), die eine Schiene (1472) umfasst, die zu einer Einsatzposition führt;
eine erste Kartusche (1420f), die einen ersten Gewebeanker (1020) hält, wobei die erste Kartusche (1420f) geformt ist, von einem menschlichen Operator mit der Hand gegriffen zu werden, und mit der Steuereinheit (1474) verbunden ist, und, während sie mit der Steuereinheit (1474) verbunden bleibt, von Hand durch den menschlichen Operator entlang der Schiene (1472) von einer ersten Ausgangsposition zu der Einsatzposition bewegbar ist, so dass die erste Kartusche (1420f) den ersten Gewebeanker (1020) gegenüber der proximalen Öffnung (1073) hält; und
eine zweite Kartusche (1420), die einen zweiten Gewebeanker (1020) hält, wobei die zweite Kartusche (1420) geformt ist, von einem menschlichen Operator mit der Hand gegriffen zu werden, und mit der Steuereinheit (1474) verbunden ist, und, während sie mit der Steuereinheit (1474) verbunden bleibt, von Hand durch den menschlichen Operator entlang der Schiene (1472) von einer zweiten Ausgangsposition zu der Einsatzposition bewegbar ist, so dass die zweite Kartusche (1420) den zweiten Gewebeanker (1020) gegenüber der proximalen Öffnung (1073) hält.

2. System gemäß Anspruch 1, wobei der erste Gewebeanker (1020) ein erstes gewebegreifendes Element (1022) und einen ersten Kopf (1024), der eine erste Öse (1026) aufweist, umfasst, und der zweite Gewebeanker (1020) ein zweites gewebegreifendes Element (1022) und einen zweiten Kopf (1024), der eine zweite Öse (1026) aufweist, umfasst.

3. System gemäß Anspruch 2, das ferner eine Leine (1012) umfasst, die durch die erste Öse (1026) und die zweite Öse (1026) gefädelt ist, wobei die Leine (1012) einen proximalen Abschnitt (1012p) aufweist, der ein proximales Ende der Leine (1012) umfasst, und einen distalen Abschnitt (1012d) aufweist, der ein distales Ende der Leine (1012) umfasst, wobei das distale Ende der Leine (1012) distal durch den Schlauch (1072) in den Patienten vorgeschoben werden kann, während das proximale Ende der Leine (1012) außerhalb des Patienten verbleibt;
wobei, vorzugsweise, ein Ankerantrieb (1060) dazu ausgelegt ist, den ersten Anker distal aus der ersten Kartusche (1420f) durch die proximale Öffnung und durch den Schlauch (1072) vorzuschieben, während die erste Öse (1026) des ersten Ankers auf der Leine (1012) aufgefädelt bleibt, und wobei der Ankerantrieb (1060) dazu ausgelegt ist, den zweiten Anker distal aus der zweiten Kartusche (1420) durch die proximale Öffnung (1073) und durch den Schlauch (1072) vorzuschieben, während die zweite Öse (1026) des zweiten Ankers auf der Leine (1012) aufgefädelt bleibt.

4. System gemäß Anspruch 3, wobei die Kathetervorrichtung (1470) ferner eine Spannvorrichtung (1080) umfasst, die dazu ausgelegt ist, Spannung auf die Leine (1012) auszuüben;
wobei, vorzugsweise, die Spannungsvorrichtung (1080) eine Feder (1084) und eine Spule (1084) umfasst, wobei die Spule (1084) mit der Feder (1084) verbunden ist, so dass eine Rotation der Spule (1084) in eine erste Richtung Spannung auf die Feder (1084) ausübt, und wobei der proximale Abschnitt (1012p) der Leine (1012) um die Spule (1084) gewickelt ist, so dass ein Vorschieben des distalen Abschnitts (1012d) der Leine distal durch den Schlauch (1072) die Spule (1084) in die erste Richtung dreht.

5. System gemäß einem der Ansprüche 1 bis 4, wobei sowohl die erste Kartusche (1420f) als auch die zweite Kartusche (1420) dazu ausgelegt sind, bei Erreichen der Einsatzposition die Steuereinheit (1474) zu verriegeln.

6. System gemäß einem der Ansprüche 1 bis 5, ferner umfassend einen Ankerantrieb (1060), der mit dem ersten Anker verbunden werden kann, während der erste Anker von der ersten Kartusche (1420f) gegenüber der proximalen Öffnung (1073) gehalten wird, und dazu ausgelegt ist, den ersten Anker distal aus der ersten Kartusche (1420f) durch die proximale Öffnung (1073) und den Schlauch (1072) vorzuschieben, und der mit dem zweiten Anker verbunden werden kann, während der zweite Anker von der zweiten Kartusche (1420) gegenüber der proximalen Öffnung (1073) gehalten wird, und dazu ausgelegt ist, den zweiten Anker distal aus der zweiten Kartusche (1420) durch die proximale Öffnung (1073) und durch den Schlauch (1072) vorzuschieben;
wobei, vorzugsweise, sowohl die erste Kartusche (1420f) als auch die zweite Kartusche (1420) von der Einsatzposition entfernt werden können, indem sie von der Steuereinheit (1474) wegbewegt werden.

7. System gemäß einem der Ansprüche 1 bis 6, wobei die erste Kartusche (1420f) eine erste verschiebbare Barriere (1224) umfasst, die verhindert, dass der erste Anker distal aus der ersten Kartusche (1420f) vorgeschoben wird, und wobei die zweite Kartusche (1420) eine zweite verschiebbare Barriere (1224) umfasst, die verhindert, dass der zweite Anker distal aus der zweiten Kartusche (1420) vorgeschoben wird.

8. System gemäß Anspruch 7, wobei die erste Kartusche (1420f) einen ersten Verschiebemechanismus (1226) umfasst, der bei Betätigung des ersten Verschiebemechanismus (1226) die erste verschiebbare Barriere (1224) verschiebt, so dass die erste verschiebbare Barriere (1224) aufhört zu verhindern, dass der erste Anker distal aus der ersten Kartusche (1420f) vorgeschoben wird, und wobei die zweite Kartusche (1420) einen zweiten Verschiebemechanismus (1226) umfasst, der bei Betätigung des zweiten Verschiebemechanismus (1226) die zweite verschiebbare Barriere (1224) verschiebt, so dass die zweite verschiebbare Barriere (1224) aufhört zu verhindern, dass der zweite Anker distal aus der zweiten Kartusche (1420) vorgeschoben wird.

9. System gemäß Anspruch 8, wobei sowohl der erste Verschiebemechanismus (1226) als auch der zweite Verschiebemechanismus (1226) federbelastet sind.

10. System gemäß Anspruch 8, wobei der erste Verschiebemechanismus (1226) dazu ausgelegt ist, durch eine auf den ersten Anker ausgeübte Kraft betätigt zu werden, und wobei der zweite Verschiebemechanismus (1226) dazu ausgelegt ist, durch eine auf den zweiten Anker ausgeübte Kraft betätigt zu werden.

11. System gemäß Anspruch 10, wobei der erste Verschiebemechanismus (1226) dazu ausgelegt ist, durch einen Ankerantrieb (1060), der den ersten Anker proximal zieht, betätigt zu werden, und wobei der zweite Verschiebemechanismus (1226) dazu ausgelegt ist, durch den Ankerantrieb (1060), der den zweiten Anker proximal zieht, betätigt zu werden.

12. System gemäß Anspruch 11, wobei die erste Kartusche (1420f) einen ersten Teil (1230), der eine Barriere umfasst, und einen zweiten Teil (1232), der die erste Kartusche (1420f) mit der extrakorporalen Einheit verbindet und den ersten Anker hält, umfasst.

13. System gemäß Anspruch 12, wobei der zweite Teil (1232) eine Arretierung (1238) definiert, die den ersten Verschiebemechanismus (1226) einschränkt, und wobei der erste Verschiebemechanismus (1226) dazu ausgelegt ist, durch den Ankerantrieb (1060), der den ersten Anker mit ausreichender Kraft proximal zieht, dass der erste Anker den zweiten Teil (1232) proximal in Bezug auf den ersten Teil (1230) zieht, so dass die Arretierung (1238) aufhört, den ersten Verschiebemechanismus (1226) zu beschränken;
wobei, vorzugsweise, der zweite Teil (1232) die erste Kartusche (1420f) verschiebbar mit der Schiene (1472) verbindet;
wobei, noch bevorzugter, die erste Kartusche (1420f) dazu ausgelegt ist, dass der Ankerantrieb (1060), der den ersten Anker mit ausreichender Kraft proximal zieht, dass der erste Anker den zweiten Teil (1232) proximal in Bezug auf den ersten Teil (1230) zieht, die erste Kartusche (1420f) in einen entfernbaren Zustand umkonfiguriert, der ein Entfernen der ersten Kartusche (1420f) von der Einsatzposition erleichtert.

14. System gemäß Anspruch 12, wobei der zweite Teil (1232) innerhalb des ersten Teils (1230) angebracht ist, und der erste Teil (1230) so geformt ist, dass es von einem menschlichen Operator mit der Hand gegriffen werden kann.

15. System gemäß einem der Ansprüche 1 bis 14, ferner umfassend eine dritte Kartusche (1420), die einen dritten Gewebeanker (1020) aufweist und mit der Steuereinheit (1474) verbunden ist und, während sie mit der Steuereinheit (1474) verbunden bleibt, entlang der Schiene (1472) von einer dritten Ausgansposition zu der Einsatzposition bewegbar ist, so dass die dritte Kartusche (1420) den dritten Gewebeanker (1020) gegenüber der proximalen Öffnung (1073) hält.

## Revendications

1. Système destiné à être utilisé avec un sujet, comprenant :
un dispositif de cathéter (1470), comprenant :
un tube (1072) présentant une ouverture proximale (1073) et
un dispositif de commande (1474) extracorporel qui comprend une piste (1472) qui mène à une position de déploiement ;
une première cartouche (1420f) maintenant un premier ancrage tissulaire (1020), la première cartouche (1420f) étant façonnée pour être saisie à la main par un opérateur humain et étant accouplée au dispositif de commande (1474) et, tout en restant accouplée au dispositif de commande (1474), étant mobile le long de la piste (1472) à la main par l'opérateur humain depuis une première position initiale vers la position de déploiement de telle sorte que la première cartouche (1420f) maintient le premier ancrage tissulaire (1020) à l'opposé de l'ouverture proximale (1073) ; et
une deuxième cartouche (1420) maintenant un deuxième ancrage tissulaire (1020), la deuxième cartouche (1420) étant façonnée pour être saisie à la main par l'opérateur humain et étant accouplée au dispositif de commande (1474) et, tout en restant accouplée au dispositif de commande (1474), étant mobile le long de la piste (1472) à la main par l'opérateur humain depuis une deuxième position initiale vers la position de déploiement de telle sorte que la deuxième cartouche (1420) maintient le deuxième ancrage tissulaire (1020) à l'opposé de l'ouverture proximale (1073).

2. Système selon la revendication 1, le premier ancrage tissulaire (1020) comprenant un premier élément de mise en prise (1022) de tissu et une première tête (1024) comprenant un premier œillet (1026) et le deuxième ancrage tissulaire (1020) comprenant un second élément de mise en prise (1022) de tissu et une seconde tête (1024) comprenant un second œillet (1026).

3. Système selon la revendication 2, comprenant en outre une attache (1012) filetée à travers le premier œillet (1026) et le second œillet (1026), l'attache (1012) présentant une partie proximale (1012p) qui comprend une extrémité proximale de l'attache (1012) et présentant une partie distale (1012d) qui comprend une extrémité distale de l'attache (1012), l'extrémité distale de l'attache (1012) pouvant être avancée de manière distale à travers le tube (1072) dans le sujet tandis que l'extrémité proximale de l'attache (1012) reste à l'extérieur du sujet ;
de préférence, un dispositif d'entraînement (1060) d'ancrage étant conçu pour faire avancer le premier ancrage de manière distale hors de la première cartouche (1420f), à travers l'ouverture proximale (1073) et à travers le tube (1072), tandis que le premier œillet (1026) du premier ancrage reste fileté sur l'attache (1012) et le dispositif d'entraînement (1060) d'ancrage étant conçu pour faire avancer le deuxième ancrage de manière distale hors de la deuxième cartouche (1420), à travers l'ouverture proximale (1073) et à travers le tube (1072), tandis que le second œillet (1026) du deuxième ancrage reste fileté sur l'attache (1012).

4. Système selon la revendication 3, le dispositif de cathéter (1470) comprenant en outre un dispositif de tension (1080) conçu pour appliquer une tension à l'attache (1012) ; de préférence, le dispositif de tension (1080) comprenant un ressort (1084) et une bobine (1084), la bobine (1084) étant accouplée au ressort (1084) de telle sorte que la rotation de la bobine (1084) dans un premier sens applique une contrainte au ressort (1084) et la partie proximale (1012p) de l'attache (1012) étant enroulée autour de la bobine (1084) de telle sorte que l'avancement de la partie distale (1012d) de l'attache (1012) de manière distale à travers le tube (1072) fait tourner la bobine (1084) dans le premier sens.

5. Système selon l'une quelconque des revendications 1 à 4, chaque cartouche parmi la première cartouche (1420f) et la deuxième cartouche (1420) étant conçue pour se verrouiller au dispositif de commande (1474) lors de l'arrivée à la position de déploiement.

6. Système selon l'une quelconque des revendications 1 à 5, comprenant en outre un dispositif d'entraînement (1060) d'ancrage qui peut être accouplé au premier ancrage tandis que le premier ancrage est maintenu par la première cartouche (1420f) à l'opposé de l'ouverture proximale (1073) et qui est conçu pour faire avancer le premier ancrage de manière distale hors de la première cartouche (1420f) à travers l'ouverture proximale (1073) et à travers le tube (1072) et qui peut être accouplé au deuxième ancrage tandis que le deuxième ancrage est maintenu par la deuxième cartouche (1420) à l'opposé de l'ouverture proximale (1073) et qui est conçu pour faire avancer le deuxième ancrage de manière distale hors de la deuxième cartouche (1420) à travers l'ouverture proximale (1073) et à travers le tube (1072) ;
de préférence, chaque cartouche parmi la première cartouche (1420f) et la deuxième cartouche (1420) pouvant être retirée de la position de déploiement en étant retirée du dispositif de commande (1474).

7. Système selon l'une quelconque des revendications 1 à 6, la première cartouche (1420f) comprenant une première barrière déplaçable (1224) qui empêche l'avancement du premier ancrage de manière distale hors de la première cartouche (1420f) et la deuxième cartouche (1420) comprenant une seconde barrière déplaçable (1224) qui empêche l'avancement du deuxième ancrage de manière distale hors de la deuxième cartouche (1420).

8. Système selon la revendication 7, la première cartouche (1420f) comprenant un premier mécanisme de déplacement (1226) qui, lors de l'actionnement du premier mécanisme de déplacement (1226), déplace la première barrière déplaçable (1224) de telle sorte que la première barrière déplaçable (1224) cesse d'obstruer l'avancement du premier ancrage de manière distale hors de la première cartouche (1420f) et la deuxième cartouche (1420) comprenant un second mécanisme de déplacement (1226) qui, lors de l'actionnement du second mécanisme de déplacement (1226), déplace la seconde barrière déplaçable (1224) de telle sorte que la seconde barrière déplaçable (1224) cesse d'obstruer l'avancement du deuxième ancrage de manière distale hors de la deuxième cartouche (1420).

9. Système selon la revendication 8, le premier mécanisme de déplacement (1226) et le second mécanisme de déplacement (1226) étant chacun chargés par ressort.

10. Système selon la revendication 8, le premier mécanisme de déplacement (1226) étant conçu pour être actionné par une force appliquée au premier ancrage et le second mécanisme de déplacement (1226) étant conçu pour être actionné par une force appliquée au deuxième ancrage.

11. Système selon la revendication 10, le premier mécanisme de déplacement (1226) étant conçu pour être actionné par un dispositif d'entraînement (1060) d'ancrage tirant le premier ancrage de manière proximale et le second mécanisme de déplacement (1226) étant conçu pour être actionné par le dispositif d'entraînement (1060) d'ancrage tirant le deuxième ancrage de manière proximale.

12. Système selon la revendication 11, la première cartouche (1420f) comprenant une première pièce (1230) qui comprend la barrière et une seconde pièce (1232) qui accouple la première cartouche (1420f) à l'unité extracorporelle et maintient le premier ancrage.

13. Système selon la revendication 12, la seconde pièce (1232) définissant un cliquet (1238) qui contraint le premier mécanisme de déplacement (1226) et le premier mécanisme de déplacement (1226) étant conçu pour être actionné par le dispositif d'entraînement (1060) d'ancrage tirant le premier ancrage de manière proximale avec une force suffisante pour que le premier ancrage tire la seconde pièce (1232) de manière proximale par rapport à la première pièce (1230) de telle sorte que le cliquet (1238) cesse de contraindre le premier mécanisme de déplacement (1226) ;
de préférence, la seconde pièce (1232) accouplant de manière coulissante la première cartouche (1420f) à la piste (1472) ;
de façon plus préférée, la première cartouche (1420f) étant conçue de telle sorte que le dispositif d'entraînement (1060) d'ancrage, tirant le premier ancrage de manière proximale avec une force suffisante pour que le premier ancrage tire la seconde pièce (1232) de manière proximale par rapport à la première pièce (1230), reconfigure la première cartouche (1420f) dans un état amovible qui facilite le retrait de la première cartouche (1420f) de la position de déploiement.

14. Système selon la revendication 12, la seconde pièce (1232) étant montée à l'intérieur de la première pièce (1230) et la première pièce (1230) étant façonnée pour être saisie à la main par l'opérateur humain.

15. Système selon l'une quelconque des revendications 1 à 14, comprenant en outre une troisième cartouche (1420) maintenant un troisième ancrage tissulaire (1020) et étant accouplée au dispositif de commande (1474) et, tout en restant accouplée au dispositif de commande (1474), étant mobile le long de la piste (1472) depuis une troisième position initiale vers la position de déploiement de telle sorte que la troisième cartouche (1420) maintient le troisième ancrage tissulaire (1020) à l'opposé de l'ouverture proximale (1073).
